# EUROPEAN PATENT SPECIFICATION

(11) **EP 2 491 119 B1**
(45) Date of publication and mention of the grant of the patent: **08.06.2016**
(21) Application number: 10771417.2
(22) Date of filing: 19.10.2010
(51) Int. Cl.: C12N 9/02

(54) **METHODS AND MEANS TO ALTER LIPID BIOSYNTHESIS BY TARGETING MULTIPLE ENZYMES TO SUBORGANELLE DOMAINS**
VERFAHREN UND MITTEL ZUR MODIFIZIERUNG DER LIPID BIOSYNTHESE DURCH AUSRICHTUNG MEHRFACHER ENZYME AUF SUBORGANELLENDOMÄNEN
PROCÉDÉS ET MOYENS POUR ALTÉRER LA BIOSYNTHÈSE DES LIPIDES PAR CIBLAGE DE MULTIPLES ENZYMES SUR DES DOMAINES D'ORGANELLES SUB-CELLULAIRES

(30) Priority: 20.10.2009 EP 09013248
(43) Date of publication of application: 29.08.2012
(73) Proprietor: Bayer CropScience NV, 1831 Diegem (BE)
(72) Inventor: FEUSSNER, Ivo, 37085 Göttingen (DE); HEILMANN, Mareike, 37073 Göttingen (DE)
(86) International application number: PCT/EP2010/065754
(87) International publication number: WO 2011/048119

(56) References cited:
- WO-A1-01/29227

## Description

### Field of the invention

The current invention relates to the field of fatty acid and lipid metabolism. More particularly, methods are provided to alter lipid biosynthesis in non-human eukaryotic organisms by targeting at least two different polypeptides involved in fatty acid or lipid metabolism towards a similar or the same subdomain of an organelle, such as the endoplasmatic reticulum (ER), through fusion of the polypeptides with a similar or the same heterologous polypeptide targeting the chimeric fusion polypeptide to the mentioned subdomain, such as an oleosin. Conveniently, such targeting of the chimeric polypeptides is achieved via recombinant DNA techniques. In a particular embodiment, non-human eukaryotic organism (such as yeasts or plants) are provided which are capable of synthesizing wax esters through expression of a fatty acylCoA reductase and a wax ester synthetase, whereby both polypeptides are operably linked to a similar or the same oleosin polypeptide. By introducing fatty acylCoA reductase and a wax ester synthetase from mouse, with a substrate preference for C12-C18 chain fatty acids, non-human eukaryotic organisms can be provided with a new capability of synthesizing short chain wax esters. Such short chain wax esters have several applications including a potential use as high grade lubrificants.

### Background

Lipids and oils, particularly plant oils, represent an important source of nutrients for both human and animal use. Plant oils moreover represent renewable sources of long-chain hydrocarbons that can be used as chemical or fuel feedstocks. The efficient production of oils and lipids, including industrially applicable oils and lipids or oils with specific compositions, in plants is a major goal for plant biotechnology. Although significant advances towards these goals have been achieved, it remains a challenge to provide organisms, such as plants with new enzymes involved in lipid biosynthesis and to obtain efficient expression of the novel catalytic activity and resulting specific lipids. Indeed, the unique types of fatty acids or lipids produced may be incompatible with incorporation into cellular membranes or may interfere with the normal flux of fatty acids into storage oil.

Dyer and Mullen, 2008 (Physiologia Plantarum 132: 11-22) have suggested that the utilization of specific novel enzymes such as diverged FAD enzymes for production in value-added oils in transgenic plants will require placing the enzymes in the correct metabolic context to generate the desired products. These authors further reviewed data concerning the peptide sequences of plant FAD enzymes associated with insertion and maintenance of the enzymes in the ER. This so-called C-terminal retrieval motif could also be identified in enzymes such as DGAT, fatty acid elongases, PDATs and the like.

Shockey et al. 2006 (Plant Cell 18: 2294-2313) described experiments with DGAT proteins demonstrating that these proteins are located in distinct regions or 'subdomains' of ER, suggesting that that the C-terminal motifs might be involved in delivery of functionally related proteins to similar regions of ER. Addition of the C-terminal aromatic motifs of DGATs to reporter proteins, however, resulted in localization of these proteins to general ER, rather than subdomains of ER, indicating that other portions of the proteins are required for their localization and/or organization into functionally distinct ER subdomains.

The prior art therefore remains defective in providing a solution to engineer functionally related novel enzymes in such a way as to target them to specific subdomains of the ER and efficiently synthesize the novel product through concerted action of the novel enzymes.

Described herein is the production of short chain wax esters in plants. Wax esters, the main constituents of waxes, are known to be neutral lipids, consisting of long-chain fatty acids, which are esterified with long chain fatty alcohols. The two molecular species included in a wax ester can differ in chain length and number of double bonds, which leads to a broad variety of different esters. Wax esters are highly hydrophobic and polar due to the lack of a head group and, for this reason, insoluble in water. Further, they usually are ductile at room temperature and rather viscous after melting. Additional, long chain wax esters are known to have a very low volatility (Vrkoslav and Mikova, 2009). Wax esters are widespread in nature and can be found in plants, microorganisms and animals, mainly coating their surface to prevent water loss, abrasion and infection (Cheng and Russell, 2004a).

The Jojoba plant (Simmondsia chinensis) is unique based on its ability to use liquid wax esters as energy storage instead of triacylglycerols (TAG) in its seeds. It is therefore of high interest in terms of renewable sources of high quality oil. The jojoba wax esters consist of 18:1, 20:1 and 22:1 fatty acids linked to 20:1, 22:1 and 24:1 fatty alcohols, meaning that the wax contains C38 to C44 esters with one double bond in each alkyl moiety, respectively. However, jojoba plants grow only in restricted geographic areas and the seed yield of these plants is not sufficient to allow the use of jojoba was esters in high quantities.

Wax esters fulfill a variety of diverse and important biological functions. They can act as protection against desiccation, UV light and pathogens by coating plant leaf surfaces (cuticula) with a thin layer, respectively. Waxes can also function as water - proof layer to the feathers of birds. Furthermore, wax esters may have structural functions for instance while being secreted by bees to construct their honeycombs. As described above, wax esters also function as energy storage in the jojoba plant and other members of the family Euphorbiaceae. These plants contain mainly wax esters in their seeds instead of the usual triacylglycerol (TAG). Sperm whales produce spermaceti oil in their head cavities, which is needed for regulation of buoyancy, sound transmission and echo location. Mammals produce wax esters as well, mostly in the sebaceous glands, which secrete the esters together with further substances (sebum) onto the surface of the skin and eyes.

Wax esters have a multitude of important technical applications in a variety of areas, including medicine, cosmetics, and food industries, as well as a more traditional use as lubricants. A traditional source for wax esters was the spermaceti oil produced by spermaceti whales. These esters have been used extensively in lubrication and transmission fluids.

One goal of the current invention is to provide a solution to the production in sufficient amounts from a readily renewable source, of types of wax esters with high value due to lower melting points and enhanced lubrication properties impaired by shorter chain length and/or hydroxyl and methyl substitutions. Short-chain wax esters are of particular interest, because they can be used at extremely low temperatures and high pressures without any disaggregation.

### Brief description of the drawings

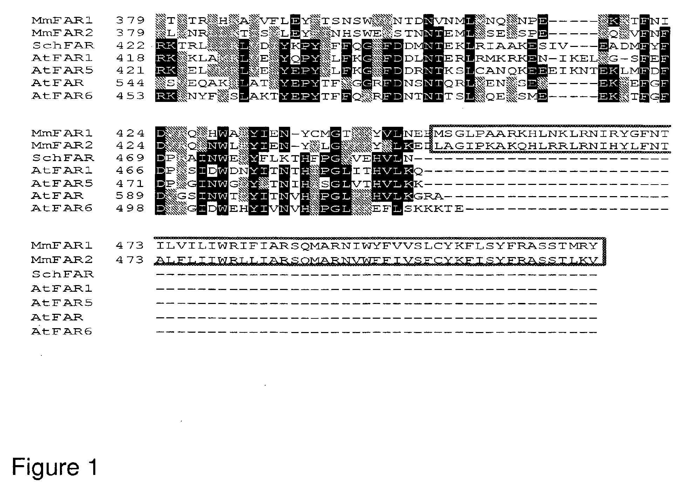

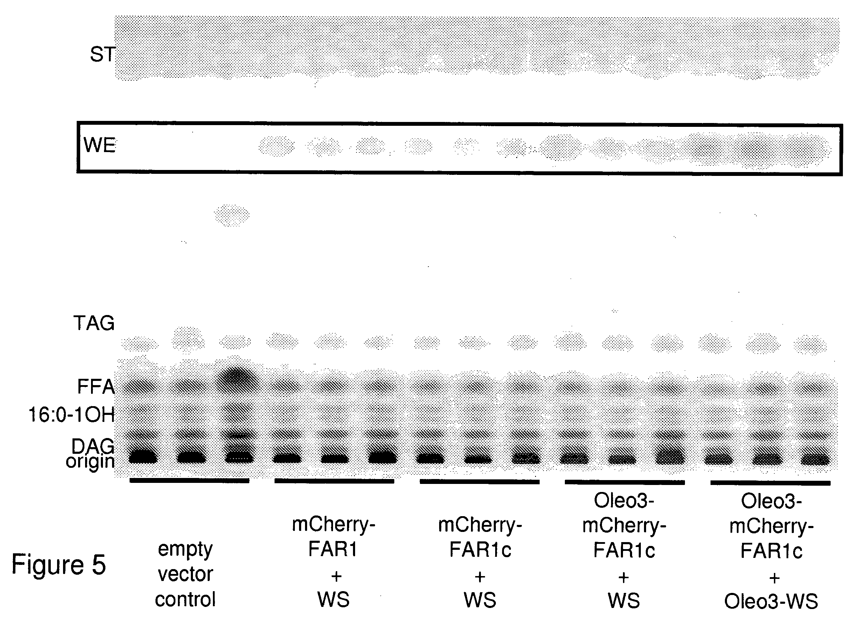

**Fig. 1****: Alignment of FAR enzymes of various organisms.**
   Alignment of the two fatty acid reductases (FAR) of Mus musculus MmFAR1 (protein ID Mm.206919) and MmFAR2 (protein ID Mm.475174) with diverse FARs from Arabidopsis thaliana AtFAR (At3g11980); AtFAR1 (At5g22500); AtFAR5 (At3g44550); AtFAR6 (Atg56700) and S. chinensis (Jojoba), SchFAR (Accession No. AF149918).

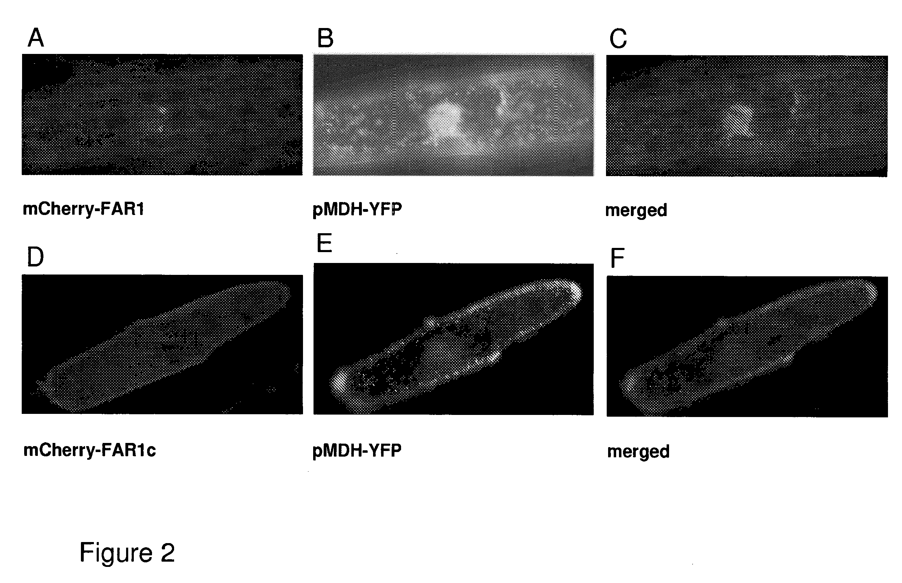

**Fig. 2****: Localization of various mCherry fusion proteins in onion epidermial cells.**
   Onion epidermal peels were bombarded with DNA encoding different mCherry fusion proteins. The bombarded cells were incubated over night at room temperature and then analyzed via fluorescence microscopy.
   A-C, Localization analysis of mCherry-FAR1 with the peroxisomal marker MDH: A, Onion epidermal cell expressing mCherry-MmFAR1 B, onion epidermal cells coexpressing the peroxisomal marker MDH with mCherry-MmFAR1 and C, overlay of A and B.
   D-F, Localization analysis of mCherry-FAR1c with the peroxisomal marker MDH: D show onion epidermal cells expressing mCherry-MmFAR1c. E, Co-expression of the peroxisomal marker MDH. I, Overlay of mCherry-MmFAR1c fluorescence image with the MDH fluorescence image.

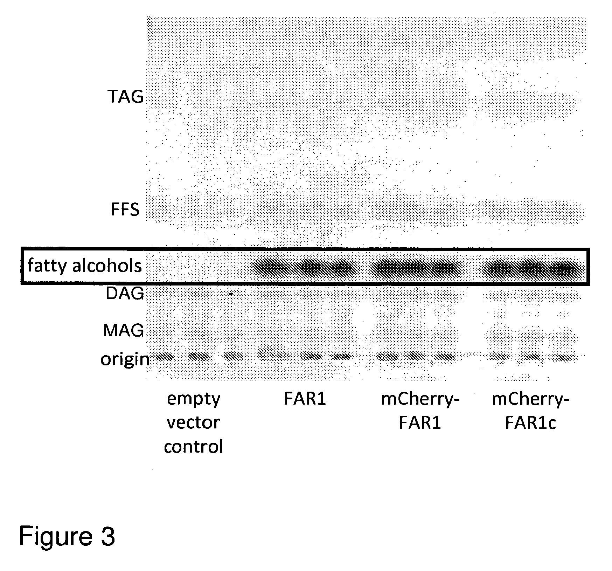

Fig. 3: TLC analysis of fatty alcohol products.
   Total lipid extracts from yeast expressing either the empty vector (control), FAR1, mCherry-MmFAR1 or the C-terminal truncated version, mCherry-FAR1c.. Standards: DAG, diacylglycerol; 16:0-OH as standard for fatty alcohols; FFA, free fatty acids; WE, wax ester; ST, sterols. Developing solvent: hexane:diethyl ether:acetic acid (65:35:1 v/v/v). This experiment was performed three times, once also using a different yeast strain (INVSc1).

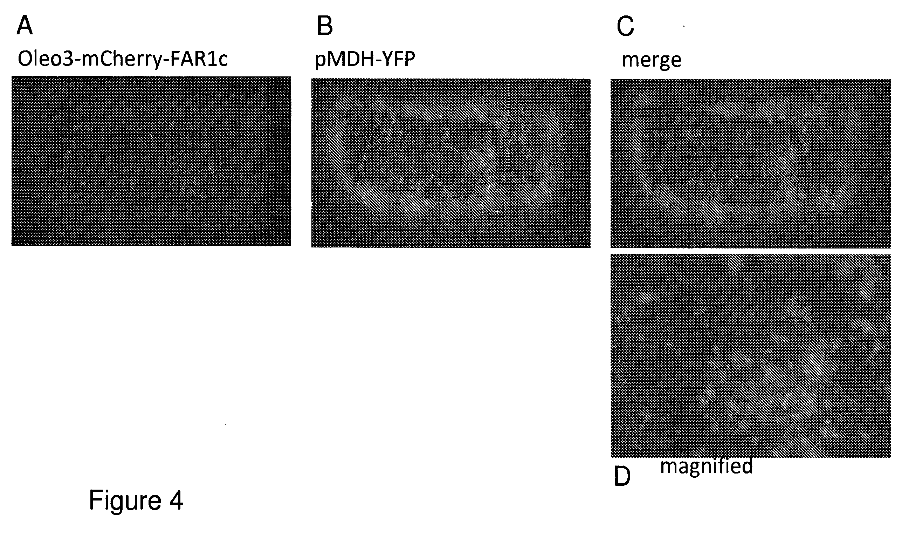

Fig. 4: **Oleo3-mCherry-MmFAR1** and **Oleo3-mCherry-MmFAR1c** constructs in **onion epidermal cells.**
   Onion epidermal peels were bombarded with DNA encoding different mCherry fusion proteins. The bombarded cells were incubated over night at room temperature and then analyzed via fluorescence microscopy. A-C: Localization analysis of Oleo3-mCherry-FAR1c. D: Onion epidermal cells expressing either Oleo3-mCherry-MmFAR1c or, E co-expressing either the peroxisomal marker MDH with Oleo3- mCherry-MmFAR1c. F overlay of D and E and G and H, respectively. Bombardment was performed twice and in each experiment about 50 cells were observed. E: Enlarged detail of the overlay of Oleo3-mCherry- MmFAR1c co-expressed with MDH. The YFP fluorescent spots represent the peroxisomes visualized by the YFP tagged MDH. The red spots represent the position of the FAR1c constructs visualized by mCherr
**Fig. 5****: TLC performed with mCherry-MmFAR1+MmWS, mCherry-MmFAR1c+MmWS, Oleo3mCherry-MmFAR1c + MmWS and Oleo3mCherry-MmFAR1c + Oleo-MmWS expressed for 48 h**
   A: Sterols, a TAG-mixture, 16:0 fatty acid, 16:0-OH fatty alcohol and wax esters (WE) were used as standards. A control containing only the empty vector, the vector containing **mCherry-FAR1+WS,** the vector containing mCherry-Far1c+WS, the vector containing the Oleo3- mCherry-FAR1c +WS and the vector containing the Oleo3-mCherry-MmFAR1c + Oleo3-WS constructs were tested. Developing solvent: hexane:diethyl ether:formic acid (90:10:1 v/v/v).
**Fig 6****: Diagram shows the quantification of wax ester accumulation in the samples 1-4 of** **Fig 5****.**
**Fig. 7****: Overview of the MmFAR2, MmFAR1 and MmWS constructs.**
   A, original unmodified MmFAR1 enzyme tagged to mCherry fused to A. thaliana oleosin 3.
   B, truncated version of MmFAR1, MmFAR1c, tagged to mCherry fused to A. thaliana oleosin 3. MmFAR1c lacks the C-terminal putative transmembrane domains.
   C MmWS fused to A. thaliana oleosin 3.
**Fig. 8****: Nile red stained onion epidermis cells.**
   Onion epidermal peels, bombarded with DNA encoding YFP-Oleosin3, or onion epidermal peels not expressing any transgene were stained with Nile red. A: Localization of Olco3-YFP. B: Localization of Nile Red staining. C: Overlay of A and B. D: Localization of Nile Red staining in untransformed onion cells. E: Magnification of C.

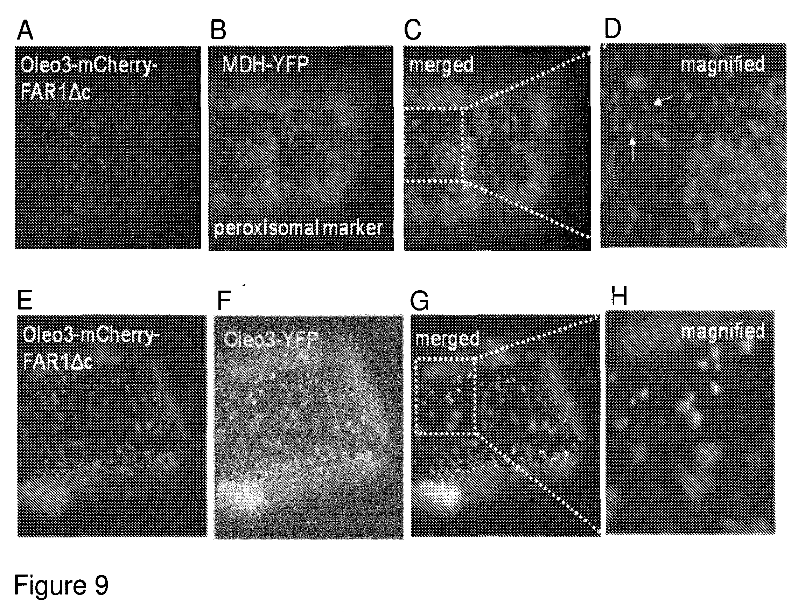

**Fig. 9****: Localization of Oleo3-mCherry-MmFAR1c in onion epidermal cells co-expressed with pMDH or YFP-Oleo3.**
   Onion epidermal peels were co-bombarded with DNA encoding the Oleo3-mCherry-MmFAR1c fusion protein together with DNA encoding the peroxisomal marker MDH-YFP or with the oil body marker Oleo3-YFP. A and E: Localization of Oleo3-mCherry-MmFAR1c. B: Localization of MDH-YFP (peroxisome). C: Overlay of A and B. D: Magnification of C. F: Localization of Oleo3-YFP (oil body). G: Overlay of E and F. H: Magnification of G.

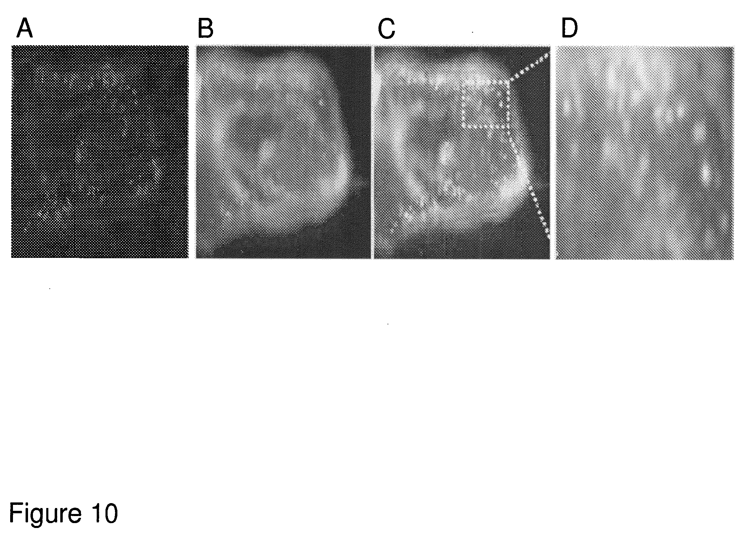

**Fig. 10****: Co-localization of Oleo3-mCherry-FAR1c with Oleo3-YFP-WS in onion epidermis cells.**
   Onion epidermal peels were co-bombarded with DNA encoding the Oleo3-mCherry-MmFAR1c fusion protein together with DNA encoding the Oleo3-YFP-WS fusion protein. A: Localization of Oleo3-mCherry-MmFAR1c. B: Localization of Oleo3-YFP-WS. C: Overlay of A and B. D: Magnification of C.

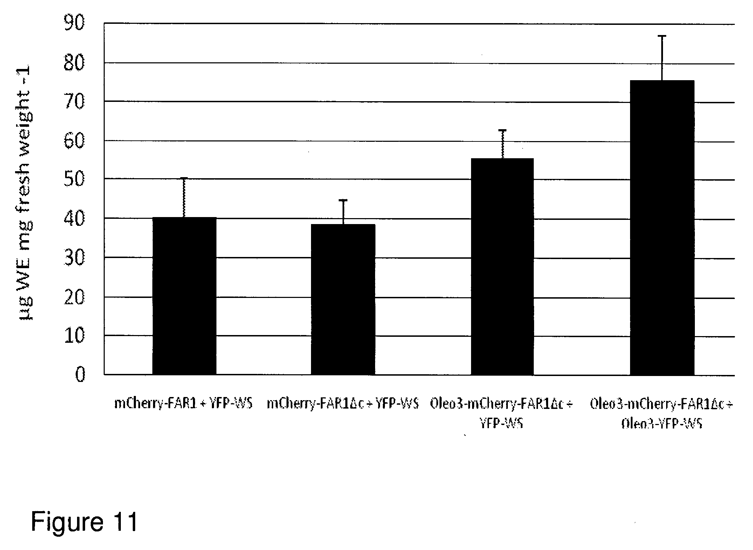

**Fig. 11****: Quantification of WE accumulation by GC-MS after co-expression of unmodified and modified MmFAR1 and MmWS.**
   mCherry-MmFAR1+YFP-MmWS, mCherry-MmFAR1c+ YFP-MmWS, Oleo3mCherry-MmFARlc + MmWS and Oleo3mCherry-MmFAR1c + Oleo3-YPF-MmWS were expressed in yeast for 72 h at 30°C. WE accumulation was quantified by GC-MS. Experiment represents data from 6 independent clones in each case.

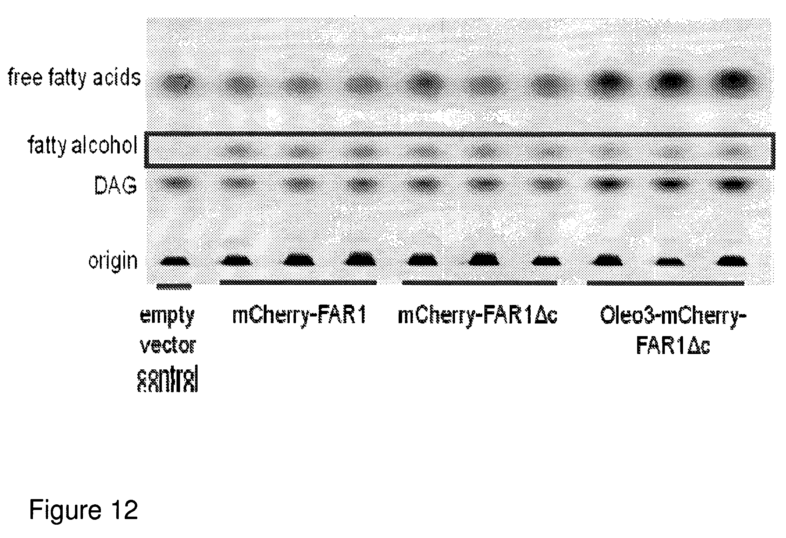

**Fig. 12****: Individual expression of different MmFAR1-versions in yeast.**
   mCherry-MmFAR1, mCherry-MmFAR1c and Oleo3-mCherry-MmFAR1c were expressed in yeast for 72 h at 30°C. Three independent clones were tested. The fatty acids were analyzed with TLC as described for Figure 5.

### Summary of the invention

In one embodiment of the invention, a method is provided for modulating lipid biosynthesis in a non-human eukaryotic organism comprising the step of providing to cells of said organism at least a first and a second chimeric protein
a. said first chimeric protein comprising:
   i. a first heterologous polypeptide targeting said proteins to a particular subdomain of an organelle in said cell operably linked to
   ii. a first polypeptide involved in fatty acid metabolism or lipid metabolism; and
b. a second chimeric protein comprising:
   i. a second heterologous polypeptide targeting said proteins to said particular subdomain of an organelle in said cell operably linked to
   ii. a second polypeptide involved in fatty acid metabolism or lipid metabolism
wherein said first and second polypeptide involved in fatty acid metabolism or lipid metabolism are different polypeptides with a catalytic activity which actively contribute to the fatty acid metabolism or lipid metabolism, and wherein said first and second polypeptide involved in fatty acid metabolism or lipid metabolism act in a concerted manner; and
wherein said method is not a method of treatment of the human or animal body by surgery or therapy, or a diagnostic method practiced on the human or animal body.

The heterologous polypeptide may comprises a polypeptide sequence of an oilbody protein or a targeting part thereof, or the heterologous polypeptide comprises a polypeptide sequence selected from an oleosin, such as a polypeptide sequence having at least 80% homology to the amino acid sequence of SEQ ID No SEQ ID No 10 from amino acid 1 to amino acid 143, or an endoplasmatic retrieval signal from fatty acid desaturases, the N-terminal domain of LBLOX or the N-terminal domain of PLA. The polypeptide involved in fatty acid metabolism or lipid metabolism may be selected from an acyl-CoA synthetase, a glycerol-phosphate acyltransferase, an O-acyltransferase, a lyso-phosphatidic acid acyltransferase, a phosphatidic acid phosphatase, a diacylglycerol acyltransferase, an oleate desaturases, a linoleate desaturases, an acyl-CoA hydroxylase, an acyl-lipid hydroxylase, a fatty acid epoxidase, a phospholipid:sterol acyltransferase, a phospholipid:diacylglycerol acyltransferase, a diacylglycerol transacylase, a lysophosphatidylcholine acyltransferase, a phosphatidylcholine:diacylglycerol cholinephosphotransferase, an acyl-CoA elongase, an acyl-lipid elongase, a phosphatidylglycerol-phosphate synthetase, a phosphatidylglycerol-phosphate phosphatase, a CDP-diacylglycerol synthetase, a phosphatidylinositol synthase, a phosphatidylserine synthase, a choline kinase, an ethanolamine kinase, a CDP-choline synthetase, a CDP-ethanolamine synthetase, a phosphatidylserine decarboxylase, a lipoxygenase, a phospholipase, a lipase, a carboxylesterase, a fatty alcohol reductase, a wax ester synthase, a bifunctional acyltranferases/wax synthase, a ketoacyl-CoA synthase, a ketoacyl-CoA reductase, a hydroxylacyl-CoA dehydrase, an enoyl-CoA reductase, an alcohol-forming fatty acyl-CoA reductase, an aldehyde-forming fatty acyl-CoA reductase, an aldehyde decarbonylase, a wax ester hydrolase, a glycerol-3-P-dehydrogenase, a CDP-choline: 1,2-diacylglycerol cholinephosphotransferase, an oxidase, a ketosphinganine reductase, a ceramide synthase, an acylglycerophosphorylcholine acyltransferase, an acylglycerol-phosphate acyltransferase, a phosphoethanolamine N-methyltransferase, a ceramide sphingobase desaturase, a glucosylceramide synthase, a acyl-ceramide synthase, a triacylglycerol lipase, a monoacylglycerol lipase, an acyl-CoA oxidase, an hydroxyacyl-CoA dehydrogenase, a dienoyl-CoA reductase, a fatty acid omega-alcohol oxidase, a monoacylglycerol lipase, an acyl-CoA oxidase, a hydroxyacyl-CoA dehydrogenase, a dienoyl-CoA reductase, a fatty acid omega-alcohol oxidase, a fatty acid/acyl-CoA transporter, a acyl-CoA dehydrogenase, a diacylglycerol-phosphate kinase, a lysophosphatidic acic phosphatase, a peroxygenase; a Δ4-desaturase; a Δ5-desaturase, a Δ6-desaturase; a Δ9-desaturase, a a Δ12-desaturase or a Δ*15-desaturase.

In a particular embodiment of the invention one chimeric protein may comprise a fatty acyl-CoA reductase and another chimeric protein may comprise a wax ester synthase such as a fatty acyl-CoA reductase with a substrate preference for fatty acyls within the range C16 to C18; and a wax ester synthase has a substrate preference for fatty alcohols and fatty acyl-CoA within the range C16-C18. Such fatty acyl-CoA reductase may derived from mouse and preferably has an amino acid sequence having about 80% sequence identity to the amino acid sequence of SEQ ID 6. Such wax ester synthase may be derived from mouse and preferably has an amino acid sequence having about 80% sequence identity to the amino acid sequence of SEQ ID 12.

Conveniently, the chimeric proteins may be expressed from one or more DNA constructs comprising the following operably linked DNA fragments:
a. A promoter functional in cells of said eukaryotic organism
b. A DNA region encoding said chimeric protein
c. A transcription termination and/or polyadenylation region.

The non-human eukaryotic organism may be selected from a plant, such as rapeseed (*Brassica spp.*), flax (*Linum usitatissimum*), safflower (*Carthamus tinctorius*), sunflower (*Helianthus annuus*), maize or corn (*Zeα mays*), soybean (*Glycine max*), mustard (*Brassica spp. and Sinapis alba*), crambe(*Crambe abyssinica),* eruca (*Eruca saiva*), oil palm (*Elaeis guineeis*), cottonseed (*Gossypium spp.*), groundnut (*Arachis hypogaea*), coconut (*Cocus nucifera*), castor bean (*Ricinus communis*), coriander (*Coriandrum sativum),* squash (*Cucurbita maxima*), Brazil nut (*Bertholletia excelsa*) or jojoba (*Simmondsia chinensis*), or an animal, a fungus or a yeast.

Suitable to the invention is a method for producing selected wax esters in plants comprising the steps of providing to cell of said plant organism at least two chimeric proteins wherein the first of said chimeric proteins comprises a first heterologous polypeptide targeting said protein to a subdomain of an organelle in said cell operably linked to a fatty-acyl CoA reductase and wherein the second of said chimeric proteins comprises a second heterologous polypeptide targeting said protein to said subdomain of said organelle in said cell operably linked to a wax ester synthase.

Also described herein is a composition of lipids derived from a plant obtained according to the methods herein described.

Yet another object of the invention is to provide a non-human eukaryotic organism comprising at least a first and a second DNA construct,
a. said first DNA construct comprising:
   i. a first promoter functional in cells of said eukaryotic organism
   ii. a first DNA region encoding a first chimeric protein comprising
      1. a DNA region encoding a first heterologous polypeptide targeting said proteins to a particular subdomain of an organelle in said cell; operably linked to
      2. a DNA region encoding a first polypeptide involved in fatty acid metabolism or lipid metabolism;
   iii. a transcription termination and/or polyadenylation region; and
b. said second DNA construct comprising
   iv. a second promoter functional in cells of said eukaryotic organism
   v. a second DNA region encoding a second chimeric protein comprising
      3. a DNA region encoding a second heterologous polypeptide targeting said proteins to said particular subdomain of an organelle in said cell; operably linked to
      4. a DNA region encoding a second polypeptide involved in fatty acid metabolism or lipid metabolism;
   vi. a transcription termination and/or polyadenylation region;
c. wherein said first and second heterologous polypeptide may be the same or different and wherein said first and second polypeptide involved in fatty acid metabolism or lipid metabolism are different polypeptides with a catalytic activity which actively contribute to the fatty acid metabolism or lipid metabolism, and wherein said first and second polypeptide involved in fatty acid metabolism or lipid metabolism act in a concerted manner.

Suitable is also a method to alter the subcellular location of an animal fatty-acyl CoA reductase comprising the steps of deleting the C-terminal amino acid sequences having the amino acid sequence of SEQ ID 2 from amino acid 467 to amino acid 515 or an amino acid sequence having at least 80% sequence identity thereto.

### Description of different embodiments of the invention

The current invention is based on the inventors observation that, when expressing in a eukaryotic organism, a first enzyme involved in lipid biosynthesis such as a fatty acyl CoA reductase and a second enzyme involved in lipid biosynthesis such as a wax ester synthase, which are supposed to act in a concerted manner to produce the desired wax esters, the amount of desired end product is much higher when both enzymes are fused to a polypeptide targeting the fused enzymes to a similar subdomain of the endoplasmatic reticulum (ER), such as the oleosin 3 polypeptide, than when the enzymes are not targeted to the same subdomain in the ER.

Accordingly, in a first embodiment, the invention provides a method for modulating lipid biosynthesis in a non-human eukaryotic organism comprising the step of providing to cells of the non-human eukaryotic organism at least one chimeric protein, but preferably a multitude of chimeric proteins wherein each of said chimeric proteins comprises a polypeptide involved in fatty acid metabolism or lipid metabolism operably linked to a heterologous polypeptide targeting the chimeric proteins to the same subdomain of an organelle, such as the ER.

As used herein "a polypeptide targeting the chimeric proteins to a subdomain of an organelle" refers to a polypeptide having a specific amino acid sequence which results in the location of the associated polypeptide involved in lipid or fatty acid biosynthesis to a subdomain of an organelle of the eukaryotic cell, such as a subdomain of the ER where (the relevant part of ) lipid or fatty acid biosynthesis is occurring. In doing so, the associated polypeptides involved in lipid or fatty acid biosynthesis are brought in the correct metabolic context, so that the desired end-product is produced in an efficient manner.

A "heterologous" polypeptide targeting the chimeric proteins to a subdomain of an organelle refers to the fact that the targeting polypeptide is not occurring naturally within the context of the polypeptide involved in lipid or fatty acid biosynthesis with which it is associated in the context of the current invention. In other words, a novel combination, not normally occurring in nature, is made between the targeting polypeptide and the polypeptide involved in lipid or fatty acid biosynthesis.

In one embodiment of the invention, the heterologous targeting polypeptide may comprise the amino acid sequence of an oleosin.

Oleosin are proteins associated with oil bodies, wherein triacylglycerides (TAG) are sequestered. Oil bodies (OB) are spherical organelles 0.2-2.0 mm in diameter with a simple structure consisting of a TAG core encased in a half-unit membrane consisting of phospholipids and a few different proteins of which oleosin form the majority. The sequence/structure of oleosins consists of three distinct domains: first, an amino-terminal domain, which may be either hydrophilic or hydrophobic; second, a central 70-77 amino acid hydrophobic domain with a conserved proline knot in the center of the domain; third, a carboxyterminal amphipathic domain of variable length. The signature characteristic of the oleosins is that they all possess the central hydrophobic domain. The central hydrophobic region constitutes the longest continuous region of hydrophobic amino acids known in any protein and is unique to these proteins. This hydrophobic domain has been modeled as an anti-parallel helical structure anchored in the TAG core with the proline knot reversing the direction of the polypeptide. The C-terminal amphipathic domain is largely conserved as an amphipathic alpha-helical structure but the sequence of this domain is highly variable. Oleosin proteins from one species will correctly associate with forming OBs of another species; for example, the soybean oleosin gene transferred into Brassica napus resulted in soybean oleosin being correctly expressed in seed development and the soybean oleosin protein was inserted into the Brassica OBs as a mixed population of proteins with the intrinsic Brassica oleosin.

Oleosins suitable for the purpose of the invention are known in the art and the following is a list of amino acid sequence and nucleotide sequences encoding such amino acid sequences: A84654 Arabidopsis thaliana probable oleosin; AAA87295 Arabidopsis thaliana oleosin (Gene L40954); AAC42242 Arabidopsis thaliana oleosin (Gene AC005395);AAF015421 Arabidopsis thaliana putative oleosin (Gene AC009325); AAF 697121 Arabidopsis thaliana F27J15.22 (Gene AC016041); AAK96731 Arabidopsis thaliana oleosin-like protein (Gene AY054540); AAL143 85 Arabidopsis thaliana AT5g 40420 / MPO12_130 oleosin isoform (Gene AY057590); AAL24418 Arabidopsis thaliana putative oleosin (Gene AY059936); AAL473566 Arabidopsis thaliana oleosin-like protein (Gene AY064657); AAM10217 Arabidopsis thaliana putative oleosin (Gene AY081655); AAM47319 Arabidopsis thaliana AT5g40420/MP012_130 olesoin isoform (Gene AY113011); AAM63098 Arabidopsis thaliana oleosin isoform (Gene AY085886); AAO22633 Arabidopsis thaliana putative oleosin (Gene BT002813); AAO22794 Arabidopsis thaliana putative oleosin protein (Gene BT002985); AAO42120 Arabidopsis thaliana putative oleosin (Gene BT004094); AAO50491 Arabidopsis thaliana putative oleosin (Gene BT004958); AAO63989 Arabidopsis thaliana putative oleosin (Gene BT005569); AAQ22658 Arabidopsis thaliana At4g25140 (Gene BT010189.1); AAQ56108 Arabidopsis lyrata susp. Lyrata Oleosin (Gene AY292860); BAA97384 Arabidopsis thaliana oleosin-like (Gene AB023044); BAB02690 Arabidopsis thaliana olcosin-like protein (Gene AB018114); BAB11599 Arabidopsis thaliana oleosin isoform 21K (Gene AB006702); BAC42839 Arabidopsis thaliana putative oleosin protein (Gene AK118217); BAD94320 Arabidopsis thaliana oleosin (Gene AK220898.1); CAA44225 Arabidopsis thaliana oleosin (Gene X62353); CAA63011 Arabidopsis thaliana oleosin type 4 (Gene X91918); CAA63022 Arabidopsis thaliana oleosin type 2 (Gene X91956); CAA90877 Arabidopsis thaliana oleosin (Gene Z54164); CAA90878) Arabidopsis thaliana oleosin (Gene Z54165); CAB36756 Arabidopsis thaliana oleosin 18.5 K (Gene AL035523); CAB79243 Arabidopsis thaliana oleosin, 18.5 K (Gene AL161562); CAB87945 Arabidopsis thaliana oleosin-like Protein (Gene ALI63912); P29525 Arabidopsis thaliana oleosin 18.5 kDa (Gene X62353, CAA44225,AL0355 23, CAB36756, CAB79423, Z17738, S22538); Q39165 Arabidopsis thaliana Oleosin 21.2kDa (Oleosin type 2) (Gene L40954, AAA87295, X91956, CAA63022, Z17657, AB006702, BAB11599,AY057590, AAL14385, S71253); Q42431 Arabidopsis thaliana oleosin 20.3 kDa (Oleosin type 4) (Gene Z54164, CAA90877, X91918, CAA63011, AB018114, BAB02690, AY054540, AAK96731, AY064657, A.AL47366, AY085886, AAM 63098, Z27260, Z29859, S71286); Q43284 Arabidopsis thaliana Oleosin 14.9 kDa. (Gene Z54165, CAA90878, AB023044, BAA97384, Z27008, CAA81561);S22538 Arabidopsis thaliana oleosin 18.5K; S751253 oleosin, 21K; S71286 Arabidopsis thaliana 20K; T49895 Arabidopsis thaliana oleosin like protein; AAB22218 Brassica napus oleosin napII; AAB22219 Brassica napus oleosin napI; AAD24547 Brassica napus oleosin; AAK38471 Brassica napus oleracea putative oleosin (Gene AY028608.1); AAK38472 Brassica oleracea putative oleosin (Gene AY028608.1) ; AAK38473 Brassica oleracea putative oleosin (Gene AY028608.1); AAK38474 Brassica oleracea putative oleosin (Gene AY028608.1); AAK38475 Brassica oleracea putative oleosin (Gene AY028608.1); AAW70038 Brassica rapa oleosin-like protein (Gene AY747625.1); CAA41064 Brassica napus oleosin Nap-II( Gene X58000.1); CAA43941 Brassica napus oleosin BN-III (Gene X63779); CAA45313 Brassica napus oleosin BN-V (Gene X63779); CAA57544 Brassica napus oleosin (Gene X82019.1); CAA57545 Brassica napus oleosin (X82020.1); CAA64800 Brassica napus oleosin-like protein (Gene X95554.1); CAA64801 Brassica napus oleosin -like protein (Gene X95555.1); CAA64802 Brassica napus oleosin-like protein (Gene X95556.1); CAA64803 Brassica napus oleosin-like protein (Gene X95557.1); CAA64804 Brassica napus oleosin-like protein (Gene X95558.1); CAA64805 Brassica napus oleosin-like protein (Gene X95559.1); CAA64806 Brassica napus oleosin-like protein (Gene X95560.1); CAA70173 Brassica napus oleosin-like protein (Gene Y08986.1); P29109 Brassica napus oleosin Bn_V (Gene X63779, CAA45313, S25089); P29110 Brassica napus oleosin Bn-III (Gene X61937, CAA43941, S22475); P29111 Brassica napus oleosin NAP-II (Gene X58000, CAA41064, S70915); P29526 Brassica napus oleosin C98 (Gene X67142.1, CAA47623.1; S24960); S13494 Brassica napus oleosin NAP-I; S22475 Brassica napus oleosin BN-III; S25089 Brassica napus oleosin BN-IV; S50195 Brassica napus oleosin; S70915 Brassica napus oleosin Nap-II; T08134 Brassica napus oleosin; 1803528A Brassica napus oleosin; 2009397 Brassica napus oleosin; AAB01098 Daucus carota oleosin; T14307 carrot oleosin; A35040 Zea mays oleosin 18; AAA67699 Zea mays oleosin KD18 (Gene J05212); AAA68065 Zea mays oleosin 16 Kda (Gene U13701); AAA68066 Zea mays oleosin 16 Kda (Gene U13702); P13436 Zea mays oleosin ZM-1 (oleosin 16kD) (Gene U 3701, AAA68065, M17225, AAA33481, A29788); P21641 Zea mays oleosin Zm-II (oleosin 18 KDa)(Gene J05212, AAA67699, A35040); S52029 Zea mays oleosin 16; S52030 Zea mays oleosin 17..

In another embodiment of the invention, the heterologous targeting polypeptide may comprise the amino acid sequence of the N-terminal domain of LBLOX (lipid body lipoxygenase) or the N-terminal domain of PLA (phospholipase A2) as described in WO01/2922.

A suitable heterologous targeting polypeptide may comprise the amino acid sequence of the ER retrieval sequences as described in Dyer and Mullen, 2008 (Physiologia Plantarum 132: 11-22). One such ER retrieval sequence C-terminal dilysine ER retrieval motif as can be found in FAD3 polypeptides. Another ER retrieval sequence comprises a C-terminal aromatic-rich motif similar to a 1- WxxxW - motif. Yet another ER retrieval sequence comprises the consensus sequence consisting of Φ-x-x-K/R/D/E-Φ-COOH, where Φ is any large, hydrophobic amino acid and -x- is any amino acid.

The heterologous targeting polypeptide may also comprise an ER retention signal (K/HDEL*), or an ER-retrieval signal for membrane bound proteins (KKxx*) or -KK-COOH or -KKXX-COOH or -KXKXX-COOH motif. The heterologous targeting polypeptide may also comprise to the transmembrane segment of α-2,6-sialyltransferase (particularly the first 44 or 52 amino acids thereof; Munro et al. 1991, EMBO Journal, 10: 3577-3588); the signal anchor sequence from human galactosyl transferase (particularly the first 60 amino acids thereof) or the signal anchor sequence from the Arabidopsis homologue of the yeast HDEL receptor (AtERD2) (Saint-Jore et al., 2002, The Plant Journal, 29: 661-678), the signal anchor sequence from β1,2-xylosyltransferase protein (particularly the first 36 amino acids thereof; Pagny et al., 2003, The Plant Journal 33: 189-203) or the signal anchor sequences of N-acetyl-glucosaminyl transferase I (particularly the first 77 amino acids thereof; Essl et al. 1999, FEBS Lett. 453:169-173).

In one embodiment of the invention, the heterologous targeting polypeptide may be a polypeptide having at least 80% sequence identity to oleosin 3 of Arabidopsis thaliana (SEQ ID No 10 from amino acid 1 to amino acid 143). It will be clear that a polypeptide having at sequence identity of more than at least 80%, such as 85%, 90%, 95% or 100% can also be used to the same effect.

For the purpose of this invention, the "sequence identity" of two related nucleotide or amino acid sequences, expressed as a percentage, refers to the number of positions in the two optimally aligned sequences which have identical residues (x100) divided by the number of positions compared. A gap, i.e. a position in an alignment where a residue is present in one sequence but not in the other, is regarded as a position with non-identical residues. The alignment of the two sequences is performed by the Needleman and Wunsch algorithm (Needleman and Wunsch 1970). The computer-assisted sequence alignment above, can be conveniently performed using standard software program such as GAP which is part of the Wisconsin Package Version 10.1 (Genetics Computer Group, Madision, Wisconsin, USA) using the default scoring matrix with a gap creation penalty of 50 and a gap extension penalty of 3.

It will be clear to the skilled artisan that the heterologous targeting peptide present in the different chimeric proteins may be either the same or may be different, provided that it targets the chimeric proteins to the same suborganelle domain in the eukaryotic cell.

It will also be clear to the skilled artisan that it may be sufficient to provide only one chimeric gene comprising a heterologous targeting polypeptide operably linked to a polypeptide involved in fatty acid or lipid biosynthesis, if the other components of the multitude of proteins involved in lipid or fatty acid metabolism provided to the eukayotic cells already comprise an endogenous targeting polypeptide targeting that other protein to the same subdomain of the organelle.

As used herein "a polypeptide involved in fatty acid metabolism or lipid metabolism" refers to a polypeptide, preferably a polypeptide with catalytic activity, which is actively contributes to the fatty acid metabolism or lipid metabolism (i.e. biosynthesis or degradation) in an organism. Such polypeptides include but are not limited to an acyl-CoA synthetase, a glycerol-phosphate acyltransferase, an O-acyltransferase, a lyso-phosphatidic acid acyltransferase, a phosphatidic acid phosphatase, a diacylglycerol acyltransferase, an oleate desaturases, a linoleate desaturases, an acyl-CoA hydroxylase, an acyl-lipid hydroxylase, a fatty acid epoxidase, a phospholipid:sterol acyltransferase, a phospholipid:diacylglycerol acyltransferase, a diacylglycerol transacylase, a lysophosphatidylcholine acyltransferase, a phosphatidylcholine:diacylglycerol cholinephosphotransferase, an acyl-CoA elongase, an acyl-lipid elongase, a phosphatidylglycerol-phosphate synthetase, a phosphatidylglycerol-phosphate phosphatase, a CDP-diacylglycerol synthetase, a phosphatidylinositol synthase, a phosphatidylserine synthase, a choline kinase, an ethanolamine kinase, a CDP-choline synthetase, a CDP-ethanolamine synthetase, a phosphatidylserine decarboxylase, a lipoxygenase, a phospholipase, a lipase, a carboxylesterase, a fatty alcohol reductase, a wax ester synthase, a bifunctional acyltranferases/wax synthase, a ketoacyl-CoA synthase, a ketoacyl-CoA reductase, a hydroxylacyl-CoA dehydrase, an enoyl-CoA reductase, an alcohol-forming fatty acyl-CoA reductase, an aldehyde-forming fatty acyl-CoA reductase, an aldehyde decarbonylase, a wax ester hydrolase, a glycerol-3-P-dehydrogenase, a CDP-choline: 1,2-diacylglycerol cholinephosphotransferase, an oxidase, a ketosphinganine reductase, a ceramide synthase, an acylglycerophosphorylcholine acyltransferase, an acylglycerol-phosphate acyltransferase, a phosphoethanolamine N-methyltransferase, a ceramide sphingobase desaturase, a glucosylceramide synthase, a acyl-ceramide synthase, a triacylglycerol lipase, a monoacylglycerol lipase, an acyl-CoA oxidase, an hydroxyacyl-CoA dehydrogenase, a dienoyl-CoA reductase, a fatty acid omega-alcohol oxidase, a monoacylglycerol lipase, an acyl-CoA oxidase, a hydroxyacyl-CoA dehydrogenase, a dienoyl-CoA reductase, a fatty acid omega-alcohol oxidase, a fatty acid/acyl-CoA transporter, a acyl-CoA dehydrogenase, a diacylglycerol-phosphate kinase, a lysophosphatidic acic phosphatase, a peroxygenase; a Δ4-desaturase; a Δ5-desaturase, a Δ6-desaturase; a Δ9-desaturase, a Δ12-desaturase or a Δ15-desaturase.

These polypeptides are well known in the art and may be derived from animal sources, plant sources, bacterial sources, algal sources or fungal sources. Enzymes relevant for the production of polyunsaturated fatty acids may be the ones described in WO2006/064317, WO2008/104559, WO2008/076987, WO2007/136877 or WO2007/106728. Enzymes relevant for modulating oil content include Arabidopsis DGAT1 described in patent application PCT/CA99/01202, Tropaeolum DGAT1 described in patent application pct/ca2007/001225, Umbelopsis ramanniana DGAT2; diacylglycerol acyltransferase 2 genes and proteins encoded thereby from algae described WO2009/085169; pyruvate kinase genes described in WO2008/135467, plant sucrose synthase like proteins described in WO2006/133166 or yeast glycerol-3-phosphate dehydrogenase genes described in WO2003/095655.

In one embodiment of the invention, one of the polypeptides involved in fatty acid or lipid metabolism comprise a fatty acyl-CoA reductase and another comprises a wax ester synthase. As used herein, a fatty acyl-Co reductase is an enzyme reducing a fatty acyl CoA to the corresponding fatty alcohol using electrons from NADPH cofactor, and thereby cleaving the thioester bond in the fatty acyl-CoA. Fatty acyl-CoA reductases having a substrate preference for C12-C20 fatty acyl-CoA molecules are especially suited for the methods according to the invention. As an example, the current invention employs fatty acyl-CoA reductase from mouse. Accordingly, one of the chimeric proteins may comprise a polypeptide involved in fatty acid or lipid biosynthesis comprising an amino acid sequence having at least 80% sequence identity to the amino acid sequence of SEQ ID No 2. During the experiment leading to the current invention, the inventors have observed that the mouse fatty acyl-CoA reductase comprises a C-terminal domain (amino acids 467 to 515 of SEQ ID No 2) which is involved in the location of the mouse fatty acyl-CoA reductase to the peroxisomes and which can be deleted (and preferably should be deleted when retargeting the fatty acyl-CoA reductase in accordance with the invention) without interfering with the catalytic activity of the fatty acyl-CoA reductase. Thus, in another embodiment of the invention one of the chimeric proteins comprises a polypeptide involved in fatty acid or lipid biosynthesis comprising an amino acid sequence having at least 80% sequence identity to the amino acid sequence of SEQ ID No 6. Again, it will be clear that a polypeptide having at sequence identity of more than at least 80%, such as 85%, 90%, 95% or 100% can also be used to the same effect.

As used herein, a wax ester synthase is an enzyme catalyzing the trans-esterification of a fatty alcohol and a fatty acyl-CoA producing a wax monoester. CoASH is released and the energy linked to the thioester bond is used to create the new ester linkage.

In higher plants, mammals and bacteria, ester biosynthesis is catalyzed by one of three classes of wax synthase (WS) enzymes: jojoba-type WS, mammalian WS, and WS/diacylglycerol O-acyltransferases (DGAT) bifunctional enzymes (Jetter and Kunst, 2008). Jojoba-type WS uses a wide range of saturated and unsaturated acyl CoAs ranging from C14 to C24, with 20:1 as the preferred acyl and 18:1 as the preferred alcohol substrate (Lardizabal et al., 2000). Mammalian WS enzymes do not have homologues in plants, and have highest activities with C12-C16 acyl-CoAs and alcohols shorter than C20 (Cheng and Russell, 2004b). It is generally accepted that WS are localized to the ER membrane. WS catalyzes the transesterification of a fatty alcohol and a fatty acyl-CoA. The mouse WS in particular is found in the preputial glands and the eyelids and prefers mainly C16:0, C18:1, andC18:2 fatty alcohols and fatty acyl-CoAs in the following order: C16:1 > C18:1 > C16:0 > C20:0 > C14:0. This shows, that wax monoesters emerging in mouse cells are notably short and therefore of great industrial interest (see 1.3; Cheng and Russell, 2004b; Lassner et al.,1999). In one embodiment of the invention, the wax ester synthase may comprise an amino acid sequence having at least 80% sequence identity to the amino acid sequence of SEQ ID No 12.

The chimeric proteins according to the invention are conveniently provided to the non-human eukaryotic cells by expressing them from one or more DNA constructs comprising a promoter functional in cells of said non-human eukaryotic organism operably linked to a DNA region encoding the chimeric protein and a transcription termination and/or polyadenylation region. It will be clear to the skilled artisan that the DNA constructs may be organized as an operon whereby the expressed (i.e. the transcribed and translated region) may be under control of a single promoter, or as separate transcription units, whereby each coding DNA region is under control of a single promoter.

Said promoter may be a plant expressible promoter. As used herein, the term "plant-expressible promoter" means a DNA sequence which is capable of controlling (initiating) transcription in a plant cell. This includes any promoter of plant origin, but also any promoter of non-plant origin which is capable of directing transcription in a plant cell, i.e., certain promoters of viral or bacterial origin such as the CaMV35S (Harpster et al., 1988 Mol. Gen. Genet. 212, 182-190), the subterranean clover virus promoter No 4 or No 7 (WO9606932), or T-DNA gene promoters but also tissue-specific or organ-specific promoters including but not limited to seed-specific promoters (e.g., WO89/03887), organ-primordia specific promoters (An et al., 1996, The Plant Cell 8, 15-30), stem-specific promoters (Keller et al., 1988, EMBO J. 7, 3625-3633), leaf specific promoters (Hudspeth et al., 1989, Plant Mol Biol 12, 579-589), mesophyl-specific promoters (such as the light-inducible Rubisco promoters), root-specific promoters (Keller et al.,1989, Genes Devel. 3, 1639-1646), tuber-specific promoters (Keil et al., 1989, EMBO J. 8, 1323-1330), vascular tissue specific promoters (Peleman et al., 1989, Gene 84, 359-369), stamen-selective promoters (WO89/10396, WO92/13956), dehiscence zone specific promoters (WO 97/13865) and the like.

Seed specific promoters are well known in the art, including the USP promoter from Vicia faba described in DE10211617; the promoter sequences described in WO2009/073738; promoters from Brassica napus for seed specific gene expression as described in WO2009/077478; the plant seed specific promoters described in US2007/0022502; the plant seed specific promoters described in WO03/014347; the seed specific promoter described in WO2009/125826; the promoters of the omega_3 fatty acid desaturase family described in WO2006/005807 and the like.

Methods to obtain transgenic plants are not deemed critical for the current invention and any transformation method and regeneration suitable for a particular plant species can be used. Such methods are well known in the art and include Agrobacterium-mediated transformation, particle gun delivery, microinjection, electroporation of intact cells, polyethyleneglycol-mediated protoplast transformation, electroporation of protoplasts, liposome-mediated transformation, silicon-whiskers mediated transformation etc. The transformed cells obtained in this way may then be regenerated into mature fertile plants.

The obtained transformed plant can be used in a conventional breeding scheme to produce more transformed plants with the same characteristics or to introduce the chimeric gene according to the invention in other varieties of the same or related plant species, or in hybrid plants. Seeds obtained from the transformed plants contain the chimeric genes of the invention as a stable genomic insert and are also encompassed by the invention.

The methods and means described herein are believed to be suitable for all plant cells and plants, both dicotyledonous and monocotyledonous plant cells and plants including but not limited to cotton, Brassica vegetables, oilseed rape, wheat, corn or maize, barley, sunflowers, rice, oats, sugarcane, soybean, vegetables (including chicory, lettuce, tomato), tobacco, potato, sugarbeet, papaya, pineapple, mango, Arabidopsis thaliana, but also plants used in horticulture, floriculture or forestry. Especially suited are oil producing plants such as rapeseed (*Brassica spp.),* flax (*Linum usitatissimum*), safflower (*Carthamus tinctorius),* sunflower (*Helianthus annuus*), maize or corn (*Zea mays*), soybean (*Glycine max*), mustard (*Brassica spp. and Sinapis alba*)*,* crambe(*Crambe abyssinica*), eruca (*Eruca saiva*), oil palm (*Elaeis guineeis),* cottonseed (*Gossypium spp.),* groundnut (*Arachis hypogaea),* coconut (*Cocus nucifera),* castor bean (*Ricinus communis*)*,* coriander (*Coriandrum sativum*), squash (*Cucurbita maxima*), Brazil nut (*Bertholletia excelsa*) or jojoba (*Simmondsia chinensis)* gold-of-pleasure (*Camelina sativa*), purging nut (*Jatropha curcas*), *Echium spp.,* calendula (*Calendula officinalis),* olive *(Olea europaea*), wheat *(Tritium spp.),* oat *(Avena spp.),* rye (*Secale cereale*), rice (*Oryza* sativa), Lesquerella *spp., Cuphea spp*., meadow foam (*Limnanthes alba*)*,* avocado (*Persea Americana*), hazelnut (*Corylus*), sesame (*Sesamum indicum*), safflower (*Carthamus tinctorius*), tung tree (Aleurites fordii), poppy (*Papaver somniferum*) tobacco (*Nicotiana spp.*)*.*

The methods and means described herein can also be used in algae such as *Scenedesmus dimorphus, Euglena gracilis, Phaeodactylum tricornutum*, *Pleurochrysis carterae, Prymnesium parvum*, *Tetraselmis chui, Tetraselmis suecica, Isochrysis galbana, Nannochloropsis salina, Botryococcus braunii, Dunaliella tertiolecta, Nannochloris spp. or Spirulina spp.*

As used herein "comprising" is to be interpreted as specifying the presence of the stated features, integers, steps or components as referred to, but does not preclude the presence or addition of one or more features, integers, steps or components, or groups thereof. Thus, e.g., a nucleic acid or protein comprising a sequence of nucleotides or amino acids, may comprise more nucleotides or amino acids than the actually cited ones, i.e., be embedded in a larger nucleic acid or protein. A chimeric gene comprising a DNA region which is functionally or structurally defined, may comprise additional DNA regions etc.

The following examples describe the relocalization of mouse FAR and mouse WS to a similar subdomain of the ER and the improved production of the desired wax esters.

Unless stated otherwise in the Examples, all recombinant techniques are carried out according to standard protocols as described in "Sambrook J and Russell DW (eds.) (2001) Molecular Cloning: A Laboratory Manual, 3rd Edition, Cold Spring Harbor Laboratory Press, New York" and in "Ausubel FA, Brent R, Kingston RE, Moore DD, Seidman JG, Smith JA and Struhl K (eds.) (2006) Current Protocols in Molecular Biology. John Wiley & Sons, New York".

Standard materials and references are described in "Croy RDD (ed.) (1993) Plant Molecular Biology LabFax, BIOS Scientific Publishers Ltd., Oxford and Blackwell Scientific Publications, Oxford" and in "Brown TA, (1998) Molecular Biology LabFax, 2nd Edition, Academic Press, San Diego". Standard materials and methods for polymerase chain reactions (PCR) can be found in "McPherson MJ and Møller SG (2000) PCR (The Basics), BIOS Scientific Publishers Ltd., Oxford" and in "PCR Applications Manual, 3rd Edition (2006), Roche Diagnostics GmbH, Mannheim or www.roche-applied-science.com.

In the description and examples, reference is made to the following sequences:

| | |
|---|---|
| SEQ ID No. 1: | mouse fatty acyl-CoA reductase - nucleotide sequence |
| SEQ ID No. 2: | mouse fatty acyl-CoA reductase - amino acid sequence |
| SEQ ID No. 3: | mouse fatty acyl-CoA reductase tagged with mCherry-nucleotide sequence |
| SEQ ID No. 4: | mouse fatty acyl-CoA reductase tagged with mCherry- amino acid sequence |
| SEQ ID No. 5: | mouse fatty acyl-CoA reductase truncated - nucleotide sequence |
| SEQ ID No. 6: | mouse fatty acyl-CoA reductase truncated - amino acid sequence |
| SEQ ID No. 7: | mouse fatty acyl-CoA reductase truncated tagged with mCherry- nucleotide sequence |
| SEQ ID No. 8: | mouse fatty acyl-CoA reductase truncated tagged with mCherry- amino acid sequence |
| SEQ ID No. 9: | mouse fatty acyl-CoA reductase truncated tagged with mCherry and fused to oleosin- nucleotide sequence |
| SEQ ID No. 10: | mouse fatty-acyl CoA reductase truncated tagged with mCherry and fused to oleosin- amino acid sequence |
| SEQ ID No. 11: | wax ester synthase from mouse -nucleotide sequence |
| SEQ ID No. 12: | wax ester synthase from mouse -amino acid sequence |
| SEQ ID No. 13: | wax ester synthase from mouse tagged with YFP -nucleotide sequence |
| SEQ ID No. 14: | wax ester synthase from mouse tagged with YFP -amino acid sequence |
| SEQ ID No. 15: | wax ester synthase from mouse tagged with YFP fused to oleosin -nucleotide sequence |
| SEQ ID No. 16: | wax ester synthase from mouse tagged with YFP fused to oleosin -amino acid sequence |

### Examples

### Example 1. Materials and Methods

For all methods sterile pipette tips and reaction tubes were used. All solutions were set up with sterile water (ddH2O).

### Hardware/Equipment

ABI PRISM® 3100 Genetic Analyzer Applied Biosystems, Foster, USA
Automatic TLC Sampler 4 Camag, Berlin, Germany
Centrifuge 5810 R Eppendorf AG, Hamburg, Germany
Centrifuge 5415 D Eppendorf AG, Hamburg, Germany
Centrifuge 5417 R Eppendorf AG, Hamburg, Germany
Chromatogramm Immersion Devise III Camag, Berlin, Germany
ColorView II 3.3 MegaPixel CCD Camera Olympus, Hamburg, Germany
Gel-Detection System DIANA Raytest, Straubenhardt, Germany
Gel-Detection System AIDA Raytest, Straubenhardt, Germany
Glass beads Roth, Karlsruhe, Germany
Gold particles BioRad, Hercules, USA
Mastercycler personal Eppendorf, Wesseling-Berzdorf, Germany
Olympus BX51 Olympus, Hamburg, Germany
Olympus U-RFL-T Olympus, Hamburg, Germany
PDS1000/He Biolistic Particle Delivery System BioRad, Hercules, USA
TLC Plate heater III Camag, Berlin, Germany
TLC Silica Gel 60 20x20 cm Merck, Darmstadt, Germany
Ultrospec 1100pro Amersham Biosciences, Freiburg, Germany
3 MM-paper Whatman, Madistone, Kent, UK
6890 Series GC System Agilent, Waldbronn, Germany
Computer programs: analysis wincat

### Chemicals

Unless otherwise indicated all chemicals were obtained from either, Sigma-Aldrich (Munich, Germany) or Roth (Karlsruhe, Germany). Organic solvents such as n-hexane, methanol, ethanol, acetonitrile or diethyl ether were obtained from Acros, Geel, Netherlands or Baker, Griesheim, Germany.

### Fatty Acid and Lipid Standards

Internal standard for fatty acid quantification:
1,2,3-triheptadecanoyl glyceryl Sigma-Aldrich (München, Germany)

Standards for thin layer chromatography:
Triacylglycerol/Diacylglycerol-mixture Sigma-Aldrich (München,Germany)
Palmitate Sigma-Aldrich (München,Germany)
Palmityl alcohol Sigma-Aldrich (München,Germany)
Palmityl palmitate Sigma-Aldrich (München,Germany)
Sterol esters kindly provided by Sabine Freitag and Dr. Cornelia Göbel

### Enzymes

### Restriction Enzymes

The restriction enzymes XhoI, EcoRI, BamHI, SailI, HindIII, NotI and SacII were purchased from MBI Fermentas (St. Leon-Rot, Germany) and the restriction enzyme Bsp1407I was obtained from NewEngland Biolabs (Ipswich, UK). All enzymes were used according to the manufacturer's protocol.

### Other Enzymes

T4-DNA-Ligase Fermentas, St.-Leon Roth, Germany
PhusionTM DNA-Polymerase Finnzymes, Espoo, Finland
Gateway®LR ClonaseTM II Enzym Mix Invitrogen, Karlsruhe, Germany

### Kits and Systems

Macherey-Nagel NucleoSpin®Plasmid Macherey-Nagel, Düren, Germany
NucleoSpin® Extract Kit Macherey-Nagel, Düren, Germany
CompactPrep® Plasmid Midi Core Kit Qiagen, Hilden, Germany
ABI PRISM® BigDyeTM Terminator Foster City, CA, USA
Cycle Applied Biosystems
Sequencing Ready Reaction Kit v1.1 Invitrogen, Karlsruhe, Germany
ProQuestTM Two-Hybrid System Invitrogen, Karlsruhe, Germany

### Oligonucleotides (Primers)

### Primer for Cloning Primer sequences

FAR2-EcoRI-for 5'-GGATGCGAATTCATGAGCATGATCGCAGCTTTC-3'
FAR2-XhoI-rev 5'-GGATGCCTCGAGTTAGACCTTCAAAGTACTGGA-3'
FAR2c-XhoI-rev 5'-GGATGCCTCGAGTTAATGTATGTTACGCAAACGCC-3'
FAR2g.for.EcoRI 5'-GGCCATGCGAATTCATGATACATTATCTTTTTAATACTG - 3'
FAR2g.rev.XhoI 5'-GGCCATGCCTCGAGTTAGACCTTCAAAGTACTGGAGGC-3'
FAR 1 -EcoRI-for 5'-GGATGCGAATTCATGGTTTCCATCCCAGAGTAC-3'
FAR 1-rcv-XhoI 5'-ATGCCTCGAGTTAGTATCGCATTGTGGAAGAGGC-3'
FAR 1 c-XhoI-rev 5'-GGATGCCTCGAGTTATCTGATGTTGCGAAGCTTGTT-3'
mCherry-for-BamHI 5'-ATGCGGATCCATGGTGAGCAAGGGCGAGGAGGAT-3'
mCherry-rev-EcoRI-stop 5'-ATGCGAATTCCTTGTACAGCTCGTCCATGCCGCC-3'

### Primer for sequencing Primer sequences

M13 uni 5'-CCCAGTCACGACGTTGTAAAACG-3'
M13rev 5'-AGCGGATAACAATTTCACACAGG-3'
mCherry-mitte.for, 5'-CACTACGACGCTGAGGTCAAGA-3'
FAR2-mitte-for 5'-GAGGTCTATTAAGGCTACTC-3'
FAR2-mitte-rev 5'-GAGTAGCCTTAATAGACCTC-3'
MmWS-mitte-for 5'-CTATGGACTTCTTGCTTAC-3'
MmWS-mitte-for 5'-GTAAGCAAGAAGTCCATAG-3'

### Plasmids

| For plant constructs: | |
|---|---|
| pUC18-ENTRY2 | AmpR, modified pUC18 vector containing attL1 and attL2 Gateway cloning sites (modified and provided by Dr. Ellen Hornung;.Hoffmann et al., 2007) |
| pCAMBIA3300 | KanR in bacteria, Glufosinate (BastaR) in plants under control of the CaMV 35S-promotor. The vector includes two recognition sites, namely *attR*1 and *attR*2, which enables insertion of the DNA fragment due to homologous recombination. It is inserted in reading direction of the 35S-promotor. (Modified and provided by Dr. Ellen Hornung) |
| Yeast expression vector: | |
| pESC-URA | AmpR (Stratagene, Amsterdam, Netherlands) |

### Organisms

### Bacteria strains

| | |
|---|---|
| *Escherichia coli* XL1blue | Genotype: endA1, hsdR17, supE44, thi-1, rec A1, gyrA96, relA1, lac, [F', proAB, laclqZΔ15, Tn10(tetR)] (Stratagene, Amsterdam, Netherlands) |

### Yeast strains

| | |
|---|---|
| *Saccharomyces cerevisiae* INVSc1 | Genotype: his3Δ1/his3Δ1, leu2/leu2, trp1-289/trp1-289, ura3-52/ura3-52 (Invitrogen, Karlsruhe, Deutschland) |
| *Saccharomyces cerevisiae* W303 H1246 | Genotype: MATα ADE2-1 can1-100 ura 3-1 are 1-Δ::HIS3 are2-Δ::LEU2, dga1-Δ::KanMX4 lro1-Δ::TRP1(Sten Styme (Scandinavian Biotechnology Research, Alnarp, Sweden) |
| Saccharomyces cerevisiae MaV103 | Genotype: MATα, leu2-3,112, trp1-901,his3Δ200, ade2-101, gal4Δ, gal80Δ, SPAL10::URA3, GAL1::lacZ, HIS3UAS GAL1::HIS3@LYS2, can1R, cyh2R) (Invitrogen, Karlsruhe, Germany) |

### Plants

### Allium cepa (Onion)

### Culture media

For producing solid media 1.5% Micro-Agar (Duchefa, Haarlem, Netherlands) was added to bacteria media; 2 % were added to yeast media. All media were autoclaved after solving.

### Medium for E. coli

LB (Luria and Bertani) medium Contains 1% (w/v) peptone, 0.5% (w/v) yeast extract and 1% (w/v) NaCl. pH value was adjusted with NaOH to pH 7.

### Yeast media

SD medium 5 g/L ammonium sulfate, 1.7 g/l YNB w/o ammonium sulfate; add sterile water to 1 L

### Dropout powder:

Ingredients Amount (g)
L-Arginine (HCl) 2
L-Histidine (HCl) 2
L-Isoleucine 2
L-Lysine (HCl) 2
L-Methionine 2
L-Phenylalanine 2
L-Serine 2
L-Threonine 2
L-Tyrosine 2
L-Valine 9

The different substances were mixed and ground to a fine powder. Depending on the selection of the vector, either uracil, leucine and/or tryptophan (2g each) were added separately to the powder. YPD medium 1% (w/v) yeast extract, 2% (w/v) peptone and 2% (w/v) glucose.

### Media supplements for selection

### antibiotics solved in stock solution end concentration

kanamycin ddH2O50 mg/ml 25 µg/l
carbenicillin ddH2O 100 mg/ml 100 µg/l
gentamicin ddH2O 50 mg/ml 50 µ/l

### Transformation of E. coli

For transformation of *E. coli* the strain XL1blue was used. This strain is known to be suited for heat shock transformation as performed.

### Preparation of competent cells

The preparation of competent *E. coli* cells was accomplished after the method established by (Inoue et al., 1990).

### Transformation of competent E. coli cells

For transformation competent cells were gently thawn on ice. Then 100 µl of cell suspension were added to either a 10 µl ligation reaction or to 1 µl of plasmid DNA for retransformation of vector constructs. The reaction were incubated on ice for 30 minutes and further put to 42 °C for 30 seconds. Afterwards, cells were placed on ice again for roughly 3 minutes. 900 µl of LB medium were added to the samples and those were incubated at 37 °C for one hour while shaking. After that, cells were spun down at 3000 rpm, supernatant was discarded and the pellet was resuspended. Cells were plated on solid LB medium containing the specific antibiotic for selection and incubated overnight at 37°C.

### Transformation of S. cerevisiae

2 ml of YPD medium were inoculated with colonies of either the INVSc1 or the H1246 yeast strain. The pre-cultures were incubated overnight at 30 °C while shaking at 200 rpm. The next day, 50 ml of YDP-medium were inoculated with the pre-culture to an optical density at 600 nm (OD600) of 0.15 and grown at 30 °C to an OD600 of 0.6-0.7. Cells were sedimented by centrifugation (700 g at room temperature), the cells were washed with 20 ml 1x TE-buffer (100 mM Tris-HCl, 10 mM EDTA, pH 8), afterwards resuspended in 1 ml 1x LiAc/1xTE and incubated for 10 minutes at room temperature. 5 µl of Plasmid-DNA were mixed with 100 µl of prepared cells and 700 µl PEG 4000/1xLiAc-mix and incubated for 30 minutes at 30 °C. In the following, 88 µl DMSO were added and cells were then incubated at 42 °C for 15 minutes. Afterwards, cells were centrifuged, the supernatant was discarded and the cells were washed in 500 µl 1x TE-buffer. The remaining yeast cells were resuspended in 100 µl 1x TE-buffer and selected on SD plates.

### Yeast expression cultures

Yeast expression cultures were performed in order to purify lipids or fatty acids. Therefore, transformed yeast colonies were resuspended in 2 ml SD medium with the corresponding dropout and incubated over night at 30 °C while shaking at 200 rpm. Then, the OD600 was measured and the amount (in ml) of cell-culture was calculated to inoculate 20 ml expression culture to an OD600 of 0.3. The expression cultures were incubated while shaking at 200 rpm for ca. 48 hours at 30 °C. Afterwards, similar OD600-units of expression cultures were harvested for lipid analysis.

### Transformation of A. cepa (Onion)

For transient transformation fresh onions were used.

### Gold particle precipitation

Aliquots containing 25 µl of gold suspension (50 mg/ml; solved in ethanol) were mixed vigorously with 3-8 µg of each plasmid-DNA (pCAMBIA3300 constructs and/or marker constructs), 55 µl of ddH2O, 50 µl CaCl2 (2.5 M) and 20 µl spermidin (0.1 mM). The gold-DNA mixture was precipitated via centrifugation at maximal speed for 10 seconds and washed three times with 100 µl 100% ethanol. Finally, the precipitate was resuspended in 30 µl 100% ethanol. The plasmid-DNA was then ready for transforming onion epidermal cells.

### Transient transformation of onion èpidermal cells via gold particle bombardment

Onion cells were transiently transformed using the PDS1000/He Biolistic Particle Delivery System (BioRad, Hercules, USA). First, 5 µl of plasmid-coated gold suspension (2.13.1) were placed centered on the macrocarrier and allowed to dry at room temperature. Further, the rupture disk, the macrocarrier (with gold particles) and the stopping screen were set into their correct position and the sliced onion was placed on the target plate. Then, a vacuum was set up in the chamber of the Particle Delivery System and high pressure was applied until the rupture disk broke. Due to this, the macrocarrier containing the gold particles was pressed down onto and the stopping screen, scattering the now accelerated gold particles, which then hit the onions surface with high speed. The transformed onion pieces were incubated overnight at room temperature and under high humidity to avoid desiccation of the onion. Microscopic evaluation was performed the next day.

### Fluorescence microscopy

Transiently transformed onion epidermal cells (see 2.13.2) were evaluated using the BX51 fluorescence microscope (Olympus, Hamburg, Germany). Therefore the epidermis of the onion piece was carefully ripped of using forceps and placed on an object plate with water before a cover slip was placed on top. Depending on the used fluorescent marker (mCherry, YFP and CFP) different UV-filters were required to detect the marked cells with 20x or 40x magnification. Pictures were taken using the ColorView II 3.3 MegaPixel CCD Camera (Olympus, Hamburg, Germany).

### Lipid analysis

### Lipid extraction from yeast and separation of lipid classes

All following extraction procedures were carried out in glass reaction tubes. For lipids analysis yeast expression has been carried out with 20 ml cultures. A certain amount of cells (measured by OD600 units) out of 20 ml yeast expression culture (see 2.12.1) were harvested by centrifugation at 700 g for 3 min at room temperature. The supernatant was discarded completely and the cells were disrupted in 1 ml methanol and glass beads (1.7-2 mm, Roth, Karlsruhe, Germany) by vigorously mixing for 20 minutes. The samples were incubated for 15 minutes at room temperature. Further, 1 ml Chloroform was added and the samples were again mixed vigorously for 20 minutes. After that, the samples were centrifuged and the supernatant (equivalent with the lipid extract) was transferred into a new glass reaction tube and was evaporated under nitrogen. The remaining cell debris was resuspended in 2 ml hexane:diethyl ether:formic acid (65:35:1 v/v/v), mixed well and incubated at room temperature for 15 minutes. The samples were centrifuged and the supernatant was fused with the already evaporating lipid extract. The dried lipid extracts were dissolved in 100 µl chloroform and transferred into glass inlays. The solvent was evaporated and finally the lipid residues were solved in 50 µl of chloroform to ensure consistent solvent volume. The lipid extracts could then be analyzed by thin layer chromatography.

### Thin layer chromatography (TLC)

Portions of 25 µl of the lipid extracts (2.20.1) were spotted on a silica gel TLC plate using the Automatic TLC Sampler 4 (Camag, Berlin, Germany). Lipid standards (hexadecanol, palmitic acid, palmitoyl palmitate, 1,2,3-triheptadecanoyl glyceryl, sterol esters, diacylglycerol, were dissolved in chloroform at a final concentration of 1 mg/ml and 2 µl aliquots were spotted on the TLC plates adjacent to the lipid extracts. TLC plates were developed in hexane:diethyl ether:formic acid (90:10:1, v/v/v) when the formation of wax esters were analyzed and hexane:diethylether:formic acid (65:35:1 v/v/v) when fatty alcohols were analyzed. After development, lipids were visualized on TLC by dipping the plate in a copper sulfate solution (10 g CuSO4 x 5 H2O; 0.8 % H3PO4 in 100 ml H2O) and charring by 180 °C.

### Fatty acid analysis

### Acidic hydrolysis and extraction of fatty acid methyl esters (FAMEs)

For fatty acid analysis yeast expression has been carried out with 20 ml cultures. A certain amount of cells (measured by OD600 units) out of 20 ml yeast expression culture were harvested by centrifugation at 700 g for 3 min at room temperature. Fatty acid methyl esters (FAMEs) were obtained by methylation of yeast cell sediments with methanol containing 2.75% (v/v) sulfuric acid and 2% (v/v) dimethoxypropane at 80 °C for 1h. As internal standard 1,2,3 triheptadecanoyl glycerol (stock solution 1 mg/ml) were added to each sample. The reaction was neutralized by adding 0.1 ml NaCl (5 M) and FAMEs were extracted in 2 ml of *n*-hexane, dried under N2, finally resolved in GC plastic inlays in 10 µl acetonitrile and analyzed by gas chromatography (GC).

### Identification of FAMEs by GC with flame ionization detection

The GC analysis was performed with an Agilent GC 6890 system coupled with a flamcionization detector equipped with a capillary 122-2332 DB-23 column (30 m x 0.32 mm; 0.5 µm coating thickness; Agilent). Helium was used as carrier gas (1 ml min-1). Samples were injected at 220 °C. The temperature gradient was 150 °C for 1 min, 150 to 200°C at 15°C min-1, 200 to 250 °C at 2 °C min-1, and 250 °C for 10 min. Data were processed using the HP ChemStation Rev. A09.03. FAMEs were identified by comparison with appropriate reference substances.

### Example 2. Plant and yeast expression constructs used herein.

To perform retargeting experiments, truncated versions of MmFAR1 were generated to determine which domains are essential for peroxisomal localization and which further domains can retarget the MmFAR enzymes from the peroxisomes to the cytosol or ER.

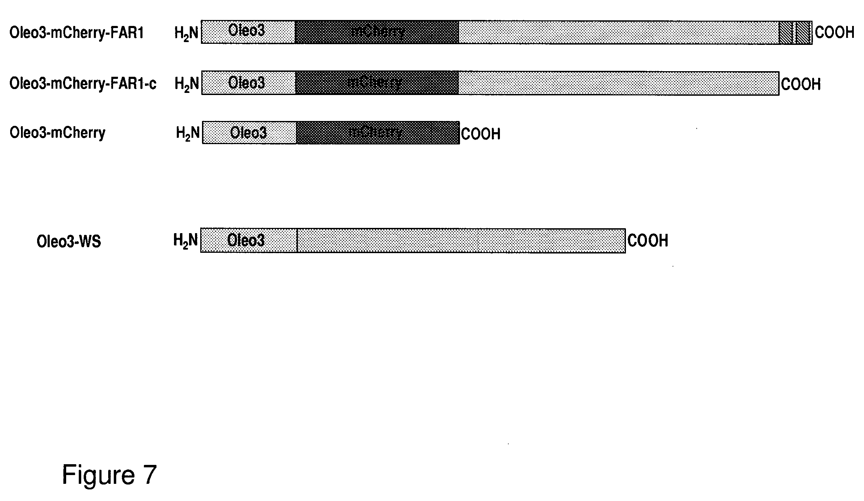

mCherry is a red fluorescent protein and its excitation and emission maxima are 587 nm and 610 nm, respectively. Expression of fusion proteins with mCherry enables to determine their localization in vivo by fluorescence microscopy. mCherry was cloned by PCR amplification into the BamHI and EcoRI sites (5' and 3' of mCherry, respectively) of the pUC18-ENTRY or the pESC-URA plasmids. The full length and the truncated open reading frames (ORFs) of MmFAR1 were modified using the Phusion Polymerase (Finnzymes, Espoo, Finland) and a standard PCR protocol with the respective primers listed in Materials and methods, introducing an EcoRI restriction site at the 5' prime end and a XhoI restriction site at the 3' prime end of the respective PCR products. The PCR products were then moved as EcoRI/XhoI fragments in frame to the already cloned Cherry either into the Gateway donor-vector pUC18-ENTRY or into the yeast expression vector pESC-URA, yielding the constructs mCherry-MmFAR1, mCherry-MmFAR1c and in pUC18-ENTRY as well as in pESC-URA (Fig. 7 and nucleotide sequences in the sequence listing).

Arabidopsis thaliana oleosin proteins are proteins which are targeted to oil bodies via the ER (Huang, 1992). The conformation of oleosins is unique with ER membrane proteins: their membrane-integrated hydrophobic domain is flanked by N- and C terminal domains located on the outer microsomal surface of the ER (Abell et al., 1997). This membrane topology on the ER allows the fusion of further proteins either to the N or the C-terminus. A. thaliana oleosin 3 fusion constructs were established by amplification of oleosin 3 from A. thaliana cDNA with the respective primers, introducing a SalI restriction site at the 5' prime end and a BamHI restriction site at the 3' prime end of the respective PCR product. The amplicon of oleosin 3 was partially restricted (within the oleosin 3 sequence an additional BamHI restriction site is localized) and moved as N-terminal fusion to the different mCherry-FAR-constructs in pUC18-ENTRY and pESC-URA, yielding Oleo3-mCherry-MmFAR1 or Oleo3-mCherry-MmFAR1c (Fig. 7 and nucleotide sequences in the sequence listing).

The MmWS was cloned as oleosin 3-YFP fusion construct, named Oleo3-YFP-WS and cloned via SalI and XhoI restriction sites into pUC18-ENTRY vector like the MmFAR constructs (Fig. 7 and nucleotide sequences in the sequence listing).

### Example 3. Subcellular localization of MmFar1 and truncated MmFar1c and fusion proteins to oleosin by transient expression in onion epidermis cells.

The fusion- or the truncated mCherry-MmFAR1 constructs, arranged in the pUC18-ENTRY vector, were transferred into the plant expression vector pCAMBIA3300 by clonase reaction. After selection, the positive clones were precipitated on gold particles along with a peroxisomal marker (MDH) . Those particles were then shot in onion cells in order of transient transformation. After incubation over night the transformed onion cells were examined via fluorescent microscopy.

Figure 2 shows the localization of the diverse MmFAR1 constructs tagged to mCherry. Images A of figure 2 shows onion epidermis cells expressing the mCherry-MmFAR1 construct. The fluorescence of mCherry-MmFAR1 was detected in the cytoplasm as small puncta that co-localized with the peroxisomal marker protein MDH (Fig. 2 B). observation corresponds to the described peroxisomal localization of MmFAR1 in animal cells reported by Cheng and Russel 2004.

As shown for MmFAR1c, the truncation of the C-terminal transmembrane domains of MmFAR1 (see Figure 1) leads to the re-localization of the MmFAR1c protein: from peroxisomal to a presumable cytosolic localization (Fig. 2 D). Co-expression of mCherry-MmFAR1c with the peroxisomal marker MDH (Fig. 2 E).

The AtOleosin 3 protein is known to be found in lipid bodies in Arabidopsis thaliana. By fusing mCherry-MmFAR1 and mCherry-MmFAR1c to AtOleosin 3 it could be examined, if oleosin 3 fusion were localized at the lipid bodies resulting in a retargeting of the associated MmFAR proteins as well. The retargeting towards lipid bodies is of high interest because lipid bodies evolve from the ER, where the MmFAR enzymes preferably should target to.

In figure 4 the images A-C show the oleosin 3-mCherry-MmFAR1 construct coexpressed in onion cells with the peroxisomal marker MDH. In image A, as well as in image B fluorescent spots are visible. By merging these two images it becomes apparent that, although in both images spots are recognizable, these spots show totally different patterns which cannot be explained by the time difference while changing the filters.

In figure 4D this different pattern can be observed more in detail in image A. Due to the difference in pattern it is taught, that the Oleo3-mCherry-MmFAR1 construct does not localize at the peroxisomes. Further, although the construct was not coexpressed with a lipid body marker, it is highly presumable that the constructs localizes at lipid bodies. One the one hand due to the dropwise distribution throughout the whole cell, on the other hand because oleosin 3 is known to localize at lipid bodies.

Similar results were obtained for Oleo3-mCherry-MmFAR1c, indicating this protein is localized at the lipid bodies as well.

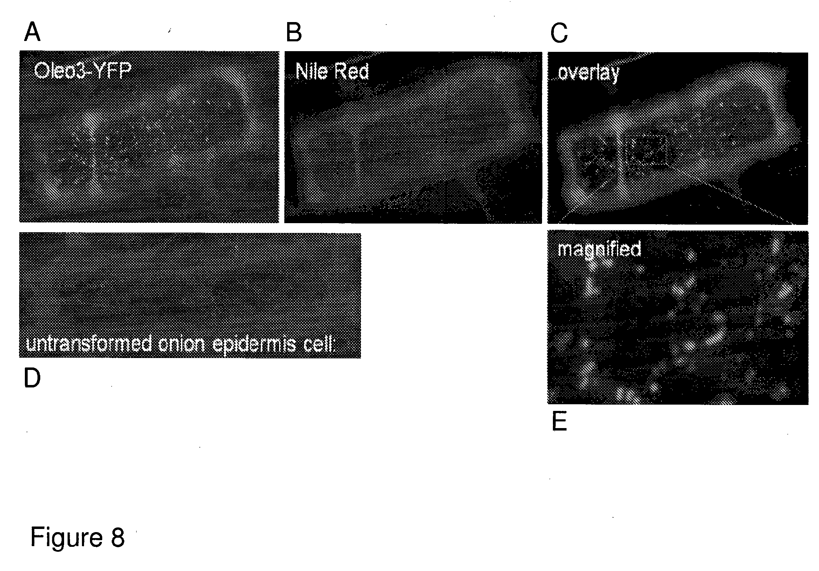

To investigate whether onion epidermis cells indeed contain lipid bodies or comparable structures, onion epidermis cells, expressing transiently YFP-Oleosin3 or without expression of any transgene, were stained with Nile red. Nile red is known to color oil bodies or lipid containing structures. As shown in Figure 8, dot like structures are visible within the untransformed onion epidermis cell.
Co-localization tests with YFP-Oleo3 show, that the localization of YFP-Oleo3 is visible as green fluorescent spots which merge with the Nile red stained droplets. Because Oleosin is known as lipid body marker, it therefore appears that, first, onion epidermal cells contain lipid bodies, or lipid body-like structures and second, that Oleo3-mCherry-FAR1c is localized at lipid bodies in onion epidermal cells, because it co-localizes with Oleo3-YFP, which on the other hand co-localizes with Nile red stained structures.

Figure 9 shows coexpression of Oleo3-mCherry-MmFAR1c with the peroxisomal marker MDH (A-D), and with YFP-Oleosin as oil body marker (E-H). As already mentioned, the fluorescent pattern does not merge with the fluorescent pattern of the Oleo3-mCherry-MmFAR1c-fusion protein although both appear as fluorescent spots.
Co-localization of Oleosin-tagged MmFAR1c with YFP-Oleosin as oil body marker shows clearly that Oleosin-tagged MmFAR1c localizes at lipid bodies.

As shown above, it is possible to retarget mCherry-FAR1c from cytosolic localization towards the oil bodies by connecting mCherry-MmFAR1c to AtOleosin3. Additionally YFP-WS was fused to At-Oleosin in order to achieve a co-localization at micro-domain level. To test the co-localization of Oleo3-mCherry-FAR1c with Oleo3-YFP-WS, both constructs were co-bombarded into onion epidermis cells and the fluorescent patterns were compared. As shown in figure 10, it is visible that both constructs localize in droplet-like structures as observed before. By merging the images of Oleosin3-mCherry-MmFAR1c and Oleo3-YFP-WS it becomes apparent that these spots show in most cases the same patterns and merges well.

### Example 4. Enzymatic activity of MmFar1, MmFar1 and Mm Far1c and oleo-FAR1c tagged with mCherry in yeast

After it was demonstrated by fluorescent microscopy that the truncated MmFAR1c constructs were no longer localized at the peroxisomes but in the cytosol, it was tested, if the activity of the wild type version and the truncated version are equal, or if a decrease/increase is detectable. It was also tested, how activity behaves by tagging the MmFAR enzyme to AtOleosin3. For this purpose, yeast (H1246) cells were transformed with the pESC-URA vectors containing the appropriate construct. After expression for 48 hours (Figure 3) or 72 hours (Figure 12), the lipids were harvested and separated by TLC.

The results demonstrated that expression of yeast of FAR1 (wt), FAR1 tagged with mCherry, Far1c tagged with mCherry, and FAR1c tagged with mCherry and oleosin all resulted in similar amounts of fatty alcohols being produced. Accordingly, neither the addition of mCherry tag, nor the truncation, nor the addition of oleosin influence the enzymatic activity in yeast.

### Example 5. Co-expression of MmFar1 and WS in yeast.

The wax biosynthetic pathway was reconstituted in cultured H1246 (yeast) cells by coexpressing cDNAs encoding unmodified mCherry-MmFAR1 or modified mCherry-MmFAR1c, Oleo3-mCherry-MmFAR1c with the MmWS, respectively. Additionally, co-expression of Oleo3-mCherry-FAR1c with Oleo3-YFP-WS was tested. The extracted lipids were then separated with the developing solvent hexane:diethyl ether:formic acid (90:10:1), which resolves mainly wax esters much better. This experiment was performed and the resulting TLC can be seen in figure 5. To identify the separated bands in the TLC (A), the following standards were used:sterols, TAG-mixture, 16:0 fatty acids, 16:0-OH fatty alcohols and wax esters. The tested samples were taken from yeasts transformed with an empty vector (control), or transformed with mCherry-MmFAR1 + MmWS (1), mCherry-MmFAR1c + MmWS (2), Oleo3mCherry-MmFAR1c + MmWS (3) or Oleo3mCherry-MmFAR1c +Oleo3YFP-MmWS (4) (three samples each).

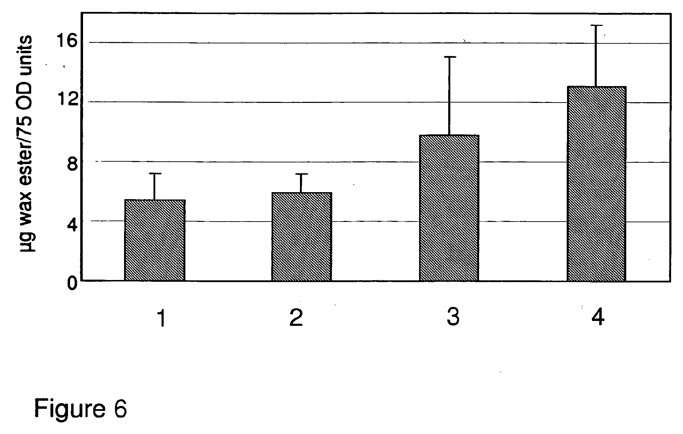

The interesting aspect on this plate is the amount of wax esters (marked with a red box). There are no bands detectable in the control samples. Looking at the mCherry-MmFAR1 + MmWS (1) samples, all three of them show weak bands in height of the WE running in the standard. Hence, this indicates a production of wax esters, which further as well indicates that the MmFAR1 must be active as well. MmFAR1 provides the 16:0-OH needed to produce WE. If MmFAR1 would not be active no wax esters could be produced as can be seen in the control samples. The samples taken from yeast cells transformed with mCherry-MmFAR1c + MmWS (2) show wax esters in an apparently equal amount compared with the mCherry-MmFAR1 + MmWS samples. In the three Oleo3mCherry-MmFAR1c + MmWS (3) samples the WE bands are visible as well, but clearly brighter than in the samples from (1) and (2). Oleo3mCherry-MmFAR1c + Oleo3YFP-MmWS (4) show even brighter bands, indicating an extremely high amount of wax esters produced in those yeast cells. To show the differences in wax ester production of each construct more in detail, a bar chart (Figure 6) was generated. The intensity of each WE band was measured and further, the average value of every construct was calculated. Next the histogram was produced, including the standard deviation. It can be observed, that the amount of wax esters produced in yeast containing mCherry-MmFAR1 + MmWS (1) and mCherry-MmFAR1c + MmWS (2) are nearly similar, whereas the amount of wax esters almost doubles in cells transformed with Oleo3mCherry-MmFAR1c + MmWS (3). Cells expressing Oleo3mCherry-MmFAR1c + Oleo3YFP-MmWS (4) show an increase in wax esters of nearly threefold.

The amounts of wax esters produced in yeast were also analyzed by GC-MS (See Figure 11). No wax esters could be detected in the empty vector control (data not shown). After expression of mCherry-FAR1 + YFP-WS, mCherry-FARIc + YFP-WS, Oleo3-mCherry-FAR1c + YFP-WS or Oleo3-mCherry-FAR1c + Oleo3-YFP-WS wax esters were produced and identified by standards. While the wax ester amount after expression of mCherry-FAR1 + YFP-WS and mCherry-FAR1c + YFP-WS seemed to be very similar, an increase of wax ester production and accumulation is observable with expression of Oleo3-mCherry-FAR1c + YFP-WS and even more with expression of Oleo3-mCherry-FAR1c + Oleo3-YFP-WS.

These results demonstrate that mCherry-MmFAR1, mCherry-MmFAR1c and Oleo3mCherry-MmFAR1c are active in yeast as they provide the substrate needed by the WS to produce wax esters. In addition, the results show that there is no significant difference in activity between MmFAR1 and the truncated MmFAR1c construct, which lacks the last two putative transmembrane domains. Finally, the results show that the activity can be increased by tagging MmFAR1c to AtOleosin3 and that activity can be further amplified by tagging MmFAR1c and MmWS to AtOleosin3.

### Example 6. Co-expression in plants of MmFar1-oleosin, MmFar1c-oleosin and MmWS oleosin.

To introduce a wax ester synthase biosynthetic pathway in plants, the following chimeric genes are constructed using conventional techniques comprising the following DNA fragments in order:
MmFar1-oleosin
   - a seed-specific promoter such as a promoter of the napin gene (Stalberg K., Ellerstrom M., Josefsson L. and Rask L. (1993). Deletion analysis of a 2S seed storage protein promoter of Brassica napus in transgenic tobacco. Plant Molecular Biology, 23, 671-683) or the USP promoter from Vicia faba described in DE10211617
   - a DNA fragment encoding an oleosin such as oleosin 3 (SEQ ID No 10 from amino acid 1 to amino acid 143) in, fused in the reading frame with
   - a DNA region encoding FAR1 from mouse (SEQ ID 1) or a DNA region encoding FAR1 from mouse tagged with mCherry (SEQ ID No 4)
   - a 3' transcription termination and polyadenylation region such as the 3' end of the nopaline synthase gene (3'nos: sequence including the 3' untranslated region of the nopaline synthase gene from the T-DNA of pTiT37 of Agrobacterium tumefaciens (Depicker et al., 1982, Journal of Molecular and Applied Genetics, 1, 561-573).
MmFar1c-oleosin
   - a seed-specific promoter such as a promoter of the napin gene or the USP promoter from Vicia faba described in DE10211617
   - a DNA fragment encoding an oleosin such as oleosin 3 (SEQ ID No 10 from amino acid 1 to amino acid 143) in, fused in the reading frame with
   - a DNA region encoding truncated FAR1 from mouse (SEQ ID 6) or a DNA region encoding truncated FAR1 from mouse tagged with mCherry (SEQ ID No 8)
   - a 3' transcription termination and polyadenylation region from the 3' end of the nopaline synthase gene (3'nos: sequence including the 3' untranslated region of the nopaline synthase gene from the T-DNA of pTiT37 of Agrobacterium tumefaciens (Depicker et al., 1982, Journal of Molecular and Applied Genetics, 1, 561-573).
MmWS-oleosin
   - a seed-specific promoter such as a promoter of the napin gene or the USP promoter from Vicia faba described in DE10211617
   - a a DNA fragment encoding an oleosin such as oleosin 3 (SEQ ID No 10 from amino acid 1 to amino acid 143) in, fused in the reading frame with
   - a DNA region encoding WS from mouse (SEQ ID 12) or a DNA region encoding WS from mouse tagged with YFP (SEQ ID No 14)
   - a 3' transcription termination and polyadenylation region from the 3' end of the nopaline synthase gene (3'nos: sequence including the 3' untranslated region of the nopaline synthase gene from the T-DNA of pTiT37 of Agrobacterium tumefaciens (Depicker et al., 1982, Journal of Molecular and Applied Genetics, 1, 561-573)

These chimeric genes are introduced in a T-DNA vector, between the left and right border sequences from the T-DNA, together with a selectable marker gene such as a marker providing resistance to the herbicide phosphinotricin or a marker providing tolerance to glyphosate or a antibiotic resistance marker such as plant expressible nptII or hygromycin phosphotransferase. The T-DNA vectors may also comprise both a MmFar1 derived oleosin fusion encoding chimeric gene and a MmWS-oleosin encoding chimeric gene.

The T-DNA vectors are introduced in Agrobacterium comprising helper Ti-plasmid as standard in the art and used to generate transformed Arabidopsis and Brassica napus plants.

Plants are transformed, crossed or co-transformed to generate transgenic plants comprising MmFar1-oleosin and MmWS-oleosin or MmFarlc-oleosin and MmWS-oleosin. Lipids are extracted from seeds and run on TLC. Short chain wax ester production is observed.

### References

Abell, B. M., Holbrook, L. A., Abensen, M., Murphy, D. J., Hills, M. J., Moloney, M. M., 1997. Role of the proline knot motif in oleosin endoplasmatic reticulum topology and oil body targeting. Plant Cell 8, 1481-1493.
Brandizzi, F., Snapp, E. L., Roberts, A. G., Lippincott-Schwartz, J., Hawes, C., 2002. Membrane protein transport between the endoplasmic reticulum and the Golgi in tobacco leaves is energy dependent but cytoskeleton independent: evidence from selective photobleaching. Plant Cell 14, 1293-1309.
Cheng, J. B., Russell, D. W., 2004a. Mammalian wax biosynthesis. I. Identification of two fatty acyl-Coenzyme A reductases with different substrate specificities and tissue distributions. Journal of Biological Chemistry 279, 37789-37797.
Cheng, J. B., Russell, D. W., 2004b. Mammalian Wax Biosynthesis. II. Expression Cloning of Wax synthase cDNAs encoding a member of the acyltransferase enzyme family. Journal of Biological Chemistry 279, 37798-37807.
Dyer, J. M., Stymne, S., Green, A. G., Carlsson, A. S., 2008. High-value oils from plants. The Plant journal: cell and molecular biology 54, 640-655.
Fang, Y., Morrell, J. C., Jones, J. M., Gould, S. J., 2004. PEX3 functions as a PEX19 docking factor in the import of class I peroxisomal membrane proteins. The Journal of Cell Biology 164.
Fitzgerald, M., Murphy, R. C., 2007. Electrospray mass spectrometry of human hair wax esters. Journal of Lipid Research 48, 1231-1246.
Fransen, M., Wylin, T., Brees, C., Mannaerts, G. P., Van Veldhoven, P. P., 2001. Human Pex19p Binds Peroxisomal Integral Membrane Proteins at Regions Distinct from Their Sorting Sequences.. Molecular and Cellular Biology 21, 4413-4424.
Fukuda, M., Viitala, J., Matteson, J., Carlsson, S. R., 1988. Cloning of cDNAs encoding human lysosomal membrane glycoproteins, h-lamp-1 and h-lamp-2. Comparison of their deduced amino acid sequences. Journal of Biological Chemistry 263, 18920-18928.
Fulda, M., Shockey, J., Werber, M., Wolter, F. P., Heinz, E., 2002. Two long-chain acyl-CoA synthetases from Arabidopsis thaliana involved in peroxisomal fatty acid ß-oxidation. The Plant Journal 32, 93-103.
Hadden, D. A., Phillipson, B. A., Johnston, K. A., Brown, L.-A., Manfield, I. W., El-Shami, M., Sparkes, I. A., Baker, A., 2006. Arabidopsis PEX19 is a dimeric protein that binds to peroxin PEX10. Molecular Membrane Biology 23, 325-336.
Heiland, I., Erdmann, R., 2005. Biogenesis of peroxisomes. Topogenesis of the peroxisomal membrane and matrix proteins. FEBS Journal 272, 2362-2372.
Hoffmann, M., Hornung, E., Busch, S., Kassner, N., Ternes, P., Braus, G. H., Feussner, I., 2007. A Small membrane-peripheral region Close to the Active Center Determines Regioselectivity of Membrane-bound Fatty Acid Desaturases from Aspergillus nidulans. The Journal of Biological Chemistry 282, 26666-26674.
Huang, A. H. C., 1992. Oil Bodies and Oleosins in Seeds. Annual Review Plant Physiology Plant Molecular Biology 43, 177-200. Inoue, H., Niojima, H., Okayama, H., 1990. High efficiency transformation of Escherichia coli with plasmids. Gene 96, 23-28.
Iyengar, B. T., Schlenk, H., 1969. Melting Points of Synthetic Wax Esters. Lipids 4, 28-30.
Jetter, R., Kunst, L., 2008. Plant surface lipid biosynthetic pathways and their utility for metabolic engineering of waxes and hydroarbon biofuels. Plant Journal 54, 670- 683.
Kunst, L., Samuels, A. L., 2003. Biosynthesis and secretion of plant cuticular wax. Progress of Lipid Research 42, 51-80.
Lardizabal, K., Metz, J. G., Sakamoto, T., Hutton, W. C., Pollard, M. R., Lassner, M. W., 2000. Purification of a Jojoba Embryo Wax Synthase, Cloning of its cDNA, and Production of High Levels of Wax in Seeds of Transgenic Arabidopsis. Plant Physiology 122, 645-655.
Lassner, M. W., Lardizabal, K., Metz, J. G., 1996. A jojoba b-ketoacyl-CoA synthase cDNA complements the canola fatty acid elongation mutation in transgenic plants. Plant Cell 8, 281-292.
Lassner, M. W., Lardizabal, K., Metz, J. G. (Eds.), 1999. Producing Wax Esters in Transgenic Plants by Expression of Genes Derived from Jojoba. ASHS Press, Alexandria, VA.
Matsuzono, Y., Fujiki, Y., 2006. In Vitro Transport of Membrane Proteins to Peroxisomes by Shuttling Receptor Pex19p. Journal of Biological Chemistry 28, 36-42.
Millar, A. A., Kunst, L., 1997. Very-long-chain fatty acid biosynthesis is controlled through the expression and specificity of the condensing enzyme. Plant Journal 12, 121-131.
Pollard, M. R., McKeon, T., Gupta, L. M., Stumpf, P. K., 1979. Studies on biosynthesis of waxes by developing Jojoba seed. 2. The demonstration of wax biosynthesis by cell-free homogenates. Lipids 14, 651-662.
Sanger, F., Nicklen, S., Coulson, A. R., 1977. DNA sequencing with chain-terminating inhibitors. Biotechnology 24, 104-108
Stryer, L. (Ed.), 1995. Biochemie. Spektrum Akademischer Verlag Heidelberg, Heidelberg.
Todd, J., Post-Beittenmiller, D., 1999. KCS1 encodes a fatty acid elongase 3-ketoacyl-CoA synthase affecting wax biosynthesis in Arabidopsis thaliana. Plant Journal 17, 119-130.
von Wettstein-Knowles, P. (Ed.), 1982. Biosynthesis of epicuticular lipids as analysed with the aid of gene mutations in barley. Elsevier, Amsterdam.
Vrkoslav, V., Mikova, R., 2009. Characterization of natural wax esters by MALDI-TOF mass spectrometry. Journal of mass spectrometry 44, 101-110.
Xu, X., Dietrich, C. R., Lessire, R., Nikolau, B. J., Schnable, P. S., 2002. The endoplasmic reticulum-associated maize GL8 protein is a component of the acylcoenzyme A elongase involved in the production of cuticular waxes. Plant Physiology 128, 924-934.
Zheng, H., Rowland, O., Kunst, L., 2005. Disruptions of the Arabidopsis enoyl-CoA reductase gene reveal an essential role for very-long-chain fatty acid synthesis in cell expansion during plant morphogenesis. Plant Cell 17.

### Acknowledgment

The work leading to this invention has received funding from the European Community's Seventh Framework Programme FP7/2007-13 under agreement number 211400.

## Claims

1. A method for modulating lipid biosynthesis in a non-human eukaryotic organism comprising the step of providing to cells of said organism at least a first and a second chimeric protein,
a. said first chimeric protein comprising:
i. a first heterologous polypeptide targeting said proteins to a particular subdomain of an organelle in said cell operably linked to
ii. a first polypeptide involved in fatty acid metabolism or lipid metabolism; and
b. said second chimeric protein comprising:
i. a second heterologous polypeptide targeting said proteins to said particular subdomain of an organelle in said cell operably linked to
ii. a second polypeptide involved in fatty acid metabolism or lipid metabolism
wherein said first and second polypeptide involved in fatty acid metabolism or lipid metabolism are different polypeptides with a catalytic activity which actively contribute to the fatty acid metabolism or lipid metabolism, and wherein said first and second polypeptide involved in fatty acid metabolism or lipid metabolism act in a concerted manner; and
wherein said method is not a method of treatment of the human or animal body by surgery or therapy, or a diagnostic method practiced on the human or animal body.

2. The method according to claim 1 wherein said heterologous polypeptide comprises a polypeptide sequence selected from an oleosin, an endoplasmatic retrieval signal from fatty acid desaturases, the N-terminal domain of LBLOX or the N-terminal domain of PLA.

3. The method according to claim 2, wherein said heterologous polypeptide comprises a polypeptide sequence having at least 80% homology to the amino acid sequence of SEQ ID No 10 from amino acid 1 to amino acid 143.

4. The method according to any one of claims 1 to 3, wherein said at least two chimeric proteins comprise the same heterologous polypeptide.

5. The method according to any one of the preceding claims, wherein said at least two chimeric proteins comprise at least one chimeric protein comprising a fatty acyl-CoA reductase and at least another chimeric protein comprising a wax ester synthase.

6. The method according to claim 5, wherein said fatty acyl-CoA reductase is derived from mouse and preferably has an amino acid sequence having about 80% sequence identity to the amino acid sequence of SEQ ID 6, and wherein said wax ester synthase is derived from mouse and preferably has an amino acid sequence having about 80% sequence identity to the amino acid sequence of SEQ ID No 12.

7. The method according to claim 5 or 6, wherein said chimeric proteins each comprise a heterologous targeting polypeptide which comprises the amino acid sequence of an oleosin.

8. The method according to claim 7, wherein said heterologous targeting polypeptide comprises a polypeptide sequence having at least 80% homology to the amino acid sequence of SEQ ID No 10 from amino acid 1 to amino acid 143.

9. The method according to any one of claims 5 to 8 for producing selected wax esters.

10. The method of any one of claims 1 to 9 wherein said chimeric proteins are expressed from one or more DNA constructs comprising the following operably linked DNA fragments:
a. a promoter functional in cells of said eukaryotic organism
b. a DNA region encoding said chimeric protein
c. a transcription termination and/or polyadenylation region.

11. The method according to any one of claims 1 to 10 wherein said eukaryotic organism is an oil producing plant.

12. A non-human eukaryotic organism comprising at least a first and a second DNA construct,
a. said first DNA construct comprising:
i. a first promoter functional in cells of said eukaryotic organism
ii. a first DNA region encoding a first chimeric protein comprising
1. a DNA region encoding a first heterologous polypeptide targeting said proteins to a particular subdomain of an organelle in said cell; operably linked to
2. a DNA region encoding a first polypeptide involved in fatty acid metabolism or lipid metabolism;
iii. a transcription termination and/or polyadenylation region; and
b. said second DNA construct comprising
iv. a second promoter functional in cells of said eukaryotic organism
v. a second DNA region encoding a second chimeric protein comprising
3. a DNA region encoding a second heterologous polypeptide targeting said proteins to said particular subdomain of an organelle in said cell; operably linked to
4. a DNA region encoding a second polypeptide involved in fatty acid metabolism or lipid metabolism;
vi. a transcription termination and/or polyadenylation region;
c. wherein said first and second heterologous polypeptide may be the same or different and wherein said first and second polypeptide involved in fatty acid metabolism or lipid metabolism are different polypeptides with a catalytic activity which actively contribute to the fatty acid metabolism or lipid metabolism, and wherein said first and second polypeptide involved in fatty acid metabolism or lipid metabolism act in a concerted manner.

13. The organism according to claim 12, wherein said first and said second heterologous polypeptide comprise a polypeptide sequence selected from an oleosin, an endoplasmatic retrieval signal from fatty acid desaturases, the N-terminal domain of LBLOX or the N-terminal domain of PLA.

14. The organism according to claim 12 or 13, wherein said first and second heterologous polypeptide comprises a polypeptide sequence having at least 80% homology to the amino acid sequence of SEQ ID No 10 from amino acid 1 to amino acid 143.

15. The organism according to any one of claims 12 to 14, wherein said first polypeptide involved in fatty acid or lipid metabolism comprises the amino acid sequence of a fatty acyl-CoA reductase and wherein said second polypeptide involved in fatty acid or lipid metabolism comprises the amino acid sequence of a wax ester synthase.

16. The organism according to claim 15, wherein said fatty acyl-CoA reductase is derived from mouse and preferably has an amino acid sequence having about 80% sequence identity to the amino acid sequence of SEQ ID 6, and wherein said wax ester synthase is derived from mouse and preferably has an amino acid sequence having about 80% sequence identity to the amino acid sequence of SEQ ID No 12.

17. The organism according to any one of claims 12 to 16 wherein said eukaryotic organism is an oil producing plant.

## Patentansprüche

1. Verfahren zur Modulation der Lipid-Biosynthese in einem nichtmenschlichen eukaryontischen Organismus, umfassend den Schritt, bei dem Zellen des Organismus wenigstens ein erstes und ein zweites chimäres Protein bereitgestellt werden,
a. wobei das erste chimäre Protein
i. ein erstes heterologes Polypeptid, das die Proteine auf eine bestimmte Subdomäne einer Organelle in der Zelle ausrichtet, in operativer Verknüpfung mit
ii. einem ersten Polypeptid, das am Fettsäurestoffwechsel oder Lipidstoffwechsel beteiligt ist,
umfasst; und
b. wobei das zweite chimäre Protein
i. ein zweites heterologes Polypeptid, das die Proteine auf die bestimmte Subdomäne einer Organelle in der Zelle ausrichtet, in operativer Verknüpfung mit
ii. einem zweiten Polypeptid, das am Fettsäurestoffwechsel oder Lipidstoffwechsel beteiligt ist,
umfasst;
wobei es sich bei dem am Fettsäurestoffwechsel oder Lipidstoffwechsel beteiligten ersten und zweiten Polypeptid um unterschiedliche Polypeptide mit einer katalytischen Aktivität handelt, die aktiv zum Fettsäurestoffwechsel oder Lipidstoffwechsel beitragen, und wobei das am Fettsäurestoffwechsel oder Lipidstoffwechsel beteiligte erste und zweite Polypeptid konzertiert wirken; und
wobei es sich bei dem Verfahren weder um ein Verfahren zur therapeutischen oder chirurgischen Behandlung des menschlichen oder tierischen Körpers noch um ein Diagnostizierverfahren, das am menschlichen oder tierischen Körper vorgenommen wird, handelt.

2. Verfahren nach Anspruch 1, wobei das heterologe Polypeptid eine aus einem Oleosin, einem endoplasmatischen Rückholsignal aus Fettsäure-Desaturasen, der N-terminalen Domäne von LBLOX oder der N-terminalen Domäne von PLA ausgewählte Polypeptidsequenz umfasst.

3. Verfahren nach Anspruch 2, wobei das heterologe Polypeptid eine Polypeptidsequenz mit wenigstens 80% Homologie zur Aminosäuresequenz unter SEQ ID Nr. 10 von Aminosäure 1 bis Aminosäure 143 umfasst.

4. Verfahren nach einem der Ansprüche 1 bis 3, wobei die wenigstens zwei chimären Proteine das gleiche heterologe Polypeptid umfassen.

5. Verfahren nach einem der vorhergehenden Ansprüche, wobei die wenigstens zwei chimären Proteine wenigstens ein chimäres Protein, das eine Fettsäure-Acyl-CoA-Reduktase umfasst, und wenigstens ein weiteres chimäres Protein, das eine Wachsester-Synthase umfasst, umfassen.

6. Verfahren nach Anspruch 5, wobei die Fettsäure-Acyl-CoA-Reduktase aus der Maus stammt und vorzugsweise eine Aminosäuresequenz mit etwa 80% Sequenzidentität mit der Aminosäuresequenz unter SEQ ID 6 aufweist und wobei die Wachsester-Synthase aus der Maus stammt und vorzugsweise eine Aminosäuresequenz mit etwa 80% Sequenzidentität mit der Aminosäuresequenz unter SEQ ID Nr. 12 aufweist.

7. Verfahren nach Anspruch 5 oder 6, wobei die chimären Proteine jeweils ein heterologes Ausrichtungs-Polypeptid umfassen, das die Aminosäuresequenz eines Oleosins umfasst.

8. Verfahren nach Anspruch 7, wobei das heterologe Ausrichtungs-Polypeptid eine Polypeptidsequenz mit wenigstens 80% Homologie zur Aminosäuresequenz unter SEQ ID Nr. 10 von Aminosäure 1 bis Aminosäure 143 umfasst.

9. Verfahren nach einem der Ansprüche 5 bis 8 zur Herstellung ausgewählter Wachsester.

10. Verfahren nach einem der Ansprüche 1 bis 9, wobei die chimären Proteine von einem oder mehreren DNA-Konstrukten exprimiert werden, die die folgenden in operativer Verknüpfung stehenden DNA-Fragmente umfassen:
a. einen in Zellen des eukaryontischen Organismus funktionsfähigen Promotor
b. einen das chimäre Protein codierenden DNA-Bereich
c. einen Transkriptionsterminations- und/oder Polyadenylierungsbereich.

11. Verfahren nach einem der Ansprüche 1 bis 10, wobei es sich bei dem eukaryontischen Organismus um eine ölproduzierende Pflanze handelt.

12. Nichtmenschlicher eukaryontischer Organismus, umfassend wenigstens ein erstes und ein zweites DNA-Konstrukt,
a. wobei das erste DNA-Konstrukt
i. einen ersten in Zellen des eukaryontischen Organismus funktionsfähigen Promotor;
ii. einen ersten ein erstes chimäres Protein codierenden DNA-Bereich, der
1. einen DNA-Bereich, der ein erstes heterologes Polypeptid, das die Proteine auf eine bestimmte Subdomäne einer Organelle in der Zelle ausrichtet, codiert, in operativer Verknüpfung mit
2. einem DNA-Bereich, der ein erstes Polypeptid, das am Fettsäurestoffwechsel oder Lipidstoffwechsel beteiligt ist, codiert,
umfasst;
iii. einen Transkriptionsterminations- und/oder Polyadenylierungsbereich
umfasst; und
b. wobei das zweite DNA-Konstrukt
iv. einen zweiten in Zellen des eukaryontischen Organismus funktionsfähigen Promotor;
v. einen zweiten ein zweites chimäres Protein codierenden DNA-Bereich, der
3. einen DNA-Bereich, der ein zweites heterologes Polypeptid, das die Proteine auf die bestimmte Subdomäne einer Organelle in der Zelle ausrichtet, codiert, in operativer Verknüpfung mit
4. einem DNA-Bereich, der ein zweites Polypeptid, das am Fettsäurestoffwechsel oder Lipidstoffwechsel beteiligt ist, codiert,
umfasst;
vi. einen Transkriptionsterminations- und/oder Polyadenylierungsbereich
umfasst;
c. wobei das erste und zweite heterologe Polypeptid gleich oder verschieden sein können und wobei es sich bei dem am Fettsäurestoffwechsel oder Lipidstoffwechsel beteiligten ersten und zweiten Polypeptid um unterschiedliche Polypeptide mit einer katalytischen Aktivität handelt, die aktiv zum Fettsäurestoffwechsel oder Lipidstoffwechsel beitragen, und wobei das am Fettsäurestoffwechsel oder Lipidstoffwechsel beteiligte erste und zweite Polypeptid konzertiert wirken.

13. Organismus nach Anspruch 12, wobei das erste und das zweite heterologe Polypeptid eine aus einem Oleosin, einem endoplasmatischen Rückholsignal aus Fettsäure-Desaturasen, der N-terminalen Domäne von LBLOX oder der N-terminalen Domäne von PLA ausgewählte Polypeptidsequenz umfassen.

14. Organismus nach Anspruch 12 oder 13, wobei das erste und zweite heterologe Polypeptid eine Polypeptidsequenz mit wenigstens 80% Homologie zur Aminosäuresequenz unter SEQ ID Nr. 10 von Aminosäure 1 bis Aminosäure 143 umfassen.

15. Organismus nach einem der Ansprüche 12 bis 14, wobei das erste am Fettsäure- oder Lipidstoffwechsel beteiligte Polypeptid die Aminosäuresequenz einer Fettsäure-Acyl-CoA-Reduktase umfasst und wobei das zweite am Fettsäure- oder Lipidstoffwechsel beteiligte Polypeptid die Aminosäuresequenz einer Wachsester-Synthase umfasst.

16. Organismus nach Anspruch 15, wobei die Fettsäure-Acyl-CoA-Reduktase aus der Maus stammt und vorzugsweise eine Aminosäuresequenz mit etwa 80% Sequenzidentität mit der Aminosäuresequenz unter SEQ ID 6 aufweist und wobei die Wachsester-Synthase aus der Maus stammt und vorzugsweise eine Aminosäuresequenz mit etwa 80% Sequenzidentität mit der Aminosäuresequenz unter SEQ ID Nr. 12 aufweist.

17. Organismus nach einem der Ansprüche 12 bis 16, wobei es sich bei dem eukaryontischen Organismus um eine ölproduzierende Pflanze handelt.

## Revendications

1. Procédé de modulation de la biosynthèse des lipides dans un organisme eucaryote non humain, comprenant l'étape de fourniture à des cellules dudit organisme d'au moins une première et une deuxième protéine chimère,
a. ladite première protéine chimère comprenant :
i. un premier polypeptide hétérologue ciblant lesdites protéines vers un sous-domaine particulier d'un organite dans ladite cellule, fonctionnellement lié à
ii. un premier polypeptide impliqué dans le métabolisme des acides gras ou le métabolisme des lipides ; et
b. ladite deuxième protéine chimère comprenant :
i. un deuxième polypeptide hétérologue ciblant lesdites protéines vers ledit sous-domaine particulier d'un organite dans ladite cellule, fonctionnellement lié à
ii. un deuxième polypeptide impliqué dans le métabolisme des acides gras ou le métabolisme des lipides,
dans lequel ledit premier et ledit deuxième polypeptide impliqué dans le métabolisme des acides gras ou le métabolisme des lipides sont des polypeptides différents ayant une activité catalytique, qui contribuent activement au métabolisme des acides gras ou au métabolisme des lipides, et ledit premier et ledit deuxième polypeptide impliqué dans le métabolisme des acides gras ou dans le métabolisme des lipides agissant d'une manière concertée ; et
ledit procédé n'étant pas un procédé de traitement du corps humain ou animal par chirurgie ou thérapie, ni un procédé diagnostique pratiqué sur le corps humain ou animal.

2. Procédé selon la revendication 1, dans lequel ledit polypeptide hétérologue comprend une séquence polypeptidique choisie parmi une oléosine, un signal de récupération endoplasmique provenant d'acide gras désaturases, le domaine N-terminal de LBLOX ou le domaine N-terminal de PLA.

3. Procédé selon la revendication 2, dans lequel ledit polypeptide hétérologue comprend une séquence polypeptidique ayant une homologie d'au moins 80 % avec la séquence d'acides aminés de SEQ ID NO:10 de l'acide aminé 1 à l'acide aminé 143.

4. Procédé selon l'une quelconque des revendications 1 à 3, dans lequel lesdites au moins deux protéines chimères comprennent le même polypeptide hétérologue.

5. Procédé selon l'une quelconque des revendications précédentes, dans lequel lesdites au moins deux protéines chimères comprennent au moins une protéine chimère comprenant une acyle gras-CoA réductase et au moins une autre protéine chimère comprenant un ester de cire synthase.

6. Procédé selon la revendication 5, dans lequel ladite acyle gras-CoA réductase provient de souris et de préférence a une séquence d'acides aminés ayant une identité de séquence d'environ 80 % avec la séquence d'acides aminés SEQ ID NO:6, et dans lequel ladite ester de cire synthase provient de souris et de préférence a une séquence d'acides aminés ayant une identité de séquence d'environ 80 % avec la séquence d'acides aminés SEQ ID NO:12.

7. Procédé selon la revendication 5 ou 6, dans lequel lesdites protéines chimères comprennent chacune un polypeptide ciblant hétérologue qui comprend la séquence d'acides aminés d'une oléosine.

8. Procédé selon la revendication 7, dans lequel ledit polypeptide ciblant hétérologue comprend une séquence polypeptidique ayant une homologie d'au moins 80 % avec la séquence d'acides aminés de SEQ ID NO:10 de l'acide aminé 1 à l'acide aminé 143.

9. Procédé selon l'une quelconque des revendications 5 à 8, pour la production d'esters de cire sélectionnés.

10. Procédé selon l'une quelconque des revendications 1 à 9, dans lequel lesdites protéines chimères sont exprimées à partir d'une ou plusieurs constructions d'ADN comprenant les fragments d'ADN fonctionnellement liés suivants :
a. un promoteur fonctionnel dans les cellules dudit organisme eucaryote,
b. une région d'ADN codant pour ladite protéine chimère,
c. une région de terminaison de transcription et/ou de polyadénylation.

11. Procédé selon l'une quelconque des revendications 1 à 10, dans lequel ledit organisme eucaryote est une plante oléagineuse.

12. Organisme eucaryote non humain comprenant au moins une première et une deuxième construction d'ADN,
a. ladite première construction d'ADN comprenant :
i. un premier promoteur fonctionnel dans les cellules dudit organisme eucaryote,
ii. une première région d'ADN codant pour une première protéine chimère, comprenant
1. une région d'ADN codant pour un premier polypeptide hétérologue ciblant lesdites protéines vers un sous-domaine particulier d'un organite dans ladite cellule ; fonctionnellement liée à
2. une région d'ADN codant pour un premier polypeptide impliqué dans le métabolisme des acides gras ou dans le métabolisme des lipides ;
iii. une région de terminaison de transcription et/ou de polyadénylation ; et
b. ladite deuxième construction d'ADN comprenant
iv. un deuxième promoteur fonctionnel dans les cellules dudit organisme eucaryote,
v. une deuxième région d'ADN codant pour une deuxième protéine chimère, comprenant
3. une région d'ADN codant pour un deuxième polypeptide hétérologue ciblant lesdites protéines vers ledit sous-domaine particulier d'un organite dans ladite cellule ; fonctionnellement liée à
4. une région d'ADN codant pour un deuxième polypeptide impliqué dans le métabolisme des acides gras ou dans le métabolisme des lipides ;
vi. une région de terminaison de transcription et/ou de polyadénylation ;
c. dans lequel ledit premier et ledit deuxième polypeptide hétérologue peuvent être identiques ou différents, et ledit premier et ledit deuxième polypeptide impliqué dans le métabolisme des acides gras ou dans le métabolisme des lipides étant des polypeptides différents ayant une activité catalytique, qui contribuent activement au métabolisme des acides gras ou au métabolisme des lipides, et ledit premier et ledit deuxième polypeptide impliqué dans le métabolisme des acides gras ou dans le métabolisme des lipides agissant d'une manière coordonée.

13. Organisme selon la revendication 12, dans lequel ledit premier et ledit deuxième polypeptide hétérologue comprennent une séquence polypeptidique choisie parmi une oléosine, un signal de récupération endoplasmique provenant d'acide gras désaturases, le domaine N-terminal de LBLOX ou le domaine N-terminal de PLA.

14. Organisme selon la revendication 12 ou 13, dans lequel ledit premier et ledit deuxième polypeptide hétérologue comprennent une séquence polypeptidique ayant une homologie d'au moins 80 % avec la séquence d'acides aminés de SEQ ID NO:10 de l'acide aminé 1 à l'acide aminé 143.

15. Organisme selon l'une quelconque des revendications 12 à 14, dans lequel ledit premier polypeptide impliqué dans le métabolisme des acides gras ou des lipides comprend la séquence d'acides aminés d'une acyle gras-CoA réductase, et dans lequel ledit deuxième polypeptide impliqué dans le métabolisme des acides gras ou des lipides comprend la séquence d'acides aminés d'un ester de cire synthase.

16. Organisme selon la revendication 15, dans lequel ladite acyle gras-CoA réductase provient de souris et de préférence a une séquence d'acides aminés ayant une identité de séquence d'environ 80 % avec la séquence d'acides aminés SEQ ID NO:6, et dans lequel ladite ester de cire synthase provient de souris et a de préférence une séquence d'acides aminés ayant une identité de séquence d'environ 80 % avec la séquence d'acides aminés SEQ ID NO:12.

17. Organisme selon l'une quelconque des revendications 12 à 16, ledit organisme eucaryote étant une plante oléagineuse.


### SEQUENCE LISTING

<110> Georg-August-Universitaet Goettingen Stiftung Oeffentlichen Rechts Feussner, Ivo Heilmann, Mareike
<120> METHODS AND MEANS TO ALTER LIPID BIOSYNTHESIS BY TARGETING MULTIPLE ENZYMES TO SUBORGANELLE DOMAINS
<130> B09165A - CA
<150> EP09013248.1
   <151> 2009-10-20
<160> 16
<170> PatentIn version 3.3
<210> 1
   <211> 1548
   <212> DNA
   <213> Artificial
<220>
   <223> mouse fatty acyl CoA reductase
<220>
   <221> CDS
   <222> (1)..(1548)
<400> 1
   atg gtt tcc atc cca gag tac tat gag gga aag aac atc ttg cta act        48
   Met Val Ser Ile Pro Glu Tyr Tyr Glu Gly Lys Asn Ile Leu Leu Thr
   1               5                   10                  15
   ggt gca act gga ttt ctt ggg aag gtt ttg ctt gag aag ctg cta aga        96
   Gly Ala Thr Gly Phe Leu Gly Lys Val Leu Leu Glu Lys Leu Leu Arg
               20                  25                  30
   tct tgc cct aga gtc aac tcc gtt tac gtt cta gtc aga caa aag gct       144
   Ser Cys Pro Arg Val Asn Ser Val Tyr Val Leu Val Arg Gln Lys Ala
           35                  40                  45
   gga caa aca cct caa gaa aga gtt gag gag atc ttg tca agc aag ctc       192
   Gly Gln Thr Pro Gln Glu Arg Val Glu Glu Ile Leu Ser Ser Lys Leu
       50                  55                  60
   ttt gat aga ctg cgt gat gag aat cca gat ttc cga gag aag atc atc       240
   Phe Asp Arg Leu Arg Asp Glu Asn Pro Asp Phe Arg Glu Lys Ile Ile
   65                  70                  75                  80
   gcc atc aac tct gaa ttg acc caa cct aag tta gcg ctt tct gaa gag       288
   Ala Ile Asn Ser Glu Leu Thr Gln Pro Lys Leu Ala Leu Ser Glu Glu
                   85                  90                  95
   gat aag gag atc atc atc gac tca acg aac gtt atc ttc cat tgc gct       336
   Asp Lys Glu Ile Ile Ile Asp Ser Thr Asn Val Ile Phe His Cys Ala
               100                 105                 110
   gca aca gtt cgt ttc aat gag aac cta cgg gat gct gtt caa ttg aac       384
   Ala Thr Val Arg Phe Asn Glu Asn Leu Arg Asp Ala Val Gln Leu Asn
           115                 120                 125
   gtc att gct acg aga caa ctg atc tta ctc gct cag cag atg aag aac       432
   Val Ile Ala Thr Arg Gln Leu Ile Leu Leu Ala Gln Gln Met Lys Asn
       130                 135                 140
   ctc gaa gtc ttc atg cac gtt agt act gct tac gca tac tgt aac cgc     480
   Leu Glu Val Phe Met His Val Ser Thr Ala Tyr Ala Tyr Cys Asn Arg
   145                 150                 155                 160
   aag cac atc gat gaa gtt gtt tac cca cct cca gtt gat cct aag aag     528
   Lys His Ile Asp Glu Val Val Tyr Pro Pro Pro Val Asp Pro Lys Lys
                   165                 170                 175
   ttg atc gac tct ctt gag tgg atg gat gat gga ctt gtg aac gac ata     576
   Leu Ile Asp Ser Leu Glu Trp Met Asp Asp Gly Leu Val Asn Asp Ile
               180                 185                 190
   aca cca aag ctt atc gga gac aga cca aac act tac atc tac act aag     624
   Thr Pro Lys Leu Ile Gly Asp Arg Pro Asn Thr Tyr Ile Tyr Thr Lys
           195                 200                 205
   gca ctg gct gag tat gtt gtt caa caa gag gga gct aag ttg aac gtt     672
   Ala Leu Ala Glu Tyr Val Val Gln Gln Glu Gly Ala Lys Leu Asn Val
       210                 215                 220
   gca atc gtt aga ccg tct att gtt gga gct tca tgg aaa gaa cca ttc     720
   Ala Ile Val Arg Pro Ser Ile Val Gly Ala Ser Trp Lys Glu Pro Phe
   225                 230                 235                 240
   cca gga tgg att gac aac ttc aat ggt cca tct gga ctt ttc att gca     768
   Pro Gly Trp Ile Asp Asn Phe Asn Gly Pro Ser Gly Leu Phe Ile Ala
                   245                 250                 255
   gct ggc aag ggt atc ctc aga act atg aga gca agt aac aac gca ctt     816
   Ala Gly Lys Gly Ile Leu Arg Thr Met Arg Ala Ser Asn Asn Ala Leu
               260                 265                 270
   gca gat ctt gtt cct gtt gac gtt gtc gtt aac acc tca tta gca gct     864
   Ala Asp Leu Val Pro Val Asp Val Val Val Asn Thr Ser Leu Ala Ala
           275                 280                 285
   gca tgg tat tct gga gtt aac aga ccc agg aac atc atg gtg tac aac     912
   Ala Trp Tyr Ser Gly Val Asn Arg Pro Arg Asn Ile Met Val Tyr Asn
       290                 295                 300
   tgt aca acg gga tct aca aac cct ttt cat tgg ggt gaa gtt gag tat     960
   Cys Thr Thr Gly Ser Thr Asn Pro Phe His Trp Gly Glu Val Glu Tyr
   305                 310                 315                 320
   cac gtc atc tct acc ttc aag aga aac cct ctt gag caa gct ttc aga    1008
   His Val Ile Ser Thr Phe Lys Arg Asn Pro Leu Glu Gln Ala Phe Arg
                   325                 330                 335
   aga cct aac gtg aac ctt acc tcc aat cat cta ctc tac cac tac tgg    1056
   Arg Pro Asn Val Asn Leu Thr Ser Asn His Leu Leu Tyr His Tyr Trp
               340                 345                 350
   att gct gtt tct cat aag gct cct gct ttc ttg tac gac atc tac ctt    1104
   Ile Ala Val Ser His Lys Ala Pro Ala Phe Leu Tyr Asp Ile Tyr Leu
           355                 360                 365
   cga atg act ggt aga agt cct cgg atg atg aag acc att aca cga cta    1152
   Arg Met Thr Gly Arg Ser Pro Arg Met Met Lys Thr Ile Thr Arg Leu
       370                 375                 380
   cac aag gct atg gtc ttc ctt gag tac ttc acc tca aac tca tgg gtt    1200
   His Lys Ala Met Val Phe Leu Glu Tyr Phe Thr Ser Asn Ser Trp Val
   385                 390                 395                 400
   tgg aac act gac aac gtt aac atg ctc atg aac cag ctt aac cct gag    1248
   Trp Asn Thr Asp Asn Val Asn Met Leu Met Asn Gln Leu Asn Pro Glu
                   405                 410                 415
   gac aag aag acg ttc aac att gat gtt cga cag ctt cac tgg gct gag    1296
   Asp Lys Lys Thr Phe Asn Ile Asp Val Arg Gln Leu His Trp Ala Glu
               420                 425                 430
   tac ata gag aac tac tgt atg ggg acg aag aag tac gtt ctt aac gaa      1344
   Tyr Ile Glu Asn Tyr Cys Met Gly Thr Lys Lys Tyr Val Leu Asn Glu
           435                 440                 445
   gag atg tct gga ctt cca gca gct aga aaa cat ctg aac aag ctt cgc      1392
   Glu Met Ser Gly Leu Pro Ala Ala Arg Lys His Leu Asn Lys Leu Arg
       450                 455                 460
   aac atc aga tac ggt ttc aac acc atc ctg gtt atc ctt atc tgg agg      1440
   Asn Ile Arg Tyr Gly Phe Asn Thr Ile Leu Val Ile Leu Ile Trp Arg
   465                 470                 475                 480
   atc ttc atc gct aga agt caa atg gcg aga aac atc tgg tac ttc gtt      1488
   Ile Phe Ile Ala Arg Ser Gln Met Ala Arg Asn Ile Trp Tyr Phe Val
                   485                 490                 495
   gtt tcg ctg tgc tac aag ttc ctt tct tac ttc aga gcc tct tcc aca      1536
   Val Ser Leu Cys Tyr Lys Phe Leu Ser Tyr Phe Arg Ala Ser Ser Thr
               500                 505                 510
   atg cga tac taa                                                      1548
   Met Arg Tyr
           515
<210> 2
   <211> 515
   <212> PRT
   <213> Artificial
<220>
   <223> Synthetic Construct
<400> 2
   Met Val Ser Ile Pro Glu Tyr Tyr Glu Gly Lys Asn Ile Leu Leu Thr
   1               5                   10                  15
   Gly Ala Thr Gly Phe Leu Gly Lys Val Leu Leu Glu Lys Leu Leu Arg
               20                  25                  30
   Ser Cys Pro Arg Val Asn Ser Val Tyr Val Leu Val Arg Gln Lys Ala
           35                  40                  45
   Gly Gln Thr Pro Gln Glu Arg Val Glu Glu Ile Leu Ser Ser Lys Leu
       50                  55                  60
   Phe Asp Arg Leu Arg Asp Glu Asn Pro Asp Phe Arg Glu Lys Ile Ile
   65                  70                  75                  80
   Ala Ile Asn Ser Glu Leu Thr Gln Pro Lys Leu Ala Leu Ser Glu Glu
                   85                  90                  95
   Asp Lys Glu Ile Ile Ile Asp Ser Thr Asn Val Ile Phe His Cys Ala
               100                 105                 110
   Ala Thr Val Arg Phe Asn Glu Asn Leu Arg Asp Ala Val Gln Leu Asn
           115                 120                 125
   Val Ile Ala Thr Arg Gln Leu Ile Leu Leu Ala Gln Gln Met Lys Asn
   130 135 140
   Leu Glu Val Phe Met His Val Ser Thr Ala Tyr Ala Tyr Cys Asn Arg
   145 150 155 160
   Lys His Ile Asp Glu Val Val Tyr Pro Pro Pro Val Asp Pro Lys Lys
   165 170 175
   Leu Ile Asp Ser Leu Glu Trp Met Asp Asp Gly Leu Val Asn Asp Ile
   180 185 190
   Thr Pro Lys Leu Ile Gly Asp Arg Pro Asn Thr Tyr Ile Tyr Thr Lys
   195 200 205
   Ala Leu Ala Glu Tyr Val Val Gln Gln Glu Gly Ala Lys Leu Asn Val
   210 215 220
   Ala Ile Val Arg Pro Ser Ile Val Gly Ala Ser Trp Lys Glu Pro Phe
   225 230 235 240
   Pro Gly Trp Ile Asp Asn Phe Asn Gly Pro Ser Gly Leu Phe Ile Ala
   245 250 255
   Ala Gly Lys Gly Ile Leu Arg Thr Met Arg Ala Ser Asn Asn Ala Leu
   260 265 270
   Ala Asp Leu Val Pro Val Asp Val Val Val Asn Thr Ser Leu Ala Ala
   275 280 285
   Ala Trp Tyr Ser Gly Val Asn Arg Pro Arg Asn Ile Met Val Tyr Asn
   290 295 300
   Cys Thr Thr Gly Ser Thr Asn Pro Phe His Trp Gly Glu Val Glu Tyr
   305 310 315 320
   His Val Ile Ser Thr Phe Lys Arg Asn Pro Leu Glu Gln Ala Phe Arg
   325 330 335
   Arg Pro Asn Val Asn Leu Thr Ser Asn His Leu Leu Tyr His Tyr Trp
   340 345 350
   Ile Ala Val Ser His Lys Ala Pro Ala Phe Leu Tyr Asp Ile Tyr Leu
   355 360 365
   Arg Met Thr Gly Arg Ser Pro Arg Met Met Lys Thr Ile Thr Arg Leu
   370 375 380
   His Lys Ala Met Val Phe Leu Glu Tyr Phe Thr Ser Asn Ser Trp Val
   385 390 395 400
   Trp Asn Thr Asp Asn Val Asn Met Leu Met Asn Gln Leu Asn Pro Glu
                   405                 410                 415
   Asp Lys Lys Thr Phe Asn Ile Asp Val Arg Gln Leu His Trp Ala Glu
               420                 425                 430
   Tyr Ile Glu Asn Tyr Cys Met Gly Thr Lys Lys Tyr Val Leu Asn Glu
           435                 440                 445
   Glu Met Ser Gly Leu Pro Ala Ala Arg Lys His Leu Asn Lys Leu Arg
       450                 455                 460
   Asn Ile Arg Tyr Gly Phe Asn Thr Ile Leu Val Ile Leu Ile Trp Arg
   465                 470                 475                 480
   Ile Phe Ile Ala Arg Ser Gln Met Ala Arg Asn Ile Trp Tyr Phe Val
                   485                 490                 495
   Val Ser Leu Cys Tyr Lys Phe Leu Ser Tyr Phe Arg Ala Ser Ser Thr
               500                 505                 510
   Met Arg Tyr
           515
<210> 3
   <211> 2274
   <212> DNA
   <213> Artificial
<220>
   <223> mouse fatty acyl CoA reductase tagged with mCherry
<220>
   <221> CDS
   <222> (7)..(2268)
<400> 3
   ggatcc atg gtg agc aag ggc gag gag gat aac atg gcc atc atc aag        48
          Met Val Ser Lys Gly Glu Glu Asp Asn Met Ala Ile Ile Lys
          1               5                   10
   gag ttc atg cgc ttc aag gtg cac atg gag ggc tcc gtg aac ggc cac       96
   Glu Phe Met Arg Phe Lys Val His Met Glu Gly Ser Val Asn Gly His
   15                  20                  25                  30
   gag ttc gag atc gag ggc gag ggc gag ggc cgc ccc tac gag ggc acc      144
   Glu Phe Glu Ile Glu Gly Glu Gly Glu Gly Arg Pro Tyr Glu Gly Thr
                   35                  40                  45
   cag acc gcc aag ctg aag gtg acc aag ggt ggc ccc ctg ccc ttc gcc      192
   Gln Thr Ala Lys Leu Lys Val Thr Lys Gly Gly Pro Leu Pro Phe Ala
               50                  55                  60
   tgg gac atc ctg tcc cct cag ttc atg tac ggc tcc aag gcc tac gtg      240
   Trp Asp Ile Leu Ser Pro Gln Phe Met Tyr Gly Ser Lys Ala Tyr Val
           65                  70                  75
   aag cac ccc gcc gac atc ccc gac tac ttg aag ctg tcc ttc ccc gag      288
   Lys His Pro Ala Asp Ile Pro Asp Tyr Leu Lys Leu Ser Phe Pro Glu
       80                  85                  90
   ggc ttc aag tgg gag cgc gtg atg aac ttc gag gac ggc ggc gtg gtg        336
   Gly Phe Lys Trp Glu Arg Val Met Asn Phe Glu Asp Gly Gly Val Val
   95                  100                 105                 110
   acc gtg acc cag gac tcc tcc ctg cag gac ggc gag ttc atc tac aag        384
   Thr Val Thr Gln Asp Ser Ser Leu Gln Asp Gly Glu Phe Ile Tyr Lys
                   115                 120                 125
   gtg aag ctg cgc ggc acc aac ttc ccc tcc gac ggc ccc gta atg cag        432
   Val Lys Leu Arg Gly Thr Asn Phe Pro Ser Asp Gly Pro Val Met Gln
               130                 135                 140
   aag aag acc atg ggc tgg gag gcc tcc tcc gag cgg atg tac ccc gag        480
   Lys Lys Thr Met Gly Trp Glu Ala Ser Ser Glu Arg Met Tyr Pro Glu
           145                 150                 155
   gac ggc gcc ctg aag ggc gag atc aag cag agg ctg aag ctg aag gac        528
   Asp Gly Ala Leu Lys Gly Glu Ile Lys Gln Arg Leu Lys Leu Lys Asp
       160                 165                 170
   ggc ggc cac tac gac gct gag gtc aag acc acc tac aag gcc aag aag        576
   Gly Gly His Tyr Asp Ala Glu Val Lys Thr Thr Tyr Lys Ala Lys Lys
   175                 180                 185                 190
   ccc gtg cag ctg ccc ggc gcc tac aac gtc aac atc aag ttg gac atc        624
   Pro Val Gln Leu Pro Gly Ala Tyr Asn Val Asn Ile Lys Leu Asp Ile
                   195                 200                 205
   acc tcc cac aac gag gac tac acc atc gtg gaa cag tac gaa cgc gcc        672
   Thr Ser His Asn Glu Asp Tyr Thr Ile Val Glu Gln Tyr Glu Arg Ala
               210                 215                 220
   gag ggc cgc cac tcc acc ggc ggc atg gac gag ctg tac aag gaa ttc        720
   Glu Gly Arg His Ser Thr Gly Gly Met Asp Glu Leu Tyr Lys Glu Phe
           225                 230                 235
   atg gtt tcc atc cca gag tac tat gag gga aag aac atc ttg cta act        768
   Met Val Ser Ile Pro Glu Tyr Tyr Glu Gly Lys Asn Ile Leu Leu Thr
       240                 245                 250
   ggt gca act gga ttt ctt ggg aag gtt ttg ctt gag aag ctg cta aga        816
   Gly Ala Thr Gly Phe Leu Gly Lys Val Leu Leu Glu Lys Leu Leu Arg
   255                 260                 265                 270
   tct tgc cct aga gtc aac tcc gtt tac gtt cta gtc aga caa aag gct        864
   Ser Cys Pro Arg Val Asn Ser Val Tyr Val Leu Val Arg Gln Lys Ala
                   275                 280                 285
   gga caa aca cct caa gaa aga gtt gag gag atc ttg tca agc aag ctc        912
   Gly Gln Thr Pro Gln Glu Arg Val Glu Glu Ile Leu Ser Ser Lys Leu
               290                 295                 300
   ttt gat aga ctg cgt gat gag aat cca gat ttc cga gag aag atc atc        960
   Phe Asp Arg Leu Arg Asp Glu Asn Pro Asp Phe Arg Glu Lys Ile Ile
           305                 310                 315
   gcc atc aac tct gaa ttg acc caa cct aag tta gcg ctt tct gaa gag        1008
   Ala Ile Asn Ser Glu Leu Thr Gln Pro Lys Leu Ala Leu Ser Glu Glu
       320                 325                 330
   gat aag gag atc atc atc gac tca acg aac gtt atc ttc cat tgc gct        1056
   Asp Lys Glu Ile Ile Ile Asp Ser Thr Asn Val Ile Phe His Cys Ala
   335                 340                 345                 350
   gca aca gtt cgt ttc aat gag aac cta cgg gat gct gtt caa ttg aac        1104
   Ala Thr Val Arg Phe Asn Glu Asn Leu Arg Asp Ala Val Gln Leu Asn
                   355                 360                 365
   gtc att gct acg aga caa ctg atc tta ctc gct cag cag atg aag aac 1152
   Val Ile Ala Thr Arg Gln Leu Ile Leu Leu Ala Gln Gln Met Lys Asn
   370 375 380
   ctc gaa gtc ttc atg cac gtt agt act gct tac gca tac tgt aac cgc 1200
   Leu Glu Val Phe Met His Val Ser Thr Ala Tyr Ala Tyr Cys Asn Arg
   385 390 395
   aag cac atc gat gaa gtt gtt tac cca cct cca gtt gat cct aag aag 1248
   Lys His Ile Asp Glu Val Val Tyr Pro Pro Pro Val Asp Pro Lys Lys
   400 405 410
   ttg atc gac tct ctt gag tgg atg gat gat gga ctt gtg aac gac ata 1296
   Leu Ile Asp Ser Leu Glu Trp Met Asp Asp Gly Leu Val Asn Asp Ile
   415 420 425 430
   aca cca aag ctt atc gga gac aga cca aac act tac atc tac act aag 1344
   Thr Pro Lys Leu Ile Gly Asp Arg Pro Asn Thr Tyr Ile Tyr Thr Lys
   435 440 445
   gca ctg gct gag tat gtt gtt caa caa gag gga gct aag ttg aac gtt 1392
   Ala Leu Ala Glu Tyr Val Val Gln Gln Glu Gly Ala Lys Leu Asn Val
   450 455 460
   gca atc gtt aga ccg tct att gtt gga gct tca tgg aaa gaa cca ttc 1440
   Ala Ile Val Arg Pro Ser Ile Val Gly Ala Ser Trp Lys Glu Pro Phe
   465 470 475
   cca gga tgg att gac aac ttc aat ggt cca tct gga ctt ttc att gca 1488
   Pro Gly Trp Ile Asp Asn Phe Asn Gly Pro Ser Gly Leu Phe Ile Ala
   480 485 490
   gct ggc aag ggt atc ctc aga act atg aga gca agt aac aac gca ctt 1536
   Ala Gly Lys Gly Ile Leu Arg Thr Met Arg Ala Ser Asn Asn Ala Leu
   495 500 505 510
   gca gat ctt gtt cct gtt gac gtt gtc gtt aac acc tca tta gca gct 1584
   Ala Asp Leu Val Pro Val Asp Val Val Val Asn Thr Ser Leu Ala Ala
   515 520 525
   gca tgg tat tct gga gtt aac aga ccc agg aac atc atg gtg tac aac 1632
   Ala Trp Tyr Ser Gly Val Asn Arg Pro Arg Asn Ile Met Val Tyr Asn
   530 535 540
   tgt aca acg gga tct aca aac cct ttt cat tgg ggt gaa gtt gag tat 1680
   Cys Thr Thr Gly Ser Thr Asn Pro Phe His Trp Gly Glu Val Glu Tyr
   545 550 555
   cac gtc atc tct acc ttc aag aga aac cct ctt gag caa gct ttc aga 1728
   His Val Ile Ser Thr Phe Lys Arg Asn Pro Leu Glu Gln Ala Phe Arg
   560 565 570
   aga cct aac gtg aac ctt acc tcc aat cat cta ctc tac cac tac tgg 1776
   Arg Pro Asn Val Asn Leu Thr Ser Asn His Leu Leu Tyr His Tyr Trp
   575 580 585 590
   att gct gtt tct cat aag gct cct gct ttc ttg tac gac atc tac ctt 1824
   Ile Ala Val Ser His Lys Ala Pro Ala Phe Leu Tyr Asp Ile Tyr Leu
   595 600 605
   cga atg act ggt aga agt cct cgg atg atg aag acc att aca cga cta 1872
   Arg Met Thr Gly Arg Ser Pro Arg Met Met Lys Thr Ile Thr Arg Leu
   610 615 620
   cac aag gct atg gtc ttc ctt gag tac ttc acc tca aac tca tgg gtt 1920
   His Lys Ala Met Val Phe Leu Glu Tyr Phe Thr Ser Asn Ser Trp Val
   625 630 635
   tgg aac act gac aac gtt aac atg ctc atg aac cag ctt aac cct gag 1968
   Trp Asn Thr Asp Asn Val Asn Met Leu Met Asn Gln Leu Asn Pro Glu
       640                 645                 650
   gac aag aag acg ttc aac att gat gtt cga cag ctt cac tgg gct gag     2016
   Asp Lys Lys Thr Phe Asn Ile Asp Val Arg Gln Leu His Trp Ala Glu
   655                 660                 665                 670
   tac ata gag aac tac tgt atg ggg acg aag aag tac gtt ctt aac gaa     2064
   Tyr Ile Glu Asn Tyr Cys Met Gly Thr Lys Lys Tyr Val Leu Asn Glu
                   675                 680                 685
   gag atg tct gga ctt cca gca gct aga aaa cat ctg aac aag ctt cgc     2112
   Glu Met Ser Gly Leu Pro Ala Ala Arg Lys His Leu Asn Lys Leu Arg
               690                 695                 700
   aac atc aga tac ggt ttc aac acc atc ctg gtt atc ctt atc tgg agg     2160
   Asn Ile Arg Tyr Gly Phe Asn Thr Ile Leu Val Ile Leu Ile Trp Arg
           705                 710                 715
   atc ttc atc gct aga agt caa atg gcg aga aac atc tgg tac ttc gtt     2208
   Ile Phe Ile Ala Arg Ser Gln Met Ala Arg Asn Ile Trp Tyr Phe Val
       720                 725                 730
   gtt tcg ctg tgc tac aag ttc ctt tct tac ttc aga gcc tct tcc aca     2256
   Val Ser Leu Cys Tyr Lys Phe Leu Ser Tyr Phe Arg Ala Ser Ser Thr
   735                 740                 745                 750
   atg cga tac taa ctcgag                                              2274
   Met Arg Tyr
<210> 4
   <211> 753
   <212> PRT
   <213> Artificial
<220>
   <223> Synthetic Construct
<400> 4
   Met Val Ser Lys Gly Glu Glu Asp Asn Met Ala Ile Ile Lys Glu Phe
   1               5                   10                  15
   Met Arg Phe Lys Val His Met Glu Gly Ser Val Asn Gly His Glu Phe
               20                  25                  30
   Glu Ile Glu Gly Glu Gly Glu Gly Arg Pro Tyr Glu Gly Thr Gln Thr
           35                  40                  45
   Ala Lys Leu Lys Val Thr Lys Gly Gly Pro Leu Pro Phe Ala Trp Asp
       50                  55                  60
   Ile Leu Ser Pro Gln Phe Met Tyr Gly Ser Lys Ala Tyr Val Lys His
   65                  70                  75                  80
   Pro Ala Asp Ile Pro Asp Tyr Leu Lys Leu Ser Phe Pro Glu Gly Phe
                   85                  90                  95
   Lys Trp Glu Arg Val Met Asn Phe Glu Asp Gly Gly Val Val Thr Val
               100                 105                 110
   Thr Gln Asp Ser Ser Leu Gln Asp Gly Glu Phe Ile Tyr Lys Val Lys
           115                 120                 125
   Leu Arg Gly Thr Asn Phe Pro Ser Asp Gly Pro Val Met Gln Lys Lys
       130                 135                 140
   Thr Met Gly Trp Glu Ala Ser Ser Glu Arg Met Tyr Pro Glu Asp Gly
   145                 150                 155                 160
   Ala Leu Lys Gly Glu Ile Lys Gln Arg Leu Lys Leu Lys Asp Gly Gly
                   165                 170                 175
   His Tyr Asp Ala Glu Val Lys Thr Thr Tyr Lys Ala Lys Lys Pro Val
               180                 185                 190
   Gln Leu Pro Gly Ala Tyr Asn Val Asn Ile Lys Leu Asp Ile Thr Ser
           195                 200                 205
   His Asn Glu Asp Tyr Thr Ile Val Glu Gln Tyr Glu Arg Ala Glu Gly
       210                 215                 220
   Arg His Ser Thr Gly Gly Met Asp Glu Leu Tyr Lys Glu Phe Met Val
   225                 230                 235                 240
   Ser Ile Pro Glu Tyr Tyr Glu Gly Lys Asn Ile Leu Leu Thr Gly Ala
                   245                 250                 255
   Thr Gly Phe Leu Gly Lys Val Leu Leu Glu Lys Leu Leu Arg Ser Cys
               260                 265                 270
   Pro Arg Val Asn Ser Val Tyr Val Leu Val Arg Gln Lys Ala Gly Gln
           275                 280                 285
   Thr Pro Gln Glu Arg Val Glu Glu Ile Leu Ser Ser Lys Leu Phe Asp
       290                 295                 300
   Arg Leu Arg Asp Glu Asn Pro Asp Phe Arg Glu Lys Ile Ile Ala Ile
   305                 310                 315                 320
   Asn Ser Glu Leu Thr Gln Pro Lys Leu Ala Leu Ser Glu Glu Asp Lys
                   325                 330                 335
   Glu Ile Ile Ile Asp Ser Thr Asn Val Ile Phe His Cys Ala Ala Thr
               340                 345                 350
   Val Arg Phe Asn Glu Asn Leu Arg Asp Ala Val Gln Leu Asn Val Ile
           355                 360                 365
   Ala Thr Arg Gln Leu Ile Leu Leu Ala Gln Gln Met Lys Asn Leu Glu
       370                 375                 380
   Val Phe Met His Val Ser Thr Ala Tyr Ala Tyr Cys Asn Arg Lys His
   385 390 395 400
   Ile Asp Glu Val Val Tyr Pro Pro Pro Val Asp Pro Lys Lys Leu Ile
   405 410 415
   Asp Ser Leu Glu Trp Met Asp Asp Gly Leu Val Asn Asp Ile Thr Pro
   420 425 430
   Lys Leu Ile Gly Asp Arg Pro Asn Thr Tyr Ile Tyr Thr Lys Ala Leu
   435 440 445
   Ala Glu Tyr Val Val Gln Gln Glu Gly Ala Lys Leu Asn Val Ala Ile
   450 455 460
   Val Arg Pro Ser Ile Val Gly Ala Ser Trp Lys Glu Pro Phe Pro Gly
   465 470 475 480
   Trp Ile Asp Asn Phe Asn Gly Pro Ser Gly Leu Phe Ile Ala Ala Gly
   485 490 495
   Lys Gly Ile Leu Arg Thr Met Arg Ala Ser Asn Asn Ala Leu Ala Asp
   500 505 510
   Leu Val Pro Val Asp Val Val Val Asn Thr Ser Leu Ala Ala Ala Trp
   515 520 525
   Tyr Ser Gly Val Asn Arg Pro Arg Asn Ile Met Val Tyr Asn Cys Thr
   530 535 540
   Thr Gly Ser Thr Asn Pro Phe His Trp Gly Glu Val Glu Tyr His Val
   545 550 555 560
   Ile Ser Thr Phe Lys Arg Asn Pro Leu Glu Gln Ala Phe Arg Arg Pro
   565 570 575
   Asn Val Asn Leu Thr Ser Asn His Leu Leu Tyr His Tyr Trp Ile Ala
   580 585 590
   Val Ser His Lys Ala Pro Ala Phe Leu Tyr Asp Ile Tyr Leu Arg Met
   595 600 605
   Thr Gly Arg Ser Pro Arg Met Met Lys Thr Ile Thr Arg Leu His Lys
   610 615 620
   Ala Met Val Phe Leu Glu Tyr Phe Thr Ser Asn Ser Trp Val Trp Asn
   625 630 635 640
   Thr Asp Asn Val Asn Met Leu Met Asn Gln Leu Asn Pro Glu Asp Lys
   645 650 655
   Lys Thr Phe Asn Ile Asp Val Arg Gln Leu His Trp Ala Glu Tyr Ile
           660                 665                 670
   Glu Asn Tyr Cys Met Gly Thr Lys Lys Tyr Val Leu Asn Glu Glu Met
           675                 680                 685
   Ser Gly Leu Pro Ala Ala Arg Lys His Leu Asn Lys Leu Arg Asn Ile
       690                 695                 700
   Arg Tyr Gly Phe Asn Thr Ile Leu Val Ile Leu Ile Trp Arg Ile Phe
   705                 710                 715                 720
   Ile Ala Arg Ser Gln Met Ala Arg Asn Ile Trp Tyr Phe Val Val Ser
                   725                 730                 735
   Leu Cys Tyr Lys Phe Leu Ser Tyr Phe Arg Ala Ser Ser Thr Met Arg
               740                 745                 750
   Tyr
<210> 5
   <211> 1404
   <212> DNA
   <213> Artificial
<220>
   <223> mouse fatty acyl CoA reductase truncated
<220>
   <221> CDS
   <222> (1)..(1404)
<400> 5
   atg gtt tcc atc cca gag tac tat gag gga aag aac atc ttg cta act       48
   Met Val Ser Ile Pro Glu Tyr Tyr Glu Gly Lys Asn Ile Leu Leu Thr
   1               5                   10                  15
   ggt gca act gga ttt ctt ggg aag gtt ttg ctt gag aag ctg cta aga       96
   Gly Ala Thr Gly Phe Leu Gly Lys Val Leu Leu Glu Lys Leu Leu Arg
               20                  25                  30
   tct tgc cct aga gtc aac tcc gtt tac gtt cta gtc aga caa aag gct      144
   Ser Cys Pro Arg Val Asn Ser Val Tyr Val Leu Val Arg Gln Lys Ala
           35                  40                  45
   gga caa aca cct caa gaa aga gtt gag gag atc ttg tca agc aag ctc      192
   Gly Gln Thr Pro Gln Glu Arg Val Glu Glu Ile Leu Ser Ser Lys Leu
       50                  55                  60
   ttt gat aga ctg cgt gat gag aat cca gat ttc cga gag aag atc atc      240
   Phe Asp Arg Leu Arg Asp Glu Asn Pro Asp Phe Arg Glu Lys Ile Ile
   65                  70                  75                  80
   gcc atc aac tct gaa ttg acc caa cct aag tta gcg ctt tct gaa gag      288
   Ala Ile Asn Ser Glu Leu Thr Gln Pro Lys Leu Ala Leu Ser Glu Glu
                   85                  90                  95
   gat aag gag atc atc atc gac tca acg aac gtt atc ttc cat tgc gct      336
   Asp Lys Glu Ile Ile Ile Asp Ser Thr Asn Val Ile Phe His Cys Ala
               100                 105                 110
   gca aca gtt cgt ttc aat gag aac cta cgg gat gct gtt caa ttg aac       384
   Ala Thr Val Arg Phe Asn Glu Asn Leu Arg Asp Ala Val Gln Leu Asn
           115                 120                 125
   gtc att gct acg aga caa ctg atc tta ctc gct cag cag atg aag aac       432
   Val Ile Ala Thr Arg Gln Leu Ile Leu Leu Ala Gln Gln Met Lys Asn
       130                 135                 140
   ctc gaa gtc ttc atg cac gtt agt act gct tac gca tac tgt aac cgc       480
   Leu Glu Val Phe Met His Val Ser Thr Ala Tyr Ala Tyr Cys Asn Arg
   145                 150                 155                 160
   aag cac atc gat gaa gtt gtt tac cca cct cca gtt gat cct aag aag       528
   Lys His Ile Asp Glu Val Val Tyr Pro Pro Pro Val Asp Pro Lys Lys
                   165                 170                 175
   ttg atc gac tct ctt gag tgg atg gat gat gga ctt gtg aac gac ata       576
   Leu Ile Asp Ser Leu Glu Trp Met Asp Asp Gly Leu Val Asn Asp Ile
               180                 185                 190
   aca cca aag ctt atc gga gac aga cca aac act tac atc tac act aag       624
   Thr Pro Lys Leu Ile Gly Asp Arg Pro Asn Thr Tyr Ile Tyr Thr Lys
           195                 200                 205
   gca ctg gct gag tat gtt gtt caa caa gag gga gct aag ttg aac gtt       672
   Ala Leu Ala Glu Tyr Val Val Gln Gln Glu Gly Ala Lys Leu Asn Val
       210                 215                 220
   gca atc gtt aga ccg tct att gtt gga gct tca tgg aaa gaa cca ttc       720
   Ala Ile Val Arg Pro Ser Ile Val Gly Ala Ser Trp Lys Glu Pro Phe
   225                 230                 235                 240
   cca gga tgg att gac aac ttc aat ggt cca tct gga ctt ttc att gca       768
   Pro Gly Trp Ile Asp Asn Phe Asn Gly Pro Ser Gly Leu Phe Ile Ala
                   245                 250                 255
   gct ggc aag ggt atc ctc aga act atg aga gca agt aac aac gca ctt       816
   Ala Gly Lys Gly Ile Leu Arg Thr Met Arg Ala Ser Asn Asn Ala Leu
               260                 265                 270
   gca gat ctt gtt cct gtt gac gtt gtc gtt aac acc tca tta gca gct       864
   Ala Asp Leu Val Pro Val Asp Val Val Val Asn Thr Ser Leu Ala Ala
           275                 280                 285
   gca tgg tat tct gga gtt aac aga ccc agg aac atc atg gtg tac aac       912
   Ala Trp Tyr Ser Gly Val Asn Arg Pro Arg Asn Ile Met Val Tyr Asn
       290                 295                 300
   tgt aca acg gga tct aca aac cct ttt cat tgg ggt gaa gtt gag tat       960
   Cys Thr Thr Gly Ser Thr Asn Pro Phe His Trp Gly Glu Val Glu Tyr
   305                 310                 315                 320
   cac gtc atc tct acc ttc aag aga aac cct ctt gag caa gct ttc aga      1008
   His Val Ile Ser Thr Phe Lys Arg Asn Pro Leu Glu Gln Ala Phe Arg
                   325                 330                 335
   aga cct aac gtg aac ctt acc tcc aat cat cta ctc tac cac tac tgg      1056
   Arg Pro Asn Val Asn Leu Thr Ser Asn His Leu Leu Tyr His Tyr Trp
               340                 345                 350
   att gct gtt tct cat aag gct cct gct ttc ttg tac gac atc tac ctt      1104
   Ile Ala Val Ser His Lys Ala Pro Ala Phe Leu Tyr Asp Ile Tyr Leu
           355                 360                 365
   cga atg act ggt aga agt cct cgg atg atg aag acc att aca cga cta      1152
   Arg Met Thr Gly Arg Ser Pro Arg Met Met Lys Thr Ile Thr Arg Leu
       370                 375                 380
   cac aag gct atg gtc ttc ctt gag tac ttc acc tca aac tca tgg gtt      1200
   His Lys Ala Met Val Phe Leu Glu Tyr Phe Thr Ser Asn Ser Trp Val
   385                 390                 395                 400
   tgg aac act gac aac gtt aac atg ctc atg aac cag ctt aac cct gag      1248
   Trp Asn Thr Asp Asn Val Asn Met Leu Met Asn Gln Leu Asn Pro Glu
                   405                 410                 415
   gac aag aag acg ttc aac att gat gtt cga cag ctt cac tgg gct gag      1296
   Asp Lys Lys Thr Phe Asn Ile Asp Val Arg Gln Leu His Trp Ala Glu
               420                 425                 430
   tac ata gag aac tac tgt atg ggg acg aag aag tac gtt ctt aac gaa      1344
   Tyr Ile Glu Asn Tyr Cys Met Gly Thr Lys Lys Tyr Val Leu Asn Glu
           435                 440                 445
   gag atg tct gga ctt cca gca gct aga aaa cat ctg aac aag ctt cgc      1392
   Glu Met Ser Gly Leu Pro Ala Ala Arg Lys His Leu Asn Lys Leu Arg
       450                 455                 460
   aac atc aga taa                                                      1404
   Asn Ile Arg
   465
<210> 6
   <211> 467
   <212> PRT
   <213> Artificial
<220>
   <223> Synthetic Construct
<400> 6
   Met Val Ser Ile Pro Glu Tyr Tyr Glu Gly Lys Asn Ile Leu Leu Thr
   1               5                   10                  15
   Gly Ala Thr Gly Phe Leu Gly Lys Val Leu Leu Glu Lys Leu Leu Arg
               20                  25                  30
   Ser Cys Pro Arg Val Asn Ser Val Tyr Val Leu Val Arg Gln Lys Ala
           35                  40                  45
   Gly Gln Thr Pro Gln Glu Arg Val Glu Glu Ile Leu Ser Ser Lys Leu
       50                  55                  60
   Phe Asp Arg Leu Arg Asp Glu Asn Pro Asp Phe Arg Glu Lys Ile Ile
   65                  70                  75                  80
   Ala Ile Asn Ser Glu Leu Thr Gln Pro Lys Leu Ala Leu Ser Glu Glu
                   85                  90                  95
   Asp Lys Glu Ile Ile Ile Asp Ser Thr Asn Val Ile Phe His Cys Ala
               100                 105                 110
   Ala Thr Val Arg Phe Asn Glu Asn Leu Arg Asp Ala Val Gln Leu Asn
           115                 120                 125
   Val Ile Ala Thr Arg Gln Leu Ile Leu Leu Ala Gln Gln Met Lys Asn
       130                 135                 140
   Leu Glu Val Phe Met His Val Ser Thr Ala Tyr Ala Tyr Cys Asn Arg
   145                 150                 155                 160
   Lys His Ile Asp Glu Val Val Tyr Pro Pro Pro Val Asp Pro Lys Lys
                   165                 170                 175
   Leu Ile Asp Ser Leu Glu Trp Met Asp Asp Gly Leu Val Asn Asp Ile
               180                 185                 190
   Thr Pro Lys Leu Ile Gly Asp Arg Pro Asn Thr Tyr Ile Tyr Thr Lys
           195                 200                 205
   Ala Leu Ala Glu Tyr Val Val Gln Gln Glu Gly Ala Lys Leu Asn Val
       210                 215                 220
   Ala Ile Val Arg Pro Ser Ile Val Gly Ala Ser Trp Lys Glu Pro Phe
   225                 230                 235                 240
   Pro Gly Trp Ile Asp Asn Phe Asn Gly Pro Ser Gly Leu Phe Ile Ala
                   245                 250                 255
   Ala Gly Lys Gly Ile Leu Arg Thr Met Arg Ala Ser Asn Asn Ala Leu
               260                 265                 270
   Ala Asp Leu Val Pro Val Asp Val Val Val Asn Thr Ser Leu Ala Ala
           275                 280                 285
   Ala Trp Tyr Ser Gly Val Asn Arg Pro Arg Asn Ile Met Val Tyr Asn
       290                 295                 300
   Cys Thr Thr Gly Ser Thr Asn Pro Phe His Trp Gly Glu Val Glu Tyr
   305                 310                 315                 320
   His Val Ile Ser Thr Phe Lys Arg Asn Pro Leu Glu Gln Ala Phe Arg
                   325                 330                 335
   Arg Pro Asn Val Asn Leu Thr Ser Asn His Leu Leu Tyr His Tyr Trp
               340                 345                 350
   Ile Ala Val Ser His Lys Ala Pro Ala Phe Leu Tyr Asp Ile Tyr Leu
           355                 360                 365
   Arg Met Thr Gly Arg Ser Pro Arg Met Met Lys Thr Ile Thr Arg Leu
       370                 375                 380
   His Lys Ala Met Val Phe Leu Glu Tyr Phe Thr Ser Asn Ser Trp Val
   385                 390                 395                 400
   Trp Asn Thr Asp Asn Val Asn Met Leu Met Asn Gln Leu Asn Pro Glu
                   405                 410                 415
   Asp Lys Lys Thr Phe Asn Ile Asp Val Arg Gln Leu His Trp Ala Glu
               420                 425                 430
   Tyr Ile Glu Asn Tyr Cys Met Gly Thr Lys Lys Tyr Val Leu Asn Glu
           435                 440                 445
   Glu Met Ser Gly Leu Pro Ala Ala Arg Lys His Leu Asn Lys Leu Arg
       450                 455                 460
   Asn Ile Arg
   465
<210> 7
   <211> 2130
   <212> DNA
   <213> Artificial
<220>
   <223> mouse fatty acyl CoA reductase tagged with mCherry
<220>
   <221> CDS
   <222> (7)..(2124)
<400> 7
   ggatcc atg gtg agc aag ggc gag gag gat aac atg gcc atc atc aag        48
          Met Val Ser Lys Gly Glu Glu Asp Asn Met Ala Ile Ile Lys
          1               5                   10
   gag ttc atg cgc ttc aag gtg cac atg gag ggc tcc gtg aac ggc cac       96
   Glu Phe Met Arg Phe Lys Val His Met Glu Gly Ser Val Asn Gly His
   15                  20                  25                  30
   gag ttc gag atc gag ggc gag ggc gag ggc cgc ccc tac gag ggc acc      144
   Glu Phe Glu Ile Glu Gly Glu Gly Glu Gly Arg Pro Tyr Glu Gly Thr
                   35                  40                  45
   cag acc gcc aag ctg aag gtg acc aag ggt ggc ccc ctg ccc ttc gcc      192
   Gln Thr Ala Lys Leu Lys Val Thr Lys Gly Gly Pro Leu Pro Phe Ala
               50                  55                  60
   tgg gac atc ctg tcc cct cag ttc atg tac ggc tcc aag gcc tac gtg      240
   Trp Asp Ile Leu Ser Pro Gln Phe Met Tyr Gly Ser Lys Ala Tyr Val
           65                  70                  75
   aag cac ccc gcc gac atc ccc gac tac ttg aag ctg tcc ttc ccc gag      288
   Lys His Pro Ala Asp Ile Pro Asp Tyr Leu Lys Leu Ser Phe Pro Glu
       80                  85                  90
   ggc ttc aag tgg gag cgc gtg atg aac ttc gag gac ggc ggc gtg gtg      336
   Gly Phe Lys Trp Glu Arg Val Met Asn Phe Glu Asp Gly Gly Val Val
   95                  100                 105                 110
   acc gtg acc cag gac tcc tcc ctg cag gac ggc gag ttc atc tac aag      384
   Thr Val Thr Gln Asp Ser Ser Leu Gln Asp Gly Glu Phe Ile Tyr Lys
                   115                 120                 125
   gtg aag ctg cgc ggc acc aac ttc ccc tcc gac ggc ccc gta atg cag      432
   Val Lys Leu Arg Gly Thr Asn Phe Pro Ser Asp Gly Pro Val Met Gln
               130                 135                 140
   aag aag acc atg ggc tgg gag gcc tcc tcc gag cgg atg tac ccc gag      480
   Lys Lys Thr Met Gly Trp Glu Ala Ser Ser Glu Arg Met Tyr Pro Glu
           145                 150                 155
   gac ggc gcc ctg aag ggc gag atc aag cag agg ctg aag ctg aag gac      528
   Asp Gly Ala Leu Lys Gly Glu Ile Lys Gln Arg Leu Lys Leu Lys Asp
       160                 165                 170
   ggc ggc cac tac gac gct gag gtc aag acc acc tac aag gcc aag aag      576
   Gly Gly His Tyr Asp Ala Glu Val Lys Thr Thr Tyr Lys Ala Lys Lys
   175                 180                 185                 190
   ccc gtg cag ctg ccc ggc gcc tac aac gtc aac atc aag ttg gac atc      624
   Pro Val Gln Leu Pro Gly Ala Tyr Asn Val Asn Ile Lys Leu Asp Ile
                   195                 200                 205
   acc tcc cac aac gag gac tac acc atc gtg gaa cag tac gaa cgc gcc      672
   Thr Ser His Asn Glu Asp Tyr Thr Ile Val Glu Gln Tyr Glu Arg Ala
               210                 215                 220
   gag ggc cgc cac tcc acc ggc ggc atg gac gag ctg tac aag gaa ttc      720
   Glu Gly Arg His Ser Thr Gly Gly Met Asp Glu Leu Tyr Lys Glu Phe
           225                 230                 235
   atg gtt tcc atc cca gag tac tat gag gga aag aac atc ttg cta act      768
   Met Val Ser Ile Pro Glu Tyr Tyr Glu Gly Lys Asn Ile Leu Leu Thr
       240                 245                 250
   ggt gca act gga ttt ctt ggg aag gtt ttg ctt gag aag ctg cta aga      816
   Gly Ala Thr Gly Phe Leu Gly Lys Val Leu Leu Glu Lys Leu Leu Arg
   255                 260                 265                 270
   tct tgc cct aga gtc aac tcc gtt tac gtt cta gtc aga caa aag gct      864
   Ser Cys Pro Arg Val Asn Ser Val Tyr Val Leu Val Arg Gln Lys Ala
                   275                 280                 285
   gga caa aca cct caa gaa aga gtt gag gag atc ttg tca agc aag ctc      912
   Gly Gln Thr Pro Gln Glu Arg Val Glu Glu Ile Leu Ser Ser Lys Leu
               290                 295                 300
   ttt gat aga ctg cgt gat gag aat cca gat ttc cga gag aag atc atc      960
   Phe Asp Arg Leu Arg Asp Glu Asn Pro Asp Phe Arg Glu Lys Ile Ile
           305                 310                 315
   gcc atc aac tct gaa ttg acc caa cct aag tta gcg ctt tct gaa gag     1008
   Ala Ile Asn Ser Glu Leu Thr Gln Pro Lys Leu Ala Leu Ser Glu Glu
       320                 325                 330
   gat aag gag atc atc atc gac tca acg aac gtt atc ttc cat tgc gct     1056
   Asp Lys Glu Ile Ile Ile Asp Ser Thr Asn Val Ile Phe His Cys Ala
   335                 340                 345                 350
   gca aca gtt cgt ttc aat gag aac cta cgg gat gct gtt caa ttg aac     1104
   Ala Thr Val Arg Phe Asn Glu Asn Leu Arg Asp Ala Val Gln Leu Asn
                   355                 360                 365
   gtc att gct acg aga caa ctg atc tta ctc gct cag cag atg aag aac     1152
   Val Ile Ala Thr Arg Gln Leu Ile Leu Leu Ala Gln Gln Met Lys Asn
               370                 375                 380
   ctc gaa gtc ttc atg cac gtt agt act gct tac gca tac tgt aac cgc     1200
   Leu Glu Val Phe Met His Val Ser Thr Ala Tyr Ala Tyr Cys Asn Arg
           385                 390                 395
   aag cac atc gat gaa gtt gtt tac cca cct cca gtt gat cct aag aag     1248
   Lys His Ile Asp Glu Val Val Tyr Pro Pro Pro Val Asp Pro Lys Lys
       400                 405                 410
   ttg atc gac tct ctt gag tgg atg gat gat gga ctt gtg aac gac ata     1296
   Leu Ile Asp Ser Leu Glu Trp Met Asp Asp Gly Leu Val Asn Asp Ile
   415                 420                 425                 430
   aca cca aag ctt atc gga gac aga cca aac act tac atc tac act aag      1344
   Thr Pro Lys Leu Ile Gly Asp Arg Pro Asn Thr Tyr Ile Tyr Thr Lys
                   435                 440                 445
   gca ctg gct gag tat gtt gtt caa caa gag gga gct aag ttg aac gtt      1392
   Ala Leu Ala Glu Tyr Val Val Gln Gln Glu Gly Ala Lys Leu Asn Val
               450                 455                 460
   gca atc gtt aga ccg tct att gtt gga gct tca tgg aaa gaa cca ttc      1440
   Ala Ile Val Arg Pro Ser Ile Val Gly Ala Ser Trp Lys Glu Pro Phe
           465                 470                 475
   cca gga tgg att gac aac ttc aat ggt cca tct gga ctt ttc att gca      1488
   Pro Gly Trp Ile Asp Asn Phe Asn Gly Pro Ser Gly Leu Phe Ile Ala
       480                 485                 490
   gct ggc aag ggt atc ctc aga act atg aga gca agt aac aac gca ctt      1536
   Ala Gly Lys Gly Ile Leu Arg Thr Met Arg Ala Ser Asn Asn Ala Leu
   495                 500                 505                 510
   gca gat ctt gtt cct gtt gac gtt gtc gtt aac acc tca tta gca gct      1584
   Ala Asp Leu Val Pro Val Asp Val Val Val Asn Thr Ser Leu Ala Ala
                   515                 520                 525
   gca tgg tat tct gga gtt aac aga ccc agg aac atc atg gtg tac aac      1632
   Ala Trp Tyr Ser Gly Val Asn Arg Pro Arg Asn Ile Met Val Tyr Asn
               530                 535                 540
   tgt aca acg gga tct aca aac cct ttt cat tgg ggt gaa gtt gag tat      1680
   Cys Thr Thr Gly Ser Thr Asn Pro Phe His Trp Gly Glu Val Glu Tyr
           545                 550                 555
   cac gtc atc tct acc ttc aag aga aac cct ctt gag caa gct ttc aga      1728
   His Val Ile Ser Thr Phe Lys Arg Asn Pro Leu Glu Gln Ala Phe Arg
       560                 565                 570
   aga cct aac gtg aac ctt acc tcc aat cat cta ctc tac cac tac tgg      1776
   Arg Pro Asn Val Asn Leu Thr Ser Asn His Leu Leu Tyr His Tyr Trp
   575                 580                 585                 590
   att gct gtt tct cat aag gct cct gct ttc ttg tac gac atc tac ctt      1824
   Ile Ala Val Ser His Lys Ala Pro Ala Phe Leu Tyr Asp Ile Tyr Leu
                   595                 600                 605
   cga atg act ggt aga agt cct cgg atg atg aag acc att aca cga cta      1872
   Arg Met Thr Gly Arg Ser Pro Arg Met Met Lys Thr Ile Thr Arg Leu
               610                 615                 620
   cac aag gct atg gtc ttc ctt gag tac ttc acc tca aac tca tgg gtt      1920
   His Lys Ala Met Val Phe Leu Glu Tyr Phe Thr Ser Asn Ser Trp Val
           625                 630                 635
   tgg aac act gac aac gtt aac atg ctc atg aac cag ctt aac cct gag      1968
   Trp Asn Thr Asp Asn Val Asn Met Leu Met Asn Gln Leu Asn Pro Glu
       640                 645                 650
   gac aag aag acg ttc aac att gat gtt cga cag ctt cac tgg gct gag      2016
   Asp Lys Lys Thr Phe Asn Ile Asp Val Arg Gln Leu His Trp Ala Glu
   655                 660                 665                 670
   tac ata gag aac tac tgt atg ggg acg aag aag tac gtt ctt aac gaa      2064
   Tyr Ile Glu Asn Tyr Cys Met Gly Thr Lys Lys Tyr Val Leu Asn Glu
                   675                 680                 685
   gag atg tct gga ctt cca gca gct aga aaa cat ctg aac aag ctt cgc      2112
   Glu Met Ser Gly Leu Pro Ala Ala Arg Lys His Leu Asn Lys Leu Arg
               690                 695                 700
   aac atc aga taa ctcgag                                               2130
   Asn Ile Arg
           705
<210> 8
   <211> 705
   <212> PRT
   <213> Artificial
<220>
   <223> Synthetic Construct
<400> 8
   Met Val Ser Lys Gly Glu Glu Asp Asn Met Ala Ile Ile Lys Glu Phe
   1               5                   10                  15
   Met Arg Phe Lys Val His Met Glu Gly Ser Val Asn Gly His Glu Phe
               20                  25                  30
   Glu Ile Glu Gly Glu Gly Glu Gly Arg Pro Tyr Glu Gly Thr Gln Thr
           35                  40                  45
   Ala Lys Leu Lys Val Thr Lys Gly Gly Pro Leu Pro Phe Ala Trp Asp
       50                  55                  60
   Ile Leu Ser Pro Gln Phe Met Tyr Gly Ser Lys Ala Tyr Val Lys His
   65                  70                  75                  80
   Pro Ala Asp Ile Pro Asp Tyr Leu Lys Leu Ser Phe Pro Glu Gly Phe
                   85                  90                  95
   Lys Trp Glu Arg Val Met Asn Phe Glu Asp Gly Gly Val Val Thr Val
               100                 105                 110
   Thr Gln Asp Ser Ser Leu Gln Asp Gly Glu Phe Ile Tyr Lys Val Lys
           115                 120                 125
   Leu Arg Gly Thr Asn Phe Pro Ser Asp Gly Pro Val Met Gln Lys Lys
       130                 135                 140
   Thr Met Gly Trp Glu Ala Ser Ser Glu Arg Met Tyr Pro Glu Asp Gly
   145                 150                 155                 160
   Ala Leu Lys Gly Glu Ile Lys Gln Arg Leu Lys Leu Lys Asp Gly Gly
                   165                 170                 175
   His Tyr Asp Ala Glu Val Lys Thr Thr Tyr Lys Ala Lys Lys Pro Val
               180                 185                 190
   Gln Leu Pro Gly Ala Tyr Asn Val Asn Ile Lys Leu Asp Ile Thr Ser
           195                 200                 205
   His Asn Glu Asp Tyr Thr Ile Val Glu Gln Tyr Glu Arg Ala Glu Gly
       210                 215                 220
   Arg His Ser Thr Gly Gly Met Asp Glu Leu Tyr Lys Glu Phe Met Val
   225 230 235 240
   Ser Ile Pro Glu Tyr Tyr Glu Gly Lys Asn Ile Leu Leu Thr Gly Ala
   245 250 255
   Thr Gly Phe Leu Gly Lys Val Leu Leu Glu Lys Leu Leu Arg Ser Cys
   260 265 270
   Pro Arg Val Asn Ser Val Tyr Val Leu Val Arg Gln Lys Ala Gly Gln
   275 280 285
   Thr Pro Gln Glu Arg Val Glu Glu Ile Leu Ser Ser Lys Leu Phe Asp
   290 295 300
   Arg Leu Arg Asp Glu Asn Pro Asp Phe Arg Glu Lys Ile Ile Ala Ile
   305 310 315 320
   Asn Ser Glu Leu Thr Gln Pro Lys Leu Ala Leu Ser Glu Glu Asp Lys
   325 330 335
   Glu Ile Ile Ile Asp Ser Thr Asn Val Ile Phe His Cys Ala Ala Thr
   340 345 350
   Val Arg Phe Asn Glu Asn Leu Arg Asp Ala Val Gln Leu Asn Val Ile
   355 360 365
   Ala Thr Arg Gln Leu Ile Leu Leu Ala Gln Gln Met Lys Asn Leu Glu
   370 375 380
   Val Phe Met His Val Ser Thr Ala Tyr Ala Tyr Cys Asn Arg Lys His
   385 390 395 400
   Ile Asp Glu Val Val Tyr Pro Pro Pro Val Asp Pro Lys Lys Leu Ile
   405 410 415
   Asp Ser Leu Glu Trp Met Asp Asp Gly Leu Val Asn Asp Ile Thr Pro
   420 425 430
   Lys Leu Ile Gly Asp Arg Pro Asn Thr Tyr Ile Tyr Thr Lys Ala Leu
   435 440 445
   Ala Glu Tyr Val Val Gln Gln Glu Gly Ala Lys Leu Asn Val Ala Ile
   450 455 460
   Val Arg Pro Ser Ile Val Gly Ala Ser Trp Lys Glu Pro Phe Pro Gly
   465 470 475 480
   Trp Ile Asp Asn Phe Asn Gly Pro Ser Gly Leu Phe Ile Ala Ala Gly
   485 490 495
   Lys Gly Ile Leu Arg Thr Met Arg Ala Ser Asn Asn Ala Leu Ala Asp
               500                 505                 510
   Leu Val Pro Val Asp Val Val Val Asn Thr Ser Leu Ala Ala Ala Trp
           515                 520                 525
   Tyr Ser Gly Val Asn Arg Pro Arg Asn Ile Met Val Tyr Asn Cys Thr
       530                 535                 540
   Thr Gly Ser Thr Asn Pro Phe His Trp Gly Glu Val Glu Tyr His Val
   545                 550                 555                 560
   Ile Ser Thr Phe Lys Arg Asn Pro Leu Glu Gln Ala Phe Arg Arg Pro
                   565                 570                 575
   Asn Val Asn Leu Thr Ser Asn His Leu Leu Tyr His Tyr Trp Ile Ala
               580                 585                 590
   Val Ser His Lys Ala Pro Ala Phe Leu Tyr Asp Ile Tyr Leu Arg Met
           595                 600                 605
   Thr Gly Arg Ser Pro Arg Met Met Lys Thr Ile Thr Arg Leu His Lys
       610                 615                 620
   Ala Met Val Phe Leu Glu Tyr Phe Thr Ser Asn Ser Trp Val Trp Asn
   625                 630                 635                 640
   Thr Asp Asn Val Asn Met Leu Met Asn Gln Leu Asn Pro Glu Asp Lys
                   645                 650                 655
   Lys Thr Phe Asn Ile Asp Val Arg Gln Leu His Trp Ala Glu Tyr Ile
               660                 665                 670
   Glu Asn Tyr Cys Met Gly Thr Lys Lys Tyr Val Leu Asn Glu Glu Met
           675                 680                 685
   Ser Gly Leu Pro Ala Ala Arg Lys His Leu Asn Lys Leu Arg Asn Ile
       690                 695                 700
   Arg
   705
<210> 9
   <211> 2559
   <212> DNA
   <213> Artificial
<220>
   <223> mouse fatty acyl CoA reductase truncated, tagged with mcherry and fused to oleosin 3At
<220>
   <221> CDS
   <222> (7)..(2553)
<400> 9
   gtcgac atg gcg gac caa aca aga acc cat cac gag atg ata agc cga        48
          Met Ala Asp Gln Thr Arg Thr His His Glu Met Ile Ser Arg
          1               5                   10
   gac agt acc caa gag gcc cat ccg aag gcc agg cag atg gtg aag gca       96
   Asp Ser Thr Gln Glu Ala His Pro Lys Ala Arg Gln Met Val Lys Ala
   15                  20                  25                  30
   gca acc gct gtc aca gcc ggt gga tcc cta ctt gtc ctc tcc ggc tta      144
   Ala Thr Ala Val Thr Ala Gly Gly Ser Leu Leu Val Leu Ser Gly Leu
                   35                  40                  45
   aca ctc gct gga aca gtc atc gca ctc acg gtg gct act cct ctc ctc      192
   Thr Leu Ala Gly Thr Val Ile Ala Leu Thr Val Ala Thr Pro Leu Leu
               50                  55                  60
   gtc atc ttc agc ccc gtc ttg gtt cca gca gtg gta acc gtt gct ctc      240
   Val Ile Phe Ser Pro Val Leu Val Pro Ala Val Val Thr Val Ala Leu
           65                  70                  75
   atc att acc gga ttc ctt gca tcc ggt ggc ttt gga ata gcc gcc att      288
   Ile Ile Thr Gly Phe Leu Ala Ser Gly Gly Phe Gly Ile Ala Ala Ile
       80                  85                  90
   acc gcc ttc tct tgg ctc tac agg cac atg acg gga tct gga tcg gat      336
   Thr Ala Phe Ser Trp Leu Tyr Arg His Met Thr Gly Ser Gly Ser Asp
   95                  100                 105                 110
   aag ata gag aat gct cgg atg aag gtt gga agc aga gtg cag gat act      384
   Lys Ile Glu Asn Ala Arg Met Lys Val Gly Ser Arg Val Gln Asp Thr
                   115                 120                 125
   aag tat ggg cag cac aac att gga gtc caa cac caa caa gtt tct gga      432
   Lys Tyr Gly Gln His Asn Ile Gly Val Gln His Gln Gln Val Ser Gly
               130                 135                 140
   tcc atg gtg agc aag ggc gag gag gat aac atg gcc atc atc aag gag      480
   Ser Met Val Ser Lys Gly Glu Glu Asp Asn Met Ala Ile Ile Lys Glu
           145                 150                 155
   ttc atg cgc ttc aag gtg cac atg gag ggc tcc gtg aac ggc cac gag      528
   Phe Met Arg Phe Lys Val His Met Glu Gly Ser Val Asn Gly His Glu
       160                 165                 170
   ttc gag atc gag ggc gag ggc gag ggc cgc ccc tac gag ggc acc cag      576
   Phe Glu Ile Glu Gly Glu Gly Glu Gly Arg Pro Tyr Glu Gly Thr Gln
   175                 180                 185                 190
   acc gcc aag ctg aag gtg acc aag ggt ggc ccc ctg ccc ttc gcc tgg      624
   Thr Ala Lys Leu Lys Val Thr Lys Gly Gly Pro Leu Pro Phe Ala Trp
                   195                 200                 205
   gac atc ctg tcc cct cag ttc atg tac ggc tcc aag gcc tac gtg aag      672
   Asp Ile Leu Ser Pro Gln Phe Met Tyr Gly Ser Lys Ala Tyr Val Lys
               210                 215                 220
   cac ccc gcc gac atc ccc gac tac ttg aag ctg tcc ttc ccc gag ggc      720
   His Pro Ala Asp Ile Pro Asp Tyr Leu Lys Leu Ser Phe Pro Glu Gly
           225                 230                 235
   ttc aag tgg gag cgc gtg atg aac ttc gag gac ggc ggc gtg gtg acc      768
   Phe Lys Trp Glu Arg Val Met Asn Phe Glu Asp Gly Gly Val Val Thr
       240                 245                 250
   gtg acc cag gac tcc tcc ctg cag gac ggc gag ttc atc tac aag gtg      816
   Val Thr Gln Asp Ser Ser Leu Gln Asp Gly Glu Phe Ile Tyr Lys Val
   255                 260                 265                 270
   aag ctg cgc ggc acc aac ttc ccc tcc gac ggc ccc gta atg cag aag      864
   Lys Leu Arg Gly Thr Asn Phe Pro Ser Asp Gly Pro Val Met Gln Lys
                   275                 280                 285
   aag acc atg ggc tgg gag gcc tcc tcc gag cgg atg tac ccc gag gac      912
   Lys Thr Met Gly Trp Glu Ala Ser Ser Glu Arg Met Tyr Pro Glu Asp
               290                 295                 300
   ggc gcc ctg aag ggc gag atc aag cag agg ctg aag ctg aag gac ggc      960
   Gly Ala Leu Lys Gly Glu Ile Lys Gln Arg Leu Lys Leu Lys Asp Gly
           305                 310                 315
   ggc cac tac gac gct gag gtc aag acc acc tac aag gcc aag aag ccc     1008
   Gly His Tyr Asp Ala Glu Val Lys Thr Thr Tyr Lys Ala Lys Lys Pro
       320                 325                 330
   gtg cag ctg ccc ggc gcc tac aac gtc aac atc aag ttg gac atc acc     1056
   Val Gln Leu Pro Gly Ala Tyr Asn Val Asn Ile Lys Leu Asp Ile Thr
   335                 340                 345                 350
   tcc cac aac gag gac tac acc atc gtg gaa cag tac gaa cgc gcc gag     1104
   Ser His Asn Glu Asp Tyr Thr Ile Val Glu Gln Tyr Glu Arg Ala Glu
                   355                 360                 365
   ggc cgc cac tcc acc ggc ggc atg gac gag ctg tac aag gaa ttc atg     1152
   Gly Arg His Ser Thr Gly Gly Met Asp Glu Leu Tyr Lys Glu Phe Met
               370                 375                 380
   gtt tcc atc cca gag tac tat gag gga aag aac atc ttg cta act ggt     1200
   Val Ser Ile Pro Glu Tyr Tyr Glu Gly Lys Asn Ile Leu Leu Thr Gly
           385                 390                 395
   gca act gga ttt ctt ggg aag gtt ttg ctt gag aag ctg cta aga tct     1248
   Ala Thr Gly Phe Leu Gly Lys Val Leu Leu Glu Lys Leu Leu Arg Ser
       400                 405                 410
   tgc cct aga gtc aac tcc gtt tac gtt cta gtc aga caa aag gct gga     1296
   Cys Pro Arg Val Asn Ser Val Tyr Val Leu Val Arg Gln Lys Ala Gly
   415                 420                 425                 430
   caa aca cct caa gaa aga gtt gag gag atc ttg tca agc aag ctc ttt     1344
   Gln Thr Pro Gln Glu Arg Val Glu Glu Ile Leu Ser Ser Lys Leu Phe
                   435                 440                 445
   gat aga ctg cgt gat gag aat cca gat ttc cga gag aag atc atc gcc     1392
   Asp Arg Leu Arg Asp Glu Asn Pro Asp Phe Arg Glu Lys Ile Ile Ala
               450                 455                 460
   atc aac tct gaa ttg acc caa cct aag tta gcg ctt tct gaa gag gat     1440
   Ile Asn Ser Glu Leu Thr Gln Pro Lys Leu Ala Leu Ser Glu Glu Asp
           465                 470                 475
   aag gag atc atc atc gac tca acg aac gtt atc ttc cat tgc gct gca     1488
   Lys Glu Ile Ile Ile Asp Ser Thr Asn Val Ile Phe His Cys Ala Ala
       480                 485                 490
   aca gtt cgt ttc aat gag aac cta cgg gat gct gtt caa ttg aac gtc     1536
   Thr Val Arg Phe Asn Glu Asn Leu Arg Asp Ala Val Gln Leu Asn Val
   495                 500                 505                 510
   att gct acg aga caa ctg atc tta ctc gct cag cag atg aag aac ctc     1584
   Ile Ala Thr Arg Gln Leu Ile Leu Leu Ala Gln Gln Met Lys Asn Leu
                   515                 520                 525
   gaa gtc ttc atg cac gtt agt act gct tac gca tac tgt aac cgc aag     1632
   Glu Val Phe Met His Val Ser Thr Ala Tyr Ala Tyr Cys Asn Arg Lys
               530                 535                 540
   cac atc gat gaa gtt gtt tac cca cct cca gtt gat cct aag aag ttg      1680
   His Ile Asp Glu Val Val Tyr Pro Pro Pro Val Asp Pro Lys Lys Leu
           545                 550                 555
   atc gac tct ctt gag tgg atg gat gat gga ctt gtg aac gac ata aca      1728
   Ile Asp Ser Leu Glu Trp Met Asp Asp Gly Leu Val Asn Asp Ile Thr
       560                 565                 570
   cca aag ctt atc gga gac aga cca aac act tac atc tac act aag gca      1776
   Pro Lys Leu Ile Gly Asp Arg Pro Asn Thr Tyr Ile Tyr Thr Lys Ala
   575                 580                 585                 590
   ctg gct gag tat gtt gtt caa caa gag gga gct aag ttg aac gtt gca      1824
   Leu Ala Glu Tyr Val Val Gln Gln Glu Gly Ala Lys Leu Asn Val Ala
                   595                 600                 605
   atc gtt aga ccg tct att gtt gga gct tca tgg aaa gaa cca ttc cca      1872
   Ile Val Arg Pro Ser Ile Val Gly Ala Ser Trp Lys Glu Pro Phe Pro
               610                 615                 620
   gga tgg att gac aac ttc aat ggt cca tct gga ctt ttc att gca gct      1920
   Gly Trp Ile Asp Asn Phe Asn Gly Pro Ser Gly Leu Phe Ile Ala Ala
           625                 630                 635
   ggc aag ggt atc ctc aga act atg aga gca agt aac aac gca ctt gca      1968
   Gly Lys Gly Ile Leu Arg Thr Met Arg Ala Ser Asn Asn Ala Leu Ala
       640                 645                 650
   gat ctt gtt cct gtt gac gtt gtc gtt aac acc tca tta gca gct gca      2016
   Asp Leu Val Pro Val Asp Val Val Val Asn Thr Ser Leu Ala Ala Ala
   655                 660                 665                 670
   tgg tat tct gga gtt aac aga ccc agg aac atc atg gtg tac aac tgt      2064
   Trp Tyr Ser Gly Val Asn Arg Pro Arg Asn Ile Met Val Tyr Asn Cys
                   675                 680                 685
   aca acg gga tct aca aac cct ttt cat tgg ggt gaa gtt gag tat cac      2112
   Thr Thr Gly Ser Thr Asn Pro Phe His Trp Gly Glu Val Glu Tyr His
               690                 695                 700
   gtc atc tct acc ttc aag aga aac cct ctt gag caa gct ttc aga aga      2160
   Val Ile Ser Thr Phe Lys Arg Asn Pro Leu Glu Gln Ala Phe Arg Arg
           705                 710                 715
   cct aac gtg aac ctt acc tcc aat cat cta ctc tac cac tac tgg att      2208
   Pro Asn Val Asn Leu Thr Ser Asn His Leu Leu Tyr His Tyr Trp Ile
       720                 725                 730
   gct gtt tct cat aag gct cct gct ttc ttg tac gac atc tac ctt cga      2256
   Ala Val Ser His Lys Ala Pro Ala Phe Leu Tyr Asp Ile Tyr Leu Arg
   735                 740                 745                 750
   atg act ggt aga agt cct cgg atg atg aag acc att aca cga cta cac      2304
   Met Thr Gly Arg Ser Pro Arg Met Met Lys Thr Ile Thr Arg Leu His
                   755                 760                 765
   aag gct atg gtc ttc ctt gag tac ttc acc tca aac tca tgg gtt tgg      2352
   Lys Ala Met Val Phe Leu Glu Tyr Phe Thr Ser Asn Ser Trp Val Trp
               770                 775                 780
   aac act gac aac gtt aac atg ctc atg aac cag ctt aac cct gag gac      2400
   Asn Thr Asp Asn Val Asn Met Leu Met Asn Gln Leu Asn Pro Glu Asp
           785                 790                 795
   aag aag acg ttc aac att gat gtt cga cag ctt cac tgg gct gag tac      2448
   Lys Lys Thr Phe Asn Ile Asp Val Arg Gln Leu His Trp Ala Glu Tyr
       800                 805                 810
   ata gag aac tac tgt atg ggg acg aag aag tac gtt ctt aac gaa gag      2496
   Ile Glu Asn Tyr Cys Met Gly Thr Lys Lys Tyr Val Leu Asn Glu Glu
   815                 820                 825                 830
   atg tct gga ctt cca gca gct aga aaa cat ctg aac aag ctt cgc aac        2544
   Met Ser Gly Leu Pro Ala Ala Arg Lys His Leu Asn Lys Leu Arg Asn
                   835                 840                 845
   atc aga taa ctcgag                                                     2559
   Ile Arg
<210> 10
   <211> 848
   <212> PRT
   <213> Artificial
<220>
   <223> Synthetic Construct
<400> 10
   Met Ala Asp Gln Thr Arg Thr His His Glu Met Ile Ser Arg Asp Ser
   1               5                   10                  15
   Thr Gln Glu Ala His Pro Lys Ala Arg Gln Met Val Lys Ala Ala Thr
               20                  25                  30
   Ala Val Thr Ala Gly Gly Ser Leu Leu Val Leu Ser Gly Leu Thr Leu
           35                  40                  45
   Ala Gly Thr Val Ile Ala Leu Thr Val Ala Thr Pro Leu Leu Val Ile
       50                  55                  60
   Phe Ser Pro Val Leu Val Pro Ala Val Val Thr Val Ala Leu Ile Ile
   65                  70                  75                  80
   Thr Gly Phe Leu Ala Ser Gly Gly Phe Gly Ile Ala Ala Ile Thr Ala
                   85                  90                  95
   Phe Ser Trp Leu Tyr Arg His Met Thr Gly Ser Gly Ser Asp Lys Ile
               100                 105                 110
   Glu Asn Ala Arg Met Lys Val Gly Ser Arg Val Gln Asp Thr Lys Tyr
           115                 120                 125
   Gly Gln His Asn Ile Gly Val Gln His Gln Gln Val Ser Gly Ser Met
       130                 135                 140
   Val Ser Lys Gly Glu Glu Asp Asn Met Ala Ile Ile Lys Glu Phe Met
   145                 150                 155                 160
   Arg Phe Lys Val His Met Glu Gly Ser Val Asn Gly His Glu Phe Glu
                   165                 170                 175
   Ile Glu Gly Glu Gly Glu Gly Arg Pro Tyr Glu Gly Thr Gln Thr Ala
               180                 185                 190
   Lys Leu Lys Val Thr Lys Gly Gly Pro Leu Pro Phe Ala Trp Asp Ile
   195 200 205
   Leu Ser Pro Gln Phe Met Tyr Gly Ser Lys Ala Tyr Val Lys His Pro
   210 215 220
   Ala Asp Ile Pro Asp Tyr Leu Lys Leu Ser Phe Pro Glu Gly Phe Lys
   225 230 235 240
   Trp Glu Arg Val Met Asn Phe Glu Asp Gly Gly Val Val Thr Val Thr
   245 250 255
   Gln Asp Ser Ser Leu Gln Asp Gly Glu Phe Ile Tyr Lys Val Lys Leu
   260 265 270
   Arg Gly Thr Asn Phe Pro Ser Asp Gly Pro Val Met Gln Lys Lys Thr
   275 280 285
   Met Gly Trp Glu Ala Ser Ser Glu Arg Met Tyr Pro Glu Asp Gly Ala
   290 295 300
   Leu Lys Gly Glu Ile Lys Gln Arg Leu Lys Leu Lys Asp Gly Gly His
   305 310 315 320
   Tyr Asp Ala Glu Val Lys Thr Thr Tyr Lys Ala Lys Lys Pro Val Gln
   325 330 335
   Leu Pro Gly Ala Tyr Asn Val Asn Ile Lys Leu Asp Ile Thr Ser His
   340 345 350
   Asn Glu Asp Tyr Thr Ile Val Glu Gln Tyr Glu Arg Ala Glu Gly Arg
   355 360 365
   His Ser Thr Gly Gly Met Asp Glu Leu Tyr Lys Glu Phe Met Val Ser
   370 375 380
   Ile Pro Glu Tyr Tyr Glu Gly Lys Asn Ile Leu Leu Thr Gly Ala Thr
   385 390 395 400
   Gly Phe Leu Gly Lys Val Leu Leu Glu Lys Leu Leu Arg Ser Cys Pro
   405 410 415
   Arg Val Asn Ser Val Tyr Val Leu Val Arg Gln Lys Ala Gly Gln Thr
   420 425 430
   Pro Gln Glu Arg Val Glu Glu Ile Leu Ser Ser Lys Leu Phe Asp Arg
   435 440 445
   Leu Arg Asp Glu Asn Pro Asp Phe Arg Glu Lys Ile Ile Ala Ile Asn
   450 455 460
   Ser Glu Leu Thr Gln Pro Lys Leu Ala Leu Ser Glu Glu Asp Lys Glu
   465 470 475 480
   Ile Ile Ile Asp Ser Thr Asn Val Ile Phe His Cys Ala Ala Thr Val
   485 490 495
   Arg Phe Asn Glu Asn Leu Arg Asp Ala Val Gln Leu Asn Val Ile Ala
   500 505 510
   Thr Arg Gln Leu Ile Leu Leu Ala Gln Gln Met Lys Asn Leu Glu Val
   515 520 525
   Phe Met His Val Ser Thr Ala Tyr Ala Tyr Cys Asn Arg Lys His Ile
   530 535 540
   Asp Glu Val Val Tyr Pro Pro Pro Val Asp Pro Lys Lys Leu Ile Asp
   545 550 555 560
   Ser Leu Glu Trp Met Asp Asp Gly Leu Val Asn Asp Ile Thr Pro Lys
   565 570 575
   Leu Ile Gly Asp Arg Pro Asn Thr Tyr Ile Tyr Thr Lys Ala Leu Ala
   580 585 590
   Glu Tyr Val Val Gln Gln Glu Gly Ala Lys Leu Asn Val Ala Ile Val
   595 600 605
   Arg Pro Ser Ile Val Gly Ala Ser Trp Lys Glu Pro Phe Pro Gly Trp
   610 615 620
   Ile Asp Asn Phe Asn Gly Pro Ser Gly Leu Phe Ile Ala Ala Gly Lys
   625 630 635 640
   Gly Ile Leu Arg Thr Met Arg Ala Ser Asn Asn Ala Leu Ala Asp Leu
   645 650 655
   Val Pro Val Asp Val Val Val Asn Thr Ser Leu Ala Ala Ala Trp Tyr
   660 665 670
   Ser Gly Val Asn Arg Pro Arg Asn Ile Met Val Tyr Asn Cys Thr Thr
   675 680 685
   Gly Ser Thr Asn Pro Phe His Trp Gly Glu Val Glu Tyr His Val Ile
   690 695 700
   Ser Thr Phe Lys Arg Asn Pro Leu Glu Gln Ala Phe Arg Arg Pro Asn
   705 710 715 720
   Val Asn Leu Thr Ser Asn His Leu Leu Tyr His Tyr Trp Ile Ala Val
   725 730 735
   Ser His Lys Ala Pro Ala Phe Leu Tyr Asp Ile Tyr Leu Arg Met Thr
                   740                 745                 750
   Gly Arg Ser Pro Arg Met Met Lys Thr Ile Thr Arg Leu His Lys Ala
           755                 760                 765
   Met Val Phe Leu Glu Tyr Phe Thr Ser Asn Ser Trp Val Trp Asn Thr
       770                 775                 780
   Asp Asn Val Asn Met Leu Met Asn Gln Leu Asn Pro Glu Asp Lys Lys
   785                 790                 795                 800
   Thr Phe Asn Ile Asp Val Arg Gln Leu His Trp Ala Glu Tyr Ile Glu
                   805                 810                 815
   Asn Tyr Cys Met Gly Thr Lys Lys Tyr Val Leu Asn Glu Glu Met Ser
               820                 825                 830
   Gly Leu Pro Ala Ala Arg Lys His Leu Asn Lys Leu Arg Asn Ile Arg
           835                 840                 845
<210> 11
   <211> 1002
   <212> DNA
   <213> Artificial
<220>
   <223> wax ester synthase from mouse
<220>
   <221> CDS
   <222> (1)..(1002)
<400> 11
   atg ttc tgg cct act aag aag gac ctt aag act gca atg gag gtt ttc        48
   Met Phe Trp Pro Thr Lys Lys Asp Leu Lys Thr Ala Met Glu Val Phe
   1               5                   10                  15
   gct ctt ttc caa tgg gct ttg tct gca ctc gtt atc gtt act acc gtc        96
   Ala Leu Phe Gln Trp Ala Leu Ser Ala Leu Val Ile Val Thr Thr Val
               20                  25                  30
   atc atc gtg aac ctg tac ctt gtt gtg ttc act tca tac tgg cca gtt       144
   Ile Ile Val Asn Leu Tyr Leu Val Val Phe Thr Ser Tyr Trp Pro Val
           35                  40                  45
   acc gtt ttg atg cta aca tgg ctg gca ttc gat tgg aaa aca cct gaa       192
   Thr Val Leu Met Leu Thr Trp Leu Ala Phe Asp Trp Lys Thr Pro Glu
       50                  55                  60
   aga gga ggt aga agg ttc aca tgt gtg aga aag tgg cgt ctt tgg aaa       240
   Arg Gly Gly Arg Arg Phe Thr Cys Val Arg Lys Trp Arg Leu Trp Lys
   65                  70                  75                  80
   cac tac tct gac tac ttc cct ctt aag atg gtg aag acg aag gat atc       288
   His Tyr Ser Asp Tyr Phe Pro Leu Lys Met Val Lys Thr Lys Asp Ile
                   85                  90                  95
   tcc cct gat aga aac tac ata ctt gtc tgt cac cca cat ggt ctt atg       336
   Ser Pro Asp Arg Asn Tyr Ile Leu Val Cys His Pro His Gly Leu Met
               100                 105                 110
   gca cat agt tgt ttc gga cat ttc gct acc gat act aca gga ttc agt      384
   Ala His Ser Cys Phe Gly His Phe Ala Thr Asp Thr Thr Gly Phe Ser
           115                 120                 125
   aag acc ttc cct ggt atc aca cct tac atg ctt act ctt gga gct ttc      432
   Lys Thr Phe Pro Gly Ile Thr Pro Tyr Met Leu Thr Leu Gly Ala Phe
       130                 135                 140
   ttc tgg gtt cct ttc ttg aga gat tac gtc atg tca acc gga agt tgt      480
   Phe Trp Val Pro Phe Leu Arg Asp Tyr Val Met Ser Thr Gly Ser Cys
   145                 150                 155                 160
   tct gtg tcc aga agc tct atg gac ttc ttg ctt acg caa aag ggt aca      528
   Ser Val Ser Arg Ser Ser Met Asp Phe Leu Leu Thr Gln Lys Gly Thr
                   165                 170                 175
   gga aac atg ctt gtt gtc gtt gtt ggt gga ctt gct gaa tgt aga tac      576
   Gly Asn Met Leu Val Val Val Val Gly Gly Leu Ala Glu Cys Arg Tyr
               180                 185                 190
   tct aca cca ggc tca aca acc ctt ttc cta aag aag agg cag gga ttt      624
   Ser Thr Pro Gly Ser Thr Thr Leu Phe Leu Lys Lys Arg Gln Gly Phe
           195                 200                 205
   gtt cgt aca gca cta aag cat ggg gtt tct ttg atc cct gct tat gca      672
   Val Arg Thr Ala Leu Lys His Gly Val Ser Leu Ile Pro Ala Tyr Ala
       210                 215                 220
   ttt gga gag act gac ctc tac gat caa cat atc ttc act cct gga gga      720
   Phe Gly Glu Thr Asp Leu Tyr Asp Gln His Ile Phe Thr Pro Gly Gly
   225                 230                 235                 240
   ttc gtc aac aga ttc caa aag tgg ttc cag aag atg gtt cac atc tac      768
   Phe Val Asn Arg Phe Gln Lys Trp Phe Gln Lys Met Val His Ile Tyr
                   245                 250                 255
   cca tgt gct ttc tat gga aga ggt ctg acc aag aat tca tgg ggt ctt      816
   Pro Cys Ala Phe Tyr Gly Arg Gly Leu Thr Lys Asn Ser Trp Gly Leu
               260                 265                 270
   ttg cct tac tca caa cca gtt act aca gtt gtt gga gag cca tta ccg      864
   Leu Pro Tyr Ser Gln Pro Val Thr Thr Val Val Gly Glu Pro Leu Pro
           275                 280                 285
   ttg cct aag ata gaa aac cct agc gaa gag att gtt gcc aag tat cac      912
   Leu Pro Lys Ile Glu Asn Pro Ser Glu Glu Ile Val Ala Lys Tyr His
       290                 295                 300
   acc ctt tac atc gat gct ctc aga aag cta ttc gac cag cac aag act      960
   Thr Leu Tyr Ile Asp Ala Leu Arg Lys Leu Phe Asp Gln His Lys Thr
   305                 310                 315                 320
   aag ttc gga atc tct gag aca caa gag ctg gtt atc gtc taa             1002
   Lys Phe Gly Ile Ser Glu Thr Gln Glu Leu Val Ile Val
                   325                 330
<210> 12
   <211> 333
   <212> PRT
   <213> Artificial
<220>
   <223> synthetic Construct
<400> 12
   Met Phe Trp Pro Thr Lys Lys Asp Leu Lys Thr Ala Met Glu Val Phe
   1               5                   10                  15
   Ala Leu Phe Gln Trp Ala Leu Ser Ala Leu Val Ile Val Thr Thr Val
               20                  25                  30
   Ile Ile Val Asn Leu Tyr Leu Val Val Phe Thr Ser Tyr Trp Pro Val
           35                  40                  45
   Thr Val Leu Met Leu Thr Trp Leu Ala Phe Asp Trp Lys Thr Pro Glu
       50                  55                  60
   Arg Gly Gly Arg Arg Phe Thr Cys Val Arg Lys Trp Arg Leu Trp Lys
   65                  70                  75                  80
   His Tyr Ser Asp Tyr Phe Pro Leu Lys Met Val Lys Thr Lys Asp Ile
                   85                  90                  95
   Ser Pro Asp Arg Asn Tyr Ile Leu Val Cys His Pro His Gly Leu Met
               100                 105                 110
   Ala His Ser Cys Phe Gly His Phe Ala Thr Asp Thr Thr Gly Phe Ser
           115                 120                 125
   Lys Thr Phe Pro Gly Ile Thr Pro Tyr Met Leu Thr Leu Gly Ala Phe
       130                 135                 140
   Phe Trp Val Pro Phe Leu Arg Asp Tyr Val Met Ser Thr Gly Ser Cys
   145                 150                 155                 160
   Ser Val Ser Arg Ser Ser Met Asp Phe Leu Leu Thr Gln Lys Gly Thr
                   165                 170                 175
   Gly Asn Met Leu Val Val Val Val Gly Gly Leu Ala Glu Cys Arg Tyr
               180                 185                 190
   Ser Thr Pro Gly Ser Thr Thr Leu Phe Leu Lys Lys Arg Gln Gly Phe
           195                 200                 205
   Val Arg Thr Ala Leu Lys His Gly Val Ser Leu Ile Pro Ala Tyr Ala
       210                 215                 220
   Phe Gly Glu Thr Asp Leu Tyr Asp Gln His Ile Phe Thr Pro Gly Gly
   225                 230                 235                 240
   Phe Val Asn Arg Phe Gln Lys Trp Phe Gln Lys Met Val His Ile Tyr
                   245                 250                 255
   Pro Cys Ala Phe Tyr Gly Arg Gly Leu Thr Lys Asn Ser Trp Gly Leu
               260                 265                 270
   Leu Pro Tyr Ser Gln Pro Val Thr Thr Val Val Gly Glu Pro Leu Pro
           275                 280                 285
   Leu Pro Lys Ile Glu Asn Pro Ser Glu Glu Ile Val Ala Lys Tyr His
       290                 295                 300
   Thr Leu Tyr Ile Asp Ala Leu Arg Lys Leu Phe Asp Gln His Lys Thr
   305                 310                 315                 320
   Lys Phe Gly Ile Ser Glu Thr Gln Glu Leu Val Ile Val
                   325                 330
<210> 13
   <211> 1731
   <212> DNA
   <213> Artificial
<220>
   <223> Wax ester synthase from mouse tagged with YFP
<220>
   <221> CDS
   <222> (7)..(1725)
<400> 13
   actagt atg gtg agc aag ggc gag gag ctg ttc acc ggg gtg gtg ccc       48
          Met Val Ser Lys Gly Glu Glu Leu Phe Thr Gly Val Val Pro
          1               5                   10
   atc ctg gtc gag ctg gac ggc gac gta aac ggc cac aag ttc agc gtg      96
   Ile Leu Val Glu Leu Asp Gly Asp Val Asn Gly His Lys Phe Ser Val
   15                  20                  25                  30
   tcc ggc gag ggc gag ggc gat gcc acc tac ggc aag ctg acc ctg aag     144
   Ser Gly Glu Gly Glu Gly Asp Ala Thr Tyr Gly Lys Leu Thr Leu Lys
                   35                  40                  45
   ttc atc tgc acc acc ggc aag ctg ccc gtg ccc tgg ccc acc ctc gtg     192
   Phe Ile Cys Thr Thr Gly Lys Leu Pro Val Pro Trp Pro Thr Leu Val
               50                  55                  60
   acc acc ttc ggc tac ggc ctg cag tgc ttc gcc cgc tac ccc gac cac     240
   Thr Thr Phe Gly Tyr Gly Leu Gln Cys Phe Ala Arg Tyr Pro Asp His
           65                  70                  75
   atg aag cag cac gac ttc ttc aag tcc gcc atg ccc gaa ggc tac gtc     288
   Met Lys Gln His Asp Phe Phe Lys Ser Ala Met Pro Glu Gly Tyr Val
       80                  85                  90
   cag gag cgc acc atc ttc ttc aag gac gac ggc aac tac aag acc cgc     336
   Gln Glu Arg Thr Ile Phe Phe Lys Asp Asp Gly Asn Tyr Lys Thr Arg
   95                  100                 105                 110
   gcc gag gtg aag ttc gag ggc gac acc ctg gtg aac cgc atc gag ctg     384
   Ala Glu Val Lys Phe Glu Gly Asp Thr Leu Val Asn Arg Ile Glu Leu
                   115                 120                 125
   aag ggc atc gac ttc aag gag gac ggc aac atc ctg ggg cac aag ctg     432
   Lys Gly Ile Asp Phe Lys Glu Asp Gly Asn Ile Leu Gly His Lys Leu
               130                 135                 140
   gag tac aac tac aac agc cac aac gtc tat atc atg gcc gac aag cag     480
   Glu Tyr Asn Tyr Asn Ser His Asn Val Tyr Ile Met Ala Asp Lys Gln
           145                 150                 155
   aag aac ggc atc aag gtg aac ttc aag atc cgc cac aac atc gag gac     528
   Lys Asn Gly Ile Lys Val Asn Phe Lys Ile Arg His Asn Ile Glu Asp
           160                 165                 170
   ggc agc gtg cag ctc gcc gac cac tac cag cag aac acc ccc atc ggc       576
   Gly Ser Val Gln Leu Ala Asp His Tyr Gln Gln Asn Thr Pro Ile Gly
   175                 180                 185                 190
   gac ggc ccc gtg ctg ctg ccc gac aac cac tac ctg agc tac cag tcc       624
   Asp Gly Pro Val Leu Leu Pro Asp Asn His Tyr Leu Ser Tyr Gln Ser
                   195                 200                 205
   gcc ctg agc aaa gac ccc aac gag aag cgc gat cac atg gtc ctg ctg       672
   Ala Leu Ser Lys Asp Pro Asn Glu Lys Arg Asp His Met Val Leu Leu
               210                 215                 220
   gag ttc gtg acc gcc gcc ggg atc act ctc ggc atg gac gag ctg tac       720
   Glu Phe Val Thr Ala Ala Gly Ile Thr Leu Gly Met Asp Glu Leu Tyr
           225                 230                 235
   aag atg ttc tgg cct act aag aag gac ctt aag act gca atg gag gtt       768
   Lys Met Phe Trp Pro Thr Lys Lys Asp Leu Lys Thr Ala Met Glu Val
       240                 245                 250
   ttc gct ctt ttc caa tgg gct ttg tct gca ctc gtt atc gtt act acc       816
   Phe Ala Leu Phe Gln Trp Ala Leu Ser Ala Leu Val Ile Val Thr Thr
   255                 260                 265                 270
   gtc atc atc gtg aac ctg tac ctt gtt gtg ttc act tca tac tgg cca       864
   Val Ile Ile Val Asn Leu Tyr Leu Val Val Phe Thr Ser Tyr Trp Pro
                   275                 280                 285
   gtt acc gtt ttg atg cta aca tgg ctg gca ttc gat tgg aaa aca cct       912
   Val Thr Val Leu Met Leu Thr Trp Leu Ala Phe Asp Trp Lys Thr Pro
               290                 295                 300
   gaa aga gga ggt aga agg ttc aca tgt gtg aga aag tgg cgt ctt tgg       960
   Glu Arg Gly Gly Arg Arg Phe Thr Cys Val Arg Lys Trp Arg Leu Trp
           305                 310                 315
   aaa cac tac tct gac tac ttc cct ctt aag atg gtg aag acg aag gat      1008
   Lys His Tyr Ser Asp Tyr Phe Pro Leu Lys Met Val Lys Thr Lys Asp
       320                 325                 330
   atc tcc cct gat aga aac tac ata ctt gtc tgt cac cca cat ggt ctt      1056
   Ile Ser Pro Asp Arg Asn Tyr Ile Leu Val Cys His Pro His Gly Leu
   335                 340                 345                 350
   atg gca cat agt tgt ttc gga cat ttc gct acc gat act aca gga ttc      1104
   Met Ala His Ser Cys Phe Gly His Phe Ala Thr Asp Thr Thr Gly Phe
                   355                 360                 365
   agt aag acc ttc cct ggt atc aca cct tac atg ctt act ctt gga gct      1152
   Ser Lys Thr Phe Pro Gly Ile Thr Pro Tyr Met Leu Thr Leu Gly Ala
               370                 375                 380
   ttc ttc tgg gtt cct ttc ttg aga gat tac gtc atg tca acc gga agt      1200
   Phe Phe Trp Val Pro Phe Leu Arg Asp Tyr Val Met Ser Thr Gly Ser
           385                 390                 395
   tgt tct gtg tcc aga agc tct atg gac ttc ttg ctt acg caa aag ggt      1248
   Cys Ser Val Ser Arg Ser Ser Met Asp Phe Leu Leu Thr Gln Lys Gly
       400                 405                 410
   aca gga aac atg ctt gtt gtc gtt gtt ggt gga ctt gct gaa tgt aga      1296
   Thr Gly Asn Met Leu Val Val Val Val Gly Gly Leu Ala Glu Cys Arg
   415                 420                 425                 430
   tac tct aca cca ggc tca aca acc ctt ttc cta aag aag agg cag gga      1344
   Tyr Ser Thr Pro Gly Ser Thr Thr Leu Phe Leu Lys Lys Arg Gln Gly
                   435                 440                 445
   ttt gtt cgt aca gca cta aag cat ggg gtt tct ttg atc cct gct tat      1392
   Phe Val Arg Thr Ala Leu Lys His Gly Val Ser Leu Ile Pro Ala Tyr
               450                 455                 460
   gca ttt gga gag act gac ctc tac gat caa cat atc ttc act cct gga      1440
   Ala Phe Gly Glu Thr Asp Leu Tyr Asp Gln His Ile Phe Thr Pro Gly
           465                 470                 475
   gga ttc gtc aac aga ttc caa aag tgg ttc cag aag atg gtt cac atc      1488
   Gly Phe Val Asn Arg Phe Gln Lys Trp Phe Gln Lys Met Val His Ile
       480                 485                 490
   tac cca tgt gct ttc tat gga aga ggt ctg acc aag aat tca tgg ggt      1536
   Tyr Pro Cys Ala Phe Tyr Gly Arg Gly Leu Thr Lys Asn Ser Trp Gly
   495                 500                 505                 510
   ctt ttg cct tac tca caa cca gtt act aca gtt gtt gga gag cca tta      1584
   Leu Leu Pro Tyr Ser Gln Pro Val Thr Thr Val Val Gly Glu Pro Leu
                   515                 520                 525
   ccg ttg cct aag ata gaa aac cct agc gaa gag att gtt gcc aag tat      1632
   Pro Leu Pro Lys Ile Glu Asn Pro Ser Glu Glu Ile Val Ala Lys Tyr
               530                 535                 540
   cac acc ctt tac atc gat gct ctc aga aag cta ttc gac cag cac aag      1680
   His Thr Leu Tyr Ile Asp Ala Leu Arg Lys Leu Phe Asp Gln His Lys
           545                 550                 555
   act aag ttc gga atc tct gag aca caa gag ctg gtt atc gtc taa          1725
   Thr Lys Phe Gly Ile Ser Glu Thr Gln Glu Leu Val Ile Val
       560                 565                 570
   gagctc                                                               1731
<210> 14
   <211> 572
   <212> PRT
   <213> Artificial
<220>
   <223> Synthetic construct
<400> 14
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Phe Gly Tyr Gly Leu Gln Cys Phe Ala Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
   100 105 110
   Val Lys Phe Glu Gly Asp Thr Leu Val Asn Arg Ile Glu Leu Lys Gly
   115 120 125
   Ile Asp Phe Lys Glu Asp Gly Asn Ile Leu Gly His Lys Leu Glu Tyr
   130 135 140
   Asn Tyr Asn Ser His Asn Val Tyr Ile Met Ala Asp Lys Gln Lys Asn
   145 150 155 160
   Gly Ile Lys Val Asn Phe Lys Ile Arg His Asn Ile Glu Asp Gly Ser
   165 170 175
   Val Gln Leu Ala Asp His Tyr Gln Gln Asn Thr Pro Ile Gly Asp Gly
   180 185 190
   Pro Val Leu Leu Pro Asp Asn His Tyr Leu Ser Tyr Gln Ser Ala Leu
   195 200 205
   Ser Lys Asp Pro Asn Glu Lys Arg Asp His Met Val Leu Leu Glu Phe
   210 215 220
   Val Thr Ala Ala Gly Ile Thr Leu Gly Met Asp Glu Leu Tyr Lys Met
   225 230 235 240
   Phe Trp Pro Thr Lys Lys Asp Leu Lys Thr Ala Met Glu Val Phe Ala
   245 250 255
   Leu Phe Gln Trp Ala Leu Ser Ala Leu Val Ile Val Thr Thr Val Ile
   260 265 270
   Ile Val Asn Leu Tyr Leu Val Val Phe Thr Ser Tyr Trp Pro Val Thr
   275 280 285
   Val Leu Met Leu Thr Trp Leu Ala Phe Asp Trp Lys Thr Pro Glu Arg
   290 295 300
   Gly Gly Arg Arg Phe Thr Cys Val Arg Lys Trp Arg Leu Trp Lys His
   305 310 315 320
   Tyr Ser Asp Tyr Phe Pro Leu Lys Met Val Lys Thr Lys Asp Ile Ser
   325 330 335
   Pro Asp Arg Asn Tyr Ile Leu Val Cys His Pro His Gly Leu Met Ala
   340 345 350
   His Ser Cys Phe Gly His Phe Ala Thr Asp Thr Thr Gly Phe Ser Lys
   355 360 365
   Thr Phe Pro Gly Ile Thr Pro Tyr Met Leu Thr Leu Gly Ala Phe Phe
       370                 375                 380
   Trp Val Pro Phe Leu Arg Asp Tyr Val Met Ser Thr Gly Ser Cys Ser
   385                 390                 395                 400
   Val Ser Arg Ser Ser Met Asp Phe Leu Leu Thr Gln Lys Gly Thr Gly
                   405                 410                 415
   Asn Met Leu Val Val Val Val Gly Gly Leu Ala Glu Cys Arg Tyr Ser
               420                 425                 430
   Thr Pro Gly Ser Thr Thr Leu Phe Leu Lys Lys Arg Gln Gly Phe Val
           435                 440                 445
   Arg Thr Ala Leu Lys His Gly Val Ser Leu Ile Pro Ala Tyr Ala Phe
       450                 455                 460
   Gly Glu Thr Asp Leu Tyr Asp Gln His Ile Phe Thr Pro Gly Gly Phe
   465                 470                 475                 480
   Val Asn Arg Phe Gln Lys Trp Phe Gln Lys Met Val His Ile Tyr Pro
                   485                 490                 495
   Cys Ala Phe Tyr Gly Arg Gly Leu Thr Lys Asn Ser Trp Gly Leu Leu
               500                 505                 510
   Pro Tyr Ser Gln Pro Val Thr Thr Val Val Gly Glu Pro Leu Pro Leu
           515                 520                 525
   Pro Lys Ile Glu Asn Pro Ser Glu Glu Ile Val Ala Lys Tyr His Thr
       530                 535                 540
   Leu Tyr Ile Asp Ala Leu Arg Lys Leu Phe Asp Gln His Lys Thr Lys
   545                 550                 555                 560
   Phe Gly Ile Ser Glu Thr Gln Glu Leu Val Ile Val
                   565                 570
<210> 15
   <211> 2154
   <212> DNA
   <213> Artificial
<220>
   <223> Wax ester synthase from mouse tagged with YFP fused to oleosin 3 At
<220>
   <221> CDS
   <222> (7)..(2148)
<400> 15
   actagt atg gcg gac caa aca aga acc cat cac gag atg ata agc cga       48
          Met Ala Asp Gln Thr Arg Thr His His Glu Met Ile Ser Arg
                         1               5                  10
   gac agt acc caa gag gcc cat ccg aag gcc agg cag atg gtg aag gca      96
   Asp Ser Thr Gln Glu Ala His Pro Lys Ala Arg Gln Met Val Lys Ala
   15                  20                      25                  30
   gca acc gct gtc aca gcc ggt gga tcc cta ctt gtc ctc tcc ggc tta     144
   Ala Thr Ala Val Thr Ala Gly Gly Ser Leu Leu Val Leu Ser Gly Leu
                   35                      40                  45
   aca ctc gct gga aca gtc atc gca ctc acg gtg gct act cct ctc ctc     192
   Thr Leu Ala Gly Thr Val Ile Ala Leu Thr Val Ala Thr Pro Leu Leu
               50                  55                  60
   gtc atc ttc agc ccc gtc ttg gtt cca gca gtg gta acc gtt gct ctc     240
   Val Ile Phe Ser Pro Val Leu Val Pro Ala Val Val Thr Val Ala Leu
           65                  70                  75
   atc att acc gga ttc ctt gca tcc ggt ggc ttt gga ata gcc gcc att     288
   Ile Ile Thr Gly Phe Leu Ala Ser Gly Gly Phe Gly Ile Ala Ala Ile
       80                  85                  90
   acc gcc ttc tct tgg ctc tac agg cac atg acg gga tct gga tcg gat     336
   Thr Ala Phe Ser Trp Leu Tyr Arg His Met Thr Gly Ser Gly Ser Asp
   95                  100                 105                 110
   aag ata gag aat gct cgg atg aag gtt gga agc aga gtg cag gat act     384
   Lys Ile Glu Asn Ala Arg Met Lys Val Gly Ser Arg Val Gln Asp Thr
                   115                 120                 125
   aag tat ggg cag cac aac att gga gtc caa cac caa caa gtt tct atg     432
   Lys Tyr Gly Gln His Asn Ile Gly Val Gln His Gln Gln Val Ser Met
               130                 135                 140
   gtg agc aag ggc gag gag ctg ttc acc ggg gtg gtg ccc atc ctg gtc     480
   Val Ser Lys Gly Glu Glu Leu Phe Thr Gly Val Val Pro Ile Leu Val
           145                 150                 155
   gag ctg gac ggc gac gta aac ggc cac aag ttc agc gtg tcc ggc gag     528
   Glu Leu Asp Gly Asp Val Asn Gly His Lys Phe Ser Val Ser Gly Glu
       160                 165                 170
   ggc gag ggc gat gcc acc tac ggc aag ctg acc ctg aag ttc atc tgc     576
   Gly Glu Gly Asp Ala Thr Tyr Gly Lys Leu Thr Leu Lys Phe Ile Cys
   175                 180                 185                 190
   acc acc ggc aag ctg ccc gtg ccc tgg ccc acc ctc gtg acc acc ttc     624
   Thr Thr Gly Lys Leu Pro Val Pro Trp Pro Thr Leu Val Thr Thr Phe
                   195                 200                 205
   ggc tac ggc ctg cag tgc ttc gcc cgc tac ccc gac cac atg aag cag     672
   Gly Tyr Gly Leu Gln Cys Phe Ala Arg Tyr Pro Asp His Met Lys Gln
               210                 215                 220
   cac gac ttc ttc aag tcc gcc atg ccc gaa ggc tac gtc cag gag cgc     720
   His Asp Phe Phe Lys Ser Ala Met Pro Glu Gly Tyr Val Gln Glu Arg
           225                 230                 235
   acc atc ttc ttc aag gac gac ggc aac tac aag acc cgc gcc gag gtg     768
   Thr Ile Phe Phe Lys Asp Asp Gly Asn Tyr Lys Thr Arg Ala Glu Val
       240                 245                 250
   aag ttc gag ggc gac acc ctg gtg aac cgc atc gag ctg aag ggc atc     816
   Lys Phe Glu Gly Asp Thr Leu Val Asn Arg Ile Glu Leu Lys Gly Ile
   255                 260                 265                 270
   gac ttc aag gag gac ggc aac atc ctg ggg cac aag ctg gag tac aac     864
   Asp Phe Lys Glu Asp Gly Asn Ile Leu Gly His Lys Leu Glu Tyr Asn
                   275                 280                 285
   tac aac agc cac aac gtc tat atc atg gcc gac aag cag aag aac ggc     912
   Tyr Asn Ser His Asn Val Tyr Ile Met Ala Asp Lys Gln Lys Asn Gly
               290                 295                 300
   atc aag gtg aac ttc aag atc cgc cac aac atc gag gac ggc agc gtg     960
   Ile Lys Val Asn Phe Lys Ile Arg His Asn Ile Glu Asp Gly Ser Val
           305                 310                 315
   cag ctc gcc gac cac tac cag cag aac acc ccc atc ggc gac ggc ccc    1008
   Gln Leu Ala Asp His Tyr Gln Gln Asn Thr Pro Ile Gly Asp Gly Pro
       320                 325                 330
   gtg ctg ctg ccc gac aac cac tac ctg agc tac cag tcc gcc ctg agc    1056
   Val Leu Leu Pro Asp Asn His Tyr Leu Ser Tyr Gln Ser Ala Leu Ser
   335                 340                 345                 350
   aaa gac ccc aac gag aag cgc gat cac atg gtc ctg ctg gag ttc gtg    1104
   Lys Asp Pro Asn Glu Lys Arg Asp His Met Val Leu Leu Glu Phe Val
                   355                 360                 365
   acc gcc gcc ggg atc act ctc ggc atg gac gag ctg tac aag atg ttc    1152
   Thr Ala Ala Gly Ile Thr Leu Gly Met Asp Glu Leu Tyr Lys Met Phe
               370                 375                 380
   tgg cct act aag aag gac ctt aag act gca atg gag gtt ttc gct ctt    1200
   Trp Pro Thr Lys Lys Asp Leu Lys Thr Ala Met Glu Val Phe Ala Leu
           385                 390                 395
   ttc caa tgg gct ttg tct gca ctc gtt atc gtt act acc gtc atc atc    1248
   Phe Gln Trp Ala Leu Ser Ala Leu Val Ile Val Thr Thr Val Ile Ile
       400                 405                 410
   gtg aac ctg tac ctt gtt gtg ttc act tca tac tgg cca gtt acc gtt    1296
   Val Asn Leu Tyr Leu Val Val Phe Thr Ser Tyr Trp Pro Val Thr Val
   415                 420                 425                 430
   ttg atg cta aca tgg ctg gca ttc gat tgg aaa aca cct gaa aga gga    1344
   Leu Met Leu Thr Trp Leu Ala Phe Asp Trp Lys Thr Pro Glu Arg Gly
                   435                 440                 445
   ggt aga agg ttc aca tgt gtg aga aag tgg cgt ctt tgg aaa cac tac    1392
   Gly Arg Arg Phe Thr Cys Val Arg Lys Trp Arg Leu Trp Lys His Tyr
               450                 455                 460
   tct gac tac ttc cct ctt aag atg gtg aag acg aag gat atc tcc cct    1440
   Ser Asp Tyr Phe Pro Leu Lys Met Val Lys Thr Lys Asp Ile Ser Pro
           465                 470                 475
   gat aga aac tac ata ctt gtc tgt cac cca cat ggt ctt atg gca cat    1488
   Asp Arg Asn Tyr Ile Leu Val Cys His Pro His Gly Leu Met Ala His
       480                 485                 490
   agt tgt ttc gga cat ttc gct acc gat act aca gga ttc agt aag acc    1536
   Ser Cys Phe Gly His Phe Ala Thr Asp Thr Thr Gly Phe Ser Lys Thr
   495                 500                 505                 510
   ttc cct ggt atc aca cct tac atg ctt act ctt gga gct ttc ttc tgg    1584
   Phe Pro Gly Ile Thr Pro Tyr Met Leu Thr Leu Gly Ala Phe Phe Trp
                   515                 520                 525
   gtt cct ttc ttg aga gat tac gtc atg tca acc gga agt tgt tct gtg    1632
   Val Pro Phe Leu Arg Asp Tyr Val Met Ser Thr Gly Ser Cys Ser Val
               530                 535                 540
   tcc aga agc tct atg gac ttc ttg ctt acg caa aag ggt aca gga aac    1680
   Ser Arg Ser Ser Met Asp Phe Leu Leu Thr Gln Lys Gly Thr Gly Asn
           545                 550                 555
   atg ctt gtt gtc gtt gtt ggt gga ctt gct gaa tgt aga tac tct aca      1728
   Met Leu Val Val Val Val Gly Gly Leu Ala Glu Cys Arg Tyr Ser Thr
       560                 565                 570
   cca ggc tca aca acc ctt ttc cta aag aag agg cag gga ttt gtt cgt      1776
   Pro Gly Ser Thr Thr Leu Phe Leu Lys Lys Arg Gln Gly Phe Val Arg
   575                 580                 585                 590
   aca gca cta aag cat ggg gtt tct ttg atc cct gct tat gca ttt gga      1824
   Thr Ala Leu Lys His Gly Val Ser Leu Ile Pro Ala Tyr Ala Phe Gly
                   595                 600                 605
   gag act gac ctc tac gat caa cat atc ttc act cct gga gga ttc gtc      1872
   Glu Thr Asp Leu Tyr Asp Gln His Ile Phe Thr Pro Gly Gly Phe Val
               610                 615                 620
   aac aga ttc caa aag tgg ttc cag aag atg gtt cac atc tac cca tgt      1920
   Asn Arg Phe Gln Lys Trp Phe Gln Lys Met Val His Ile Tyr Pro Cys
           625                 630                 635
   gct ttc tat gga aga ggt ctg acc aag aat tca tgg ggt ctt ttg cct      1968
   Ala Phe Tyr Gly Arg Gly Leu Thr Lys Asn Ser Trp Gly Leu Leu Pro
       640                 645                 650
   tac tca caa cca gtt act aca gtt gtt gga gag cca tta ccg ttg cct      2016
   Tyr Ser Gln Pro Val Thr Thr Val Val Gly Glu Pro Leu Pro Leu Pro
   655                 660                 665                 670
   aag ata gaa aac cct agc gaa gag att gtt gcc aag tat cac acc ctt      2064
   Lys Ile Glu Asn Pro Ser Glu Glu Ile Val Ala Lys Tyr His Thr Leu
                   675                 680                 685
   tac atc gat gct ctc aga aag cta ttc gac cag cac aag act aag ttc      2112
   Tyr Ile Asp Ala Leu Arg Lys Leu Phe Asp Gln His Lys Thr Lys Phe
               690                 695                 700
   gga atc tct gag aca caa gag ctg gtt atc gtc taa gagctc               2154
   Gly Ile Ser Glu Thr Gln Glu Leu Val Ile Val
           705                 710
<210> 16
   <211> 713
   <212> PRT
   <213> Artificial
<220>
   <223> Synthetic Construct
<400> 16
   Met Ala Asp Gln Thr Arg Thr His His Glu Met Ile Ser Arg Asp Ser
   1               5                   10                  15
   Thr Gln Glu Ala His Pro Lys Ala Arg Gln Met Val Lys Ala Ala Thr
               20                  25                  30
   Ala Val Thr Ala Gly Gly Ser Leu Leu Val Leu Ser Gly Leu Thr Leu
           35                  40                  45
   Ala Gly Thr Val Ile Ala Leu Thr Val Ala Thr Pro Leu Leu Val Ile
       50                  55                  60
   Phe Ser Pro Val Leu Val Pro Ala Val Val Thr Val Ala Leu Ile Ile
   65                  70                  75                  80
   Thr Gly Phe Leu Ala Ser Gly Gly Phe Gly Ile Ala Ala Ile Thr Ala
   85 90 95
   Phe Ser Trp Leu Tyr Arg His Met Thr Gly Ser Gly Ser Asp Lys Ile
   100 105 110
   Glu Asn Ala Arg Met Lys Val Gly Ser Arg Val Gln Asp Thr Lys Tyr
   115 120 125
   Gly Gln His Asn Ile Gly Val Gln His Gln Gln Val Ser Met Val Ser
   130 135 140
   Lys Gly Glu Glu Leu Phe Thr Gly Val Val Pro Ile Leu Val Glu Leu
   145 150 155 160
   Asp Gly Asp Val Asn Gly His Lys Phe Ser Val Ser Gly Glu Gly Glu
   165 170 175
   Gly Asp Ala Thr Tyr Gly Lys Leu Thr Leu Lys Phe Ile Cys Thr Thr
   180 185 190
   Gly Lys Leu Pro Val Pro Trp Pro Thr Leu Val Thr Thr Phe Gly Tyr
   195 200 205
   Gly Leu Gln Cys Phe Ala Arg Tyr Pro Asp His Met Lys Gln His Asp
   210 215 220
   Phe Phe Lys Ser Ala Met Pro Glu Gly Tyr Val Gln Glu Arg Thr Ile
   225 230 235 240
   Phe Phe Lys Asp Asp Gly Asn Tyr Lys Thr Arg Ala Glu Val Lys Phe
   245 250 255
   Glu Gly Asp Thr Leu Val Asn Arg Ile Glu Leu Lys Gly Ile Asp Phe
   260 265 270
   Lys Glu Asp Gly Asn Ile Leu Gly His Lys Leu Glu Tyr Asn Tyr Asn
   275 280 285
   Ser His Asn Val Tyr Ile Met Ala Asp Lys Gln Lys Asn Gly Ile Lys
   290 295 300
   Val Asn Phe Lys Ile Arg His Asn Ile Glu Asp Gly Ser Val Gln Leu
   305 310 315 320
   Ala Asp His Tyr Gln Gln Asn Thr Pro Ile Gly Asp Gly Pro Val Leu
   325 330 335
   Leu Pro Asp Asn His Tyr Leu Ser Tyr Gln Ser Ala Leu Ser Lys Asp
   340 345 350
   Pro Asn Glu Lys Arg Asp His Met Val Leu Leu Glu Phe Val Thr Ala
   355 360 365
   Ala Gly Ile Thr Leu Gly Met Asp Glu Leu Tyr Lys Met Phe Trp Pro
   370 375 380
   Thr Lys Lys Asp Leu Lys Thr Ala Met Glu Val Phe Ala Leu Phe Gln
   385 390 395 400
   Trp Ala Leu Ser Ala Leu Val Ile Val Thr Thr Val Ile Ile Val Asn
   405 410 415
   Leu Tyr Leu Val Val Phe Thr Ser Tyr Trp Pro Val Thr Val Leu Met
   420 425 430
   Leu Thr Trp Leu Ala Phe Asp Trp Lys Thr Pro Glu Arg Gly Gly Arg
   435 440 445
   Arg Phe Thr Cys Val Arg Lys Trp Arg Leu Trp Lys His Tyr Ser Asp
   450 455 460
   Tyr Phe Pro Leu Lys Met Val Lys Thr Lys Asp Ile Ser Pro Asp Arg
   465 470 475 480
   Asn Tyr Ile Leu Val Cys His Pro His Gly Leu Met Ala His Ser Cys
   485 490 495
   Phe Gly His Phe Ala Thr Asp Thr Thr Gly Phe Ser Lys Thr Phe Pro
   500 505 510
   Gly Ile Thr Pro Tyr Met Leu Thr Leu Gly Ala Phe Phe Trp Val Pro
   515 520 525
   Phe Leu Arg Asp Tyr Val Met Ser Thr Gly Ser Cys Ser Val Ser Arg
   530 535 540
   Ser Ser Met Asp Phe Leu Leu Thr Gln Lys Gly Thr Gly Asn Met Leu
   545 550 555 560
   Val Val Val Val Gly Gly Leu Ala Glu Cys Arg Tyr Ser Thr Pro Gly
   565 570 575
   Ser Thr Thr Leu Phe Leu Lys Lys Arg Gln Gly Phe Val Arg Thr Ala
   580 585 590
   Leu Lys His Gly Val Ser Leu Ile Pro Ala Tyr Ala Phe Gly Glu Thr
   595 600 605
   Asp Leu Tyr Asp Gln His Ile Phe Thr Pro Gly Gly Phe Val Asn Arg
   610 615 620
   Phe Gln Lys Trp Phe Gln Lys Met Val His Ile Tyr Pro Cys Ala Phe
   625                 630                 635                 640
   Tyr Gly Arg Gly Leu Thr Lys Asn Ser Trp Gly Leu Leu Pro Tyr Ser
                   645                 650                 655
   Gln Pro Val Thr Thr Val Val Gly Glu Pro Leu Pro Leu Pro Lys Ile
               660                 665                 670
   Glu Asn Pro Ser Glu Glu Ile Val Ala Lys Tyr His Thr Leu Tyr Ile
           675                 680                 685
   Asp Ala Leu Arg Lys Leu Phe Asp Gln His Lys Thr Lys Phe Gly Ile
       690                 695                 700
   Ser Glu Thr Gln Glu Leu Val Ile Val
   705                 710